(19) Europäisches Patentamt
European Patent Office
Office européen des brevets

(11) **EP 2 009 108 A1**

(12) **EUROPÄISCHE PATENTANMELDUNG**


(43) Veröffentlichungstag:
**31.12.2008 Patentblatt 2009/01**

(51) Int Cl.:
***C12N 15/82*** *(2006.01)* ***C08B 30/00*** *(2006.01)*

(21) Anmeldenummer: **07075524.4**

(22) Anmeldetag: **25.06.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**



(71) Anmelder: **Bayer CropScience AG**
**40789 Monheim/Rhein (DE)**

(72) Erfinder:
- **Frohberg, Claus**
  **14532 Kleinmachnow (DE)**
- **La Cognata, Ursula**
  **14163 Berlin (DE)**

(74) Vertreter: **Kossmann, Jochen**
**Bayer BioScience GmbH**
**Hermannswerder 20a**
**14473 Potsdam (DE)**


(54) **Stärken mit hohem Amylosegehalt und verbesserten Verarbeitungseigenschaften**


(57) Die vorliegende Erfindung betrifft Maisstärken mit einem apparenten Amylosegehalt zwischen 35 Gew.-% und 90 Gew.-% und gegenüber herkömmlichen Hochamylose-Maisstärken verbesserten Verarbeitungseigenschaften. Weiterhin betrifft die vorliegende Erfindung Maismehle und Lebensmittel enthaltend diese Maisstärken oder Maismehle. Ferner betrifft die vorliegende Erfindung Verfahren zur Herstellung solcher Maisstärken/Maismehle sowie Mais-Pflanzen, die diese Maisstärken synthetisieren. Außerdem betrifft die vorliegende Erfindung Weizenstärken mit einem apparenten Amylosegehalt zwischen 35 Gew.-% und 90 Gew.-% sowie verbesserten Verarbeitungseigenschaften, sowie Weizenmehle und Lebensmittel enthaltend diese Weizenstärken oder Weizenmehle. Ferner betrifft die vorliegende Erfindung Verfahren zur Herstellung solcher Weizenstärken/Weizenmehle sowie WeizenPflanzen, die diese Weizenstärken synthetisieren.


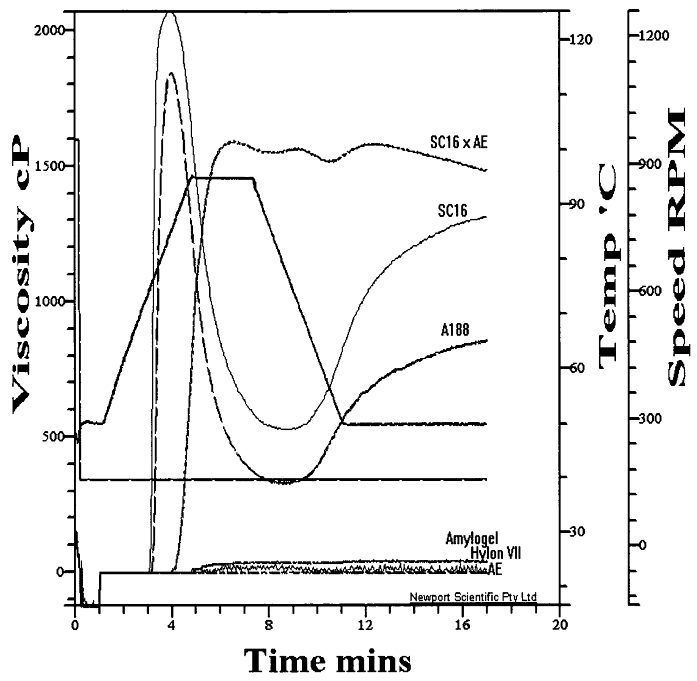


Figur 1

EP 2 009 108 A1

Printed by Jouve, 75001 PARIS (FR)

## Beschreibung

Die vorliegende Erfindung betrifft Maisstärken mit einem apparenten Amylosegehalt zwischen 35 Gew.-% und 90 Gew.-% und gegenüber herkömmlichen Hochamylose-Maisstärken verbesserten Verarbeitungseigenschaften. Weiterhin betrifft die vorliegende Erfindung Maismehle und Lebensmittel enthaltend diese Maisstärken oder Maismehle. Ferner betrifft die vorliegende Erfindung Verfahren zur Herstellung solcher Maisstärken/Maismehle sowie Mais-Pflanzen, die diese Maisstärken synthetisieren. Außerdem betrifft die vorliegende Erfindung Weizenstärken mit einem apparenten Amylosegehalt zwischen 35 Gew.-% und 90 Gew.-% sowie verbesserten Verarbeitungseigenschaften, sowie Weizenmehle und Lebensmittel enthaltend diese Weizenstärken oder Weizenmehle. Ferner betrifft die vorliegende Erfindung Verfahren zur Herstellung solcher Weizenstärken/Weizenmehle sowie Weizen-Pflanzen, die diese Weizenstärken synthetisieren.

Neben Ölen, Fetten und Proteinen stellen Polysaccharide die wesentlichen nachwachsenden Rohstoffe aus Pflanzen dar. Eine zentrale Stellung bei den Polysacchariden nimmt neben Cellulose die Stärke ein, die einer der wichtigsten Speicherstoffe in Höheren Pflanzen ist.

Weiterhin stellt Stärke einen ernährungsphysiologisch wesentlichen Bestandteil der menschlichen und tierischen Nahrung dar. Die strukturellen Merkmale der in Nahrungsmitteln enthaltenden Stärke können die funktionellen (z.B. Wasserbindungsvermögen, Quellvermögen), ernährungsphysiologischen (z.B. Verdaubarkeit, Einfluß des Nahrungsmittels auf den Glykämischen Index) oder strukturgebenden (z.B. Schnittfestigkeit, Textur, Klebrigkeit, Verarbeitbarkeit) Eigenschaften von verschiedensten Nahrungsmitteln beeinflussen. Nahrungsmittelzusammensetzungen enthalten daher häufig eine Stärke mit bestimmten strukturellen Merkmalen, die die gewünschten Eigenschaften des betreffenden Nahrungsmittels bedingen. Auch die Eigenschaften von Nahrungsmitteln, enthaltend Stärke speichernde Pflanzengewebe (z.B. Körner, Früchte, Mehle), können von der in den Pflanzengeweben enthaltenden Stärke beeinflusst werden.

Das Polysaccharid Stärke ist ein Polymer aus chemisch einheitlichen Grundbausteinen, den Glucosemolekülen. Es handelt sich dabei jedoch um ein sehr komplexes Gemisch aus unterschiedlichen Molekülformen, die sich hinsichtlich ihres Polymerisationsgrades, des Auftretens von Verzweigungen der Glucoseketten und deren Kettenlängen unterscheiden, die darüber hinaus modifiziert, z.B. phosphoryliert sein können. Daher stellt Stärke keinen einheitlichen Rohstoff dar. Man unterscheidet insbesondere die Amylose, ein im wesentlichen unverzweigtes Polymer aus alpha-1,4-glycosidisch verknüpften Glucosemolekülen, vom Amylopektin, das ein komplexes Gemisch aus unterschiedlich verzweigten Glucoseketten darstellt. Die Verzweigungen kommen dabei durch das Auftreten von zusätzlichen alpha-1,6-glycosidischen Verknüpfungen zustande. In typischen, für die industrielle Stärkeproduktion oder als Nahrungsmittel verwendeten Pflanzen, wie z.B. Mais, Reis, Weizen oder Kartoffel, besteht die synthetisierte Stärke zu ca. 20% - 25% aus Amylose und zu ca. 70% - 75% aus Amylopektin.

Es stehen unterschiedliche Methoden zur Bestimmung des Amylosegehaltes zur Verfügung, die für ein und dieselbe Stärke zu unterschiedlichen Zahlenwerten des Amylosegehaltes führen können. Viele dieser Methoden basieren auf dem Iodbindevermögen der Amylose, das potentiometrisch (Banks & Greenwood, in W. Banks & C.T. Greenwood, Starch and its components (pp. 51-66), Edinburgh, Edinburgh University Press), amperometrisch (Larson et al., Analytical Chemistry 25(5), (1953), 802-804) oder spektrophotometrisch (Morrison & Laignelet, J. Cereal Sc. 1, (1983), 9-20) bestimmt werden kann. Die Bestimmung des Amylosegehaltes kann auch kalorimetrisch mittels DSC-(Differential Scanning Calorimetry)-Messungen erfolgen (Kugimiya & Donovan, Journal of Food Science 46, (1981), 765-770; Sievert & Holm, Starch/Stärke 45 (4), (1993), 136-139). Ferner besteht die Möglichkeit, den Amylosegehalt über den Einsatz von SEC-(size exclusion chromatography)-Chromatographie von nativer oder entzweigter Stärke zu bestimmen. Diese Methode wurde insbesondere zur Bestimmung des Amylosegehaltes gentechnisch modifizierter Stärken empfohlen (Gérard et al., Carbohydrate Polymers 44, (2001),19-27).

Die funktionellen, ernährungsphysiologischen oder strukturgebenden Eigenschaften der Stärke, wie z.B. die Löslichkeit, das Retrogradationsverhalten, das Wasserbindevermögen, die Filmbildungseigenschaften, die Viskosität, die Verkleisterungseigenschaften, die Gefrier-Tau-Stabilität, die Säurestabilität, die Gelfestigkeit, das Quellvermögen, die Verdaubarkeit, die Stärkekorngröße von Stärken werden u.a. durch die strukturellen Merkmale der Stärke, wie z.B. das Amylose/Amylopektin Verhältnis, das Molekulargewicht der Glucosepolymere, das Muster der Seitenkettenverteilung, den Phosphatgehalt, den Lipid- oder Proteingehalt beeinflusst.

Mit Hilfe klassischer Züchtungsverfahren ist eine gezielte Veränderung der strukturellen/funktionellen Eigenschaften der in den Pflanzen synthetisierten Stärke nur schwer und nur für ausgewählte strukturelle Merkmale möglich. Eine Alternative zu züchterischen Verfahren besteht in der gezielten Modifikation Stärke produzierender Pflanzen durch gentechnische Methoden. Voraussetzung hierfür ist jedoch die Identifizierung und Charakterisierung der an der Stärkesynthese und/oder Stärkemodifikation beteiligten Enzyme und deren anschließende funktionelle Analyse in transgenen Pflanzen.

Maismutanten mit gegenüber Mais-Wildtyppflanzen erhöhtem Amylosegehalt sind bekannt und werden als "amylose extender" oder kurz "ae" bezeichnet. Amylose extender (ae) - Maismutanten wurden beispielsweise beschrieben bei Vineyard and Bear (Maize Genet Coop Newsletter 26: 5 (1952), die das Referenz-Allel ae1-Ref beschreiben,

sowie bei Moore und Creech (Genetics 70, (1972), 611-619), Garwood et al. (Cereal Chemistry 53(3), (1976), 355-364) und Hedman und Boyer (Biochemical Genetics 21 (11/12), (1983), 1217-1222).

Mais-Pflanzen(zellen), die eine "amylose extender-Mutation" aufweisen, zeigen eine Mutation des Gens des Stärke-Verzweigungsenzyms IIb aus Mais (= engl. Starch Branching Enzyme, Abkürzung "BE IIb" oder "SBE IIb"), das auch als amylose extender - Gen bezeichnet wird. Diese Mutation führt zu einer Verringerung der SBE IIb - Enzymaktivität im Endosperm dieser Maispflanzen im Vergleich zur BE IIb - Aktivität im Endosperm von Mais- Wildtyppflanzen. Vorzugsweise führt diese Mutation des BE IIb in Mais-Pflanzen(zellen) dazu, dass keine SBE IIb - Aktivität mehr nachweisbar ist (z.B. Fisher et al., Plant Physiol. 110, (1996), 611-619, insbesondere Figur 4; Hedman und Boyer, Biochemical Genetics 21 (11/12), (1983), 1217-1222, insbesondere Tabelle 1).

Bei der "amylose extender-Mutation" handelt es sich in der Regel um eine rezessive Mutation des amylose extender 1 - Lokus.

Amylose extender- Mutanten synthetisieren eine Hochamylose-Maisstärke, die einen im Vergleich zu Mais-Wildtyp-Pflanzen(-zellen) erhöhten apparenten Amylosegehalt aufweist, der in der Regel zwischen 45 und 75 Gew.-% liegt, je nach genetischem Hintergrund, Anbaubedingungen und Amylosebestimmungsmethode.

Maisstärken mit der Produktbezeichnung Amylogel®, Hylon® V und Hylon® VII sind im Handel erhältlich und weisen in der Regel einen apparenten Amylosegehalt von ca. 50% bzw. 70% auf (Shi et al., Journal of Cereal Science 27, (1998), 289-299).

Im Gegensatz zu Mais-Wildtyp-Stärken zeigen diese Hochamylose-Maisstärken ein wesentlich verringertes Quellvermögen (Senti und Russell, Tappi Vol. 43 No.4, (1960), 343-349; Shi et al., Journal of Cereal Science 27, (1998), 289-299).

Wässrige Suspensionen der Hochamylose-Maisstärken zeigen in der Viskositätsanalyse (z.B. RVA-Analyse) keine Viskositätsentwicklung (Senti und Russell, Tappi Vol. 43 No.4, (1960), 343-349). Native Hochamylose-Maisstärken lassen sich bisher nur in einem sehr energie- und damit kostenintensivem Verfahren im Autoklaven bei erhöhter Temperatur und Druck verkleistern, oder sie müssen nachträglich chemisch modifiziert werden.

Aufgrund der dargestellten Eigenschaften lassen sich die Hochamylose-Maisstärken nur schlecht verarbeiten, so dass die Anwendungsbereiche, in denen die vorteilhaften Eigenschaften der nativen Hochamylose-Maisstärken (z.B. sehr gute Filmbildungseigenschaften, gute Gelbildungseigenschaften, Einsatz als prebiotisch wirkende resistente Stärke) genutzt werden können, bisher stark eingeschränkt sind. Es besteht daher ein großer Bedarf an Hochamylose-Maisstärken mit verbesserten Verarbeitungseigenschaften, wie z.B. einer erhöhten Heißwasser-Löslichkeit, einem erhöhten Quellvermögen und der Fähigkeit zur Viskositätsbildung in wässrigen Suspensionen unter Normalbedingungen.

Hochamylose-Weizenstärken mit guten Verarbeitungseigenschaften stehen gleichfalls nicht zur Verfügung.

Eine Aufgabe der vorliegenden Erfindung ist es demnach, Hochamylose-Maisstärken bzw. -Maismehle, die diese Maisstärken enthalten, zur Verfügung zu stellen, welche im Vergleich zu den bekannten Hochamylose-Maisstärken von amylose extender Maismutanten verbesserte Verarbeitungseigenschaften aufweisen, sowie Mais-Pflanzen(zellen) zur Verfügung zu stellen, die solche Maisstärken synthetisieren.

Eine weitere Aufgabe der vorliegenden Erfindung ist es, Hochamylose-Weizenstärken bzw. -Weizenmehle, die diese Weizenstärken enthalten, zur Verfügung zu stellen, welche im Vergleich zu Weizenstärke bzw. Weizenmehl aus Weizenpflanzen mit amylose extender Genotyp verbesserte Verarbeitungseigenschaften aufweisen, sowie Weizen-Pflanzen(zellen) zur Verfügung zu stellen, die solche Weizenstärken synthetisieren.

Diese Aufgaben werden durch die in den Ansprüchen bezeichneten Ausführungsformen gelöst.

Die vorliegende Erfindung betrifft somit eine Maisstärke, die einen apparenten Amylosegehalt zwischen 35 Gew.-% und 90 Gew.-%, zwischen 38 Gew.-% und 85 Gew.-%, vorzugsweise zwischen zwischen 40 Gew.-% und 80 Gew.-%, besonders bevorzugt zwischen 42 Gew.-% und 75 Gew.-% aufweist und die in der RVA-Analyse bestimmte Viskositätseigenschaften zeigt.

In einer weiteren Ausführungsform weist die erfindungsgemäße Maisstärke einen apparenten Amylosegehalt von mindestens 35 Gew.-%, vorzugsweise von mindestens 40 Gew.-% auf.

In einer weiteren Ausführungsform weist die erfindungsgemäße Maisstärke einen apparenten Amylosegehalt zwischen 38 Gew.-% und 85 Gew.-%, vorzugsweise zwischen zwischen 40 Gew.-% und 80 Gew.-%, besonders bevorzugt zwischen 42 Gew.-% und 75 Gew.-%.

In einer Ausführungsform der vorliegenden Erfindung zeigt die erfindungsgemäße Mais-Stärke in einer wässrigen Suspension in der RVA-Analyse eine Zunahme der Viskosität von mehr als 60 Centi Poise (cP), vorzugsweise mehr als 100 und besonders bevorzugt mehr als 200 Centi Poise (cP).

Die vorliegende Erfindung betrifft auch eine Weizenstärke, die einen apparenten Amylosegehalt zwischen 35 Gew.-% und 90 Gew.-%, zwischen 38 Gew.-% und 85 Gew.-%, vorzugsweise zwischen zwischen 40 Gew.-% und 80 Gew.-%, besonders bevorzugt zwischen 42 Gew.-% und 75 Gew.-%. aufweist und die in der RVA-Analyse bestimmte Viskositätseigenschaften zeigt.

In einer Ausführungsform der vorliegenden Erfindung zeigt die erfindungsgemäße Weizen-Stärke in einer wässrigen Suspension in der RVA-Analyse eine Zunahme der Viskosität von mehr als 60 Centi Poise (cP), vorzugsweise mehr als 100 und besonders bevorzugt mehr als 200 Centi Poise (cP).

In einer weiteren Ausführungsform weist die erfindungsgemäße Weizenstärke einen apparenten Amylosegehalt von mindestens 35 Gew.-%, vorzugsweise von mindestens 40 Gew.-% auf.

In einer weiteren Ausführungsform weist die erfindungsgemäße Weizenstärke einen apparenten Amylosegehalt zwischen 38 Gew.-% und 85 Gew.-%, vorzugsweise zwischen zwischen 40 Gew.-% und 80 Gew.-%, besonders bevorzugt zwischen 42 Gew.-% und 75 Gew.-% auf.

Verfahren zur Bestimmung des Amylosegehaltes sind dem Fachmann bekannt. Im Zusammenhang mit der vorliegenden Erfindung wird unter dem Amylosegehalt der Gehalt an apparenter Amylose in Gewichtsprozent bezogen auf die Stärkemenge verstanden. Die Bestimmung des apparenten Amylosegehaltes einer Stärke/eines Mehles erfolgt im Zusammenhang mit der vorliegenden Erfindung vorzugsweise nach der weiter unten beschriebenen Methode (siehe "Allgemeine Methoden, Bestimmung des Gehaltes an apparenter Amylose").

In einer besonders bevorzugten Ausführungsform handelt es sich bei der erfindungsgemäßen Maisstärke um eine native Maisstärke.

Der Begriff "native Maisstärke" bedeutet im Zusammenhang mit der vorliegenden Erfindung, dass die Maisstärke nach dem Fachmann bekannten Methoden aus erfindungsgemäßen Mais-Pflanzen, aus stärkespeichernden Teilen der erfindungsgemäßen Mais-Pflanzen oder aus erfindungsgemäßem Vermehrungsmaterial isoliert wird. Nach der Isolierung erfolgt bei den erfindungsgemäßen nativen Maisstärken keine zusätzliche chemische Derivatisierung (z.B. Acetylierung, Oxidierung, Vernetzung etc.).

In einer besonders bevorzugten Ausführungsform handelt es sich bei der erfindungsgemäßen Weizenstärke um eine native Weizenstärke.

Der Begriff "native Weizenstärke" bedeutet im Zusammenhang mit der vorliegenden Erfindung, dass die Weizenstärke nach dem Fachmann bekannten Methoden aus erfindungsgemäßen Weizen-Pflanzen, aus stärkespeichernden Teilen der erfindungsgemäßen Weizen-Pflanzen oder aus erfindungsgemäßem Vermehrungsmaterial isoliert wird. Nach der Isolierung erfolgt bei den erfindungsgemäßen nativen Weizenstärken keine zusätzliche chemische Derivatisierung (z.B. Acetylierung, Oxidierung, Vernetzung etc.).

Der Begriff "RVA-Analyse" bedeutet im Zusammenhang mit der vorliegenden Erfindung, dass die Viskositätseigenschaften der (erfindungsgemäßen) Stärken mit Hilfe eines "Rapid Visco Analyzers" (=RVA) bestimmt werden. RVA-Geräte werden beispielsweise hergestellt und vertrieben durch die Firma Newport Scientific Pty Ltd, Investment Support Group, Warriewood, NSW 2102, Australien. Die RVA-Analyse ist die Standard-Analysen-Methode zur Untersuchung der Verkleisterungseigenschaften von Stärke.

Im Zusammenhang mit der vorliegenden Erfindung erfolgt die RVA-Analyse der erfindungsgemäßen Stärken vorzugsweise nach der AACC-Standard-Methode Nr. 61-02, "Determination of the Pasting Properties of Rice with the Rapid Visco Analyser" (Final Approval October 15, 1997; Reapproval November 3, 1999; Approved Methods of the American Association of Cereal Chemists (AACC) - 10th Edition - Including 2001, 2002 and 2003 Supplements, American Association of Cereal Chemists (2003), St Paul MN, USA). An Stelle der in dieser AACC-Methode beschriebenen Reismehlproben werden im Zusammenhang mit der vorliegenden Erfindung die erfindungsgemäßen Mais- oder Weizenstärken analysiert. Im Zusammenhang mit der vorliegenden Erfindung wird vorzugsweise das in der AACC-Methode 61-02 beschriebene Temperatur- und Scherprofil angewendet, das auch weiter unten (siehe "Allgemeine Methoden") beschrieben wird.

In einer besonders bevorzugten Ausführungsform der vorliegenden Erfindung beziehen sich die Änderungen der Viskositätseigenschaften in der RVA-Analyse gemäß AACC-Methode 61-02 auf eine 10% ige (w/v) wässrige Suspension der erfindungsgemäßen Stärke in Wasser.

Mit Hilfe der RVA-Analyse sind verschiedene Größen bestimmbar, wie z.B. die Verkleisterungstemperatur, die Peakviskosität, die Endviskosität und das Setback.

In einer weiteren Ausführungsform beträgt die Zunahme der Viskosität einer wässrigen Suspension der erfindungsgemäßen Mais- oder Weizenstärke in der RVA-Analyse mehr als 250 Centi Poise (cP), vorzugsweise mehr als 500 und besonders bevorzugt mehr als 1000 Centi Poise (cP) im Vergleich zur Viskosität in Centi Poise (cP) zu Beginn der RVA-Analyse.

In einer weiteren Ausführungsform beträgt die Zunahme der Viskosität einer wässrigen Suspension der erfindungsgemäßen Mais- oder Weizenstärke in der RVA-Analyse zwischen 200 und 2000 Centi Poise (cP), vorzugsweise zwischen 1000 und 1800 Centi Poise (cP) und besonders bevorzugt zwischen 1300 und 1700 Centi Poise (cP).

In einer weiteren Ausführungsform weist die erfindungsgemäße Mais-Stärke in der RVA-Analyse eine messbare Verkleisterungstemperatur auf, vorzugsweise von mindestens 75° C, bevorzugt von mindestens 80°C und besonders bevorzugt von mindestens 85°C.

In einer weiteren Ausführungsform weist die erfindungsgemäße Mais-Stärke in der RVA-Analyse eine Verkleisterungstemperatur zwischen 78°C bis 95°C, vorzugsweise zwischen 80°C und 93°C und besonders bevorzugt zwischen 82°C und 91 °C auf.

Im Gegensatz zu herkömmlichen nativen Hochamylosestärken, die in der RVA-Analyse unter Normaldruck nicht oder nur kaum verkleistern, weisen die erfindungsgemäßen Stärken den Vorteil auf, dass sie trotz des erhöhten Amylosegehaltes unter Standardbedingungen verkleistern. Dadurch wird die Verarbeitbarkeit der erfindungsgemäßen Stärke gegenüber anderen Hochamylose-Stärken deutlich verbessert. Es ist daher zum Gelatinisieren der erfindungsgemäßen-Stärken keine erhöhte Temperatur oder erhöhter Druck notwendig, was Emergie-und somit Kostenersparnisse zur Folge hat.

Der Begriff "Verkleisterungstemperatur" (RVA PT = RVA Pasting Temperature) bedeutet im Zusammenhang mit der vorliegenden Erfindung, dass der Messwert für den Beginn der Viskositätsentwicklung gemäß RVA-Analysen diejenige Temperatur ist, bei der die Viskositätskurve während des Aufheizungsprozesses die Basislinie verlässt und bei der sich in einem Zeitintervall von 0,5 Minuten die Viskosität um mehr als 50 centi Poise (cP) ändert.

In einer weiteren Ausführungsform weisen die erfindungsgemäßen Mais- oder Weizenstärken eine Peakviskosität zwischen 1200 und 2000 Centi Poise (cP), vorzugsweise zwischen 1400 und 1900 Centi Poise (cP) und besonders bevorzugt zwischen 1500 und 1850 Centi Poise (cP) auf.

Im Gegensatz zu herkömmlichen Hochamylose-Mais-Stärken, die nicht viskositätsbildend wirken, zeigen die erfindungsgemäßen Mais-Stärken überraschenderweise eine Peak-Viskosität, die der von Mais-Wildtyp-Pflanzen vergleichbar ist oder zumindest sehr nahe kommt. Entsprechendes gilt für die erfindungsgemäßen Weizenstärken.

Unter der "Peak-Viskosität" der RVA-Analyse wird im Zusammenhang mit der vorliegenden Erfindung die maximale Viskosität gemessen in Centi Poise (cP) verstanden, die nach 2 bis 8 Minuten Messzeit ermittelt wird.

In einer weiteren Ausführungsform weisen die erfindungsgemäßen Mais- oder Weizen-Stärken eine "Trough-Viskosität" von mindestens 600, vorzugsweise mindestens 750 und besonders bevorzugt von mindestens 1000 Centi Poise (cP) auf. In diesem Merkmal unterschieden sich die erfindungsgemäßen Mais-Stärken sowohl deutlich von herkömmlichen Hochamylose-Maisstärken, die aufgrund der mangelnden Viskositätsbildung keine oder allenfalls nur eine sehr geringe Trough-Viskosität von weniger als 150 Centi Poise (cP) aufweisen, als auch von Mais-Stärken von Mais-Wildtyppflanzen, die ebenfalls eine deutlich geringere Trough-Viskosität von weniger als 1000 Centi Poise (cP) aufweisen. Entsprechendes gilt für die erfindungsgemäßen Weizenstärken.

Unter der "Trough -Viskosität" der RVA-Analyse wird im Zusammenhang mit der vorliegenden Erfindung die minimale Viskosität gemessen in Centi Poise (cP) verstanden, die nach Erreichen der Peakviskosität auftritt und die nach 6 bis 12 Minuten Messzeit ermittelt wird.

In einer weiteren Ausführungsform weisen die erfindungsgemäßen Mais- oder Weizenstärken in der RVA-Analyse eine Endviskosität von mehr als 1000 Centi Poise (cP), vorzugsweise mehr als 1150 und besonders bevorzugt mehr als 1350 Centi Poise (cP) auf.

In einer weiteren Ausführungsform weisen die erfindungsgemäßen Mais- oder Weizenstärken eine Endviskosität zwischen 1100 und 2000 Centi Poise (cP), vorzugsweise zwischen 1300 und 1800 Centi Poise (cP) und besonders bevorzugt zwischen 1450 und 1700 Centi Poise (cP) auf.

Unter der "Endviskosität" (=Final Viscosity) versteht man im Zusammenhang mit der vorliegenden Erfindung den Viskositätswert in Centi-Poise, der am Ende der RVA-Analyse auftritt. Gemäß der AACC-Methode 61-02 ist das Ende der RVA-Analyse nach 12,5 Minuten Messzeit erreicht.

In einer weiteren Ausführungsform weisen die erfindungsgemäßen Mais- oder Weizenstärken ein "Breakdown" in der RVA-Analyse zwischen 30 und 500 Centi Poise (cP), vorzugsweise zwischen 40 und 250 Centi Poise (cP) und besonders bevorzugt zwischen 50 und 150 Centi Poise (cP) auf. Hierin unterscheiden sich die erfindungsgemäßen Mais-Stärken deutlich von Mais-Wildtypstärken.

Als "Breakdown" wird im Zusammenhang mit der vorliegenden Erfindung die Differenz der Peakviskosität minus der Trough-Viskosität verstanden.

In einer weiteren Ausführungsform der vorliegenden Erfindung weisen die erfindungsgemäßen Mais- oder Weizen-Stärken in der RVA-Analyse ein Setback von weniger als 350 Centi Poise (cP), vorzugsweise von weniger als 200, insbesondere weniger als 150 und besonders bevorzugt von weniger als 100 Centi Poise (cP) auf.

In einer weiteren Ausführungsform weisen die erfindungsgemäßen Mais- oder Weizenstärken ein Setback zwischen -100 und 350 Centi Poise (cP), vorzugsweise zwischen 0 und 250 Centi Poise (cP) und besonders bevorzugt zwischen 20 und 175 Centi Poise (cP) auf.

Der Begriff "Setback" bezeichnet im Zusammenhang mit der vorliegenden Erfindung die Differenz aus Endviskosität minus der Trough-Viskosität.

Die erfindungsgemäßen (Mais)-Stärken weisen somit nicht nur den Vorteil auf, dass ihre wässrigen Suspensionen über einen breiten Temperaturbereich viskositätsstabil sind, sondern auch, dass das unerwünschte Nachdicken der Stärkesuspensionen nicht oder nur in vermindertem Umfang auftritt im Vergleich zu Wildtyp-Stärken.

Ein weiterer Vorteil der erfindungsgemäßen Stärken liegt darin, dass sie bei Verarbeitungsprozessen, in denen Heißwalzen eingesetzt werden, als Suspension auf diese aufgebracht werden können. Andere Mais-Stärken mit erhöhtem Amylosegehalt würden bei dieser Verarbeitung, wenn überhaupt, nur begrenzt verkleistern und daher auch nicht als Paste oder Film auf die entsprechenden Walzen aufgebracht werden können. Besonders geeignet sind die erfindungsgemäßen Stärken für alle Anwendungen, bei welchen die Dickungsleistung, die Geliereigenschaften oder die Bindungseigenschaften zugesetzter Substanzen von Bedeutung sind. Daher eignet sich die erfindungsgemäße Stärke besonders zur Herstellung von Lebensmitteln wie z.B. Backwaren, Fertignahrungsmitteln (Instant Food), Pudding, Suppen, Konfekt, Schokolade, Eiskrem, Panaden für Fisch oder Fleisch, gefrorene Desserts oder extrudierte Snacks. Weiterhin ist die erfindungsgemäße Stärke geeignet für die Herstellung von Klebstoffen, Anwendungen bei der Textilverarbeitung, als Zusatz für Baustoffe, für Anwendungen im Bereich der Tieremährung, als Zusatzstoff für Kosmetika und in der Papierverarbeitung.
Insbesondere ist der Einsatz der erfindungsgemäßen Stärke zur Herstellung von kaltwasserlöslichen Quellstärken denkbar. Quellstärken sind physikalisch modifizierte Stärken, die vorwiegend durch nass-thermischen Aufschluss hergestellt werden. Im Unterschied zu nativer Stärke bilden sie mit kaltem Wasser Dispersionen bzw. Pasten oder Gele, je nach eingesetzter Konzentration der Quellstärke und in Abhängigkeit von der zur Herstellung der Quellstärke verwendeten Stärkeart. Aufgrund dieser Eigenschaften ergeben sich für Quellstärken eine Reihe von Anwendungsmöglichkeiten in der Lebensmittelindustrie und außerdem in vielen technischen Bereichen. Die Verwendung von Quellstärke, die auch als kaltquellende Stärke bezeichnet wird, anstelle von nativer Stärke hat in verschiedenen Fällen den Vorteil, dass Produktionsverfahren vereinfacht und verkürzt werden können.
Für die Herstellung beispielsweise von Instant-Desserts und -Puddings werden Quellstärken benötigt, die nach dem Einrühren in kalte Flüssigkeit, wie z.B. Wasser oder Milch, innerhalb kurzer Zeit schnittfeste Gele bilden, wie beispielsweise im Falle eines Kochpuddings. Diese Anforderungen erfüllen die kommerziellen Quellstärken aus Weizen-, Kartoffel- oder Maisstärke nicht. Zur Erzielung der vorgenannten Eigenschaften sind bei den bisher kommerziell verfügbaren Quellstärken Zusätze zur Quellstärke wie Gelatine, Alginat, Carrageenan (Carrageen) und/oder anorganische Salze notwendig. Dieses Zusetzen von sogenannten Hilfsstoffen ist z.B. nach Herstellung von Quellstärken mit erfindungsgemäßen Stärken, isoliert aus erfindungsgemäßen Pflanzenzellen nicht in gleichem Umfang notwendig.

Im Vergleich zu Gelen, die mit Hilfe herkömmlicher Hochamylosemaisstärken hergestellt werden, weisen die erfindungsgemäßen Gele den Vorteil auf, dass zu ihrer Herstellung weniger Energie aufgebracht und keine aufwendige Prozesstechnik zur Erzeugung hoher Temperaturen/Drücke eingesetzt werden muss, da die erfindungsgemäßen Stärken bereits unter Normalbedingungen in Wasser verkleistern.

Eine wichtige funktionelle Eigenschaft, z.B. bei der Verarbeitung von Stärken in der Nahrungsmittelindustrie, stellt das Quellvermögen dar.

Es ist zu unterscheiden zwischen dem Quellvermögen von Stärke in kaltem Wasser (z.B. Raumtemperatur) und Quellvermögen in warmem bzw. heißem Wasser.
Native Stärken weisen, wenn überhaupt, in kaltem Wasser ein vernachlässigbares Quellvermögen auf, während ausgewählte physikalisch modifizierte (vorverkleisterte, getrocknete) Stärken bereits in kaltem Wasser quellen können.

Im Zusammenhang mit der vorliegenden Erfindung bezieht sich der Begriff "Quellvermögen" auf das Verhalten von Stärke in heißen wässrigen Suspensionen, vorzugsweise bei einer Temperatur von 90°C. Im Zusammenhang mit der vorliegenden Erfindung wird das Quellvermögen vorzugsweise mit einer 3% (w/v)-igen Stärkesuspension bei einer Temperatur von 90°C nach der Methode von Leach et al. (Cereal Chemistry 36, (1959), 534-544) bestimmt. Das Quellvermögen wird ermittelt als Quotient aus dem Gewicht des Gels geteilt durch die Differenz aus Einwaage der Stärkeprobe und den löslichen Bestandteilen:

$$\text{Quellvermögen (g/g)} = \text{Gewicht des Gels} / (\text{Gewicht der eingewogene Stärke} - \text{lösliche Bestandteile})$$

Handelsübliche Hochamylose-Mais-Stärke, wie z.B. Hylon VII, weist im Vergleich zu Mais-Wildtypstärke ein deutlich verringertes Quellvermögen auf. Für Hylon VII - Stärke mit einem apparenten Amylosegehalt von ca. 70% wurde beispielsweise nach der Methode von Leach et al. (Cereal Chemistry 36, (1959), 534-544) ein Quellvermögen von ca. 6 g/g ermittelt, wohingegen Mais-Wildtyp-Stärke ein Quellvermögen von ca. 22 g/g zeigt (Shi et al., Journal of Cereal Science 27, (1998), 289-299).

Die erfindungsgemäße Mais-Stärke weist gegenüber herkömmlichen Hochamylose-Mais-Stärken den Vorteil eines wesentlich verbesserten Quellvermögens auf. In einer weiteren Ausführungsform weist die erfindungsgemäße Mais-Stärke ein Quellvermögen von mindestens 7 g/g, vorzugsweise von mindestens 10 g/g und besonders bevorzugt von mindestens 12 g/g auf.

In einer weiteren Ausführungsform weist die erfindungsgemäße Mais-Stärke ein Quellvermögen zwischen 7 g/g und 20 g/g, vorzugsweise zwischen 10 g/g und 18 g/g, besonders bevorzugt zwischen 11 g/g und 15 g/g auf.
Aufgrund des erhöhten Quellvermögens gegenüber herkömmlichen Hochamylose-Mais-Stärken sind die erfindungsgemäßen Mais-Stärken für bestimmte Anwendungen besser geeignet. Wird Stärke z.B. als Dickungsmittel eingesetzt, so führt das erhöhte Quellvermögen der Stärke dazu, dass deutlich weniger Stärke eingesetzt werden muss, um eine gleiche Dickungsleistung zu erzielen. Dieses hat zur Folge, dass z.B. der Kaloriengehalt von mit Stärke angedickten Lebensmitteln reduziert werden kann.

In einer weiteren Ausführungsform weisen die erfindungsgemäßen Mais-Stärken eine gegenüber nativen Hochamylose-Mais-Stärken erhöhte Heißwasser-Löslichkeit auf. Die Heißwasser-Löslichkeit der erfindungsgemäßen Mais-Stärken beträgt mindestens 8%, vorzugsweise mindestens 9% und besonders bevorzugt mindestens 11%.

In einer weiteren Ausführungsform weisen die erfindungsgemäßen Mais-Stärken eine Heißwasser-Löslichkeit zwischen 8% und 25%, vorzugsweise zwischen 9% und 20% und besonders bevorzugt zwischen 10% und 16% auf.

Im Zusammenhang mit der vorliegenden Erfindung erfolgt die Bestimmung der Heißwasser-Löslichkeit vorzugsweise mit einer 3% (w/v)-igen Stärkesuspension bei 90°C nach der Methode von Leach et al. (Cereal Chemistry 36, (1959), 534-544), die auch untenstehend ("Allgemeine Methoden") beschrieben ist. Die Heißwasser-Löslichkeit ergibt sich gemäß folgender Formel:

$$\text{Heißwasser-Löslichkeit (\%)} = \text{Gewicht getrockneter Überstand} / \text{Gewicht der eingewogenen Stärke} \times 100$$

In einer weiteren Ausführungsform sind die erfindungsgemäßen Mais-Stärken dadurch gekennzeichnet, dass sie einen Phosphatgehalt in C6-Position zwischen 10 und 40 nmol C6P/mg Stärke (Trockengewicht), vorzugsweise zwischen 12 und 35 nmol C6P/mg Stärke (Trockengewicht) und besonders bevorzugt zwischen 15 und 30 nmol C6P/mg Stärke (Trockengewicht) aufweisen.

Unter dem Begriff "Phosphatgehalt in C6-Position" ist im Zusammenhang mit der vorliegenden Erfindung der Gehalt an Phosphatgruppen zu verstehen, die an Kohlenstoffatomposition "6" der Glukosemonomere der Stärke gebunden sind. Grundsätzlich können in der Stärke in vivo die Positionen C3 und C6 der Glukoseeinheiten phosphoryliert sein. Die Bestimmung des Phosphatgehaltes in C6-Position (= C-6-P-Gehalt) erfolgt im Zusammenhang mit der vorliegenden Erfindung vorzugsweise über eine Glukose-6-phosphat-Bestimmung mittels des weiter unten beschriebenen optisch-enzymatischen Tests (nach Nielsen et al., 1994, Plant Physiol. 105, 111-117).

Gegenstand der vorliegenden Erfindung ist auch die von den erfindungsgemäßen Pflanzenzellen bzw. den erfindungsgemäßen Pflanzen hergestellte Mais- bzw. Weizen-Stärke mit einer oder mehreren der vorstehend beschriebenen Eigenschaften. D.h., diese Erfindungsbeschreibung offenbart jegliche Kombination der folgenden Stärkeeigenschaften: apparenter Amylosegehalt, Phosphatgehalt in C6-Position, Verkleisterungstemperatur, Peakviskosität, Trough-Viskosität, Endviskosität, Breakdown, Setback, Quellvermögen, Heißwasser-Löslichkeit, Gelfestigkeit. Als offenbart anzusehen sind alle Kombinationen aus zwei, drei, vier, fünf, sechs, sieben, acht, neun und allen Eigenschaften.

Dem Fachmann ist bekannt, dass die Eigenschaften von Stärke durch z.B. thermische, chemische, enzymatische oder mechanische Derivatisierung verändert werden können. Derivatisierte Stärken sind für verschiedene Anwendungen im Nahrungsmittel- und/oder Nicht-Nahrungsmittelbereich besonders geeignet. Die erfindungsgemäßen Stärken sind als Ausgangssubstanz besser geeignet zur Herstellung von derivatisierten Stärken als herkömmliche Hochamylose-Stärken, da sie z.B. durch den höheren Gehalt an Stärkephosphat einen höheren Anteil an reaktiven funktionalen Gruppen aufweisen und darüber hinaus leichter verkleistern.

Die vorliegende Erfindung betrifft daher auch Verfahren zur Herstellung einer derivatisierten Mais- bzw. Weizenstärke, worin erfindungsgemäße native Mais- bzw. Weizenstärke nachträglich derivatisiert wird. Weiterhin betrifft die vorliegende Erfindung auch derivatisierte Mais- bzw. Weizenstärke enthaltend die erfindungsgemäße Mais- bzw. Weizenstärke.

Unter dem Begriff "derivatisierte Stärke" soll im Zusammenhang mit der vorliegenden Erfindung eine erfindungsgemäße Mais- bzw. Weizenstärke verstanden werden, deren Eigenschaften nach der Isolierung aus den erfindungsgemäßen Mais- bzw. Weizenpflanzen(zellen) oder deren Vermehrungsmaterial mit Hilfe von chemischen, enzymatischen, thermischen oder mechanischen Verfahren verändert wurde.

In einer weiteren Ausführungsform der vorliegenden Erfindung handelt es sich bei der erfindungsgemäßen derivatisierten Mais- bzw. Weizenstärke um mit Hitze und/oder mit Säure behandelte Stärke.

In einer weiteren Ausführungsform handelt es sich bei den erfindungsgemäßen derivatisierten Mais- bzw. Weizenstärke um Stärkeether, insbesondere um Stärke-Alkylether, O-Allylether, Hydroxylalkylether, O-Carboxylmethylether, stickstoffhaltige Stärkeether, phosphathaltige Stärkeether oder schwefelhaltige Stärkeether.

In einer weiteren Ausführungsform handelt es sich bei den erfindungsgemäßen derivatisierten Mais- bzw. Weizenstärken um vernetzte Stärken.

In einer weiteren Ausführungsform handelt es sich bei den erfindungsgemäßen derivatisierten Mais- bzw. Weizenstärken um Stärke-Pfropf-Polymerisate.

In einer weiteren Ausführungsform handelt es sich bei den erfindungsgemäßen derivatisierten Mais- bzw. Weizenstärken um oxidierte Stärken.

In einer weiteren Ausführungsform handelt es sich bei den erfindungsgemäßen derivatisierten Mais- bzw. Weizenstärken um Stärkeester, insbesondere um Stärkeester, die unter Verwendung von organischen Säuren in die Stärke eingeführt wurden. Besonders bevorzugt handelt es sich um so genannte Phosphat-, Nitrat-, Sulfat-, Xanthat-, Acetat- oder Citratstärken.

Die erfindungsgemäßen derivatisierten Mais- bzw. Weizenstärken eignen sich für verschiedene Verwendungen in der Pharmaindustrie, im Nahrungsmittel- und/oder Nicht-Nahrungsmittelbereich. Methoden zur Herstellung von erfindungsgemäßen derivatisierten Mais- bzw. Weizenstärken sind dem Fachmann bekannt und in der allgemeinen Literatur beschrieben. Eine Übersicht zur Herstellung von derivatisierten Stärken findet sich z.B. bei Orthoefer (in Corn, Chemistry and Technology, 1987, eds. Watson und Ramstad, Chapter 16, 479-499).

Derivatisierte Stärke, erhältlich nach dem erfindungsgemäßen Verfahren zur Herstellung einer derivatisierten Stärke ist ebenfalls Gegenstand der vorliegenden Erfindung.

Ferner ist die Verwendung erfindungsgemäßer modifizierter Stärken zur Herstellung von derivatisierter Stärke Gegenstand der vorliegenden Erfindung.

Gegenstand der vorliegenden Erfindung sind weiterhin Mais-Pflanze(nzellen), die ein heterologes GWD-Gen exprimieren und eine amylose extender-Mutation aufweisen.

Die vorliegende Erfindung betrifft auch Mais-Pflanzen, die die erfindungsgemäßen Mais-Pflanzenzellen enthalten.

In einer bevorzugten Ausführungsform synthetisieren die erfindungsgemäßen Mais-Pflanzen(zellen) die erfindungsgemäße Maisstärke.

In einer weiteren bevorzugten Ausführungsform sind die erfindungsgemäßen Mais-Pflanzen(zellen) transgene Mais-Pflanzen(zellen), die ein Transgen enthalten, das zu einer nachweisbaren Expression eines heterologen GWD-Gens führt.

Die vorliegende Erfindung betrifft ferner Weizen-Pflanzen(zellen), die ein heterologes GWD-Gen exprimieren und eine amylose extender-Mutation aufweisen. In einer bevorzugten Ausführungsform synthetisieren die erfindungsgemäßen Weizen-Pflanzen(zellen) die erfindungsgemäße Weizenstärke.

Die vorliegende Erfindung betrifft auch Weizen-Pflanzen, die die erfindungsgemäßen Weizen-Pflanzenzellen enthalten.

In einer weiteren bevorzugten Ausführungsform sind die erfindungsgemäßen Weizen-Pflanzen(zellen) transgene Weizen-Pflanzen(zellen), die ein Transgen enthalten, das zu einer nachweisbaren Expression eines heterologen GWD-Gens führt.

Im Zusammenhang mit der vorliegenden Erfindung soll unter einem GWD-Protein ein Protein mit der enzymatischen Aktivität einer alpha-Glucan-Wasser-Dikinase (GWD, E.C.: 2.7.9.4) (Ritte et al., 2002, PNAS 99, 7166-7171) verstanden werden, das insbesondere in der älteren wissenschaftlichen Literatur (z.B. WO9711188, WO9827212) häufig auch als R1-Protein bezeichnet wurde. In der von GWD katalysierten Reaktion werden die Edukte alpha-1,4-Glucan bzw. Stärke, Adenosintriphosphat (ATP) und Wasser zu den Produkten Glucan-Phosphat (Stärkephosphat), Monophosphat und Adenosinmonophosphat umgesetzt (Kötting et al., 2005, Plant Physiol. 137, 2424-252, Ritte et al., 2002, PNAS 99, 7166-7171). Dabei wird ein beta-Phosphatrest von ATP auf Stärke übertragen.

GWD kann nicht phosphorylierte Stärke als Substrat verwenden, d.h. eine *de novo* Phosphorylierung von nicht phosphorylierter Stärke kann durch GWD katalysiert werden (Kötting et al., 2005, Plant Physiol. 137, 2424-252, Baunsgaard et al., 2005, Plant Journal 41, 595-605). GWD kann Phosphatgruppen in C6-Position der Glucosemonomere von Stärke einführen.

Man geht davon aus, dass bei der Katalyse der Phosphorylierungsreaktion einer Stärke durch ein Protein mit der Aktivität einer Glucan-Wasser-Dikinase als Zwischenprodukt ein phosphoryliertes Protein entsteht, bei welchem der beta-Phosphatrest des ATP kovalent an eine Aminosäure des Proteins mit der Aktivität einer Glucan-Wasser-Dikinase gebunden ist (Ritte et al., 2002, PNAS 99, 7166-7171). Das Zwischenprodukt entsteht durch Autophosphorylierung des Proteins mit der Aktivität einer Glucan-Wasser-Dikinase, d.h. das Protein mit der Aktivität einer Glucan-Wasser-Dikinase selbst katalysiert die Reaktion, die zu dem Zwischenprodukt führt (Ritte et al., 2002, PNAS 99, 7166-7171).

In Kartoffelpflanzen liegt das GWD-(R1)-Protein an die Stärkekörner der Speicherstärke in Kartoffelknollen gebunden vor (Lorberth et al., 1998, Nature Biotechnology 16, 473-477).

Stärken, isoliert aus Blättern einer *Arabidopsis thaliana sex1-3* Mutante weisen keine nachweisbaren Mengen an kovalent gebundenen Phosphatresten auf, werden jedoch von einem Protein mit der Aktivität einer Glucan-Wasser-Dikinase phosphoryliert. D.h. nicht-phosphorylierte-Stärke, z.B. isoliert aus Blättern einer *Arabidopsis thaliana sex1-3* Mutante, wird in einer durch ein Protein mit der Aktivität einer Glucan-Wasser-Dikinase katalysierten Phosphorylierungsreaktion als Substrat verwendet (Ritte et al., Planta 216, (2003), 798-801).

Aminosäuresequenzen, die Proteine mit der Aktivität einer Glucan-Wasser-Dikinase codieren, enthalten eine Phosphohistidindomäne. Phosphohistidindomänen sind z.B. beschrieben bei Tien-Shin Yu et al. (2001, Plant Cell 13, 1907-1918). Bei der Autophosphorylierung eines Proteins mit der Aktivität einer Glucan-Wasser-Dikinase wird ein Histidinrest in der Phosphohistidindomäne der Aminosäuresequenz, codierend ein Protein mit der Aktivität einer Glucan-Wasser-Dikinase, phosphoryliert (Mikkelsen et al., 2004, Biochemical Journal 377, 525-532). In der unter SEQ ID NO 2 dargestellten Proteinsequenz eines Proteins mit der Aktivität einer Glucan-Wasser-Dikinase aus *Solanum tuberosum* stellen beispielsweise die Aminosäuren 1064 bis 1075 die Phosphohistidindomäne dar. Wird der konservierte Histidinrest (in der unter SEQ ID NO 2 dargestellten Proteinsequenz beispielsweise die Aminosäure 1069) der Phosphohistidindomäne durch eine andere Aminosäure ersetzt, findet keine Autophosphorylierung und damit auch keine Phosphorylierung von Glucanen durch das mutagenisierte Protein mehr statt (Mikkelsen et al., 2004, Biochemical Journal 377, 525-532). Weiterhin zeichnet sich ein Protein mit der Aktivität einer Glucan-Wasser-Dikinase dadurch aus, dass es eine C-terminale Nukleotidbindedomäne aufweist, die in der unter SEQ ID NO. 2 als Beispiel dargestellten Aminosäuresequenz von den Aminosäuren 1121 bis 1464 umfasst wird. Eine Deletion der Nukleotidbindedomäne führt zur Inaktivierung eines Proteins mit der Aktivität einer Glucan-Wasser-Dikinase (Mikkelsen und Blennow, 2005, Biochemical Journal 385, 355-361).

Am N-Terminus weisen Proteine mit der Aktivität einer Glucan-Wasser-Dikinase eine Kohlenhydratbindedomäne auf, die auch als Carbohydrate Binding Module (kurz: CBM) bezeichnet wird, und die in der unter SEQ ID NO 2 dargestellten Aminosäuresequenz von den Aminosäuren 78 bis 362 umfasst wird. Kohlenhydratbindedomänen zeichnen sich u.a. dadurch aus, dass ihre Aminosäuresequenz konservierte Tryptophanreste aufweisen. Werden diese konservierten Aminosäurereste gegen andere Aminosäuren ausgetauscht, so können die Kohlenhydratbindedomänen ihre Fähigkeit verlieren, Glucane zu binden. So führt z.B. ein Austausch der Aminosäuren W139 oder W194 in der unter SEQ ID NO. 2 dargestellten Aminosäuresequenz zu einem Verlust der Funktion dieser Kohlenhydratbindedomäne. Wird die Kohlenhydratbindedomäne einer Glucan-Wasser-Dikinase deletiert (beispielsweise eine Deletion der Aminosäuren 1 bis 362 der unter SEQ ID NO. 2 dargestellten Aminosäuresequenz, wobei die Aminosäuren 1 bis 77 ein plastidäres Signalpeptid darstellen), führt dies jedoch nicht zur Inaktivierung der phosphorylierenden Aktivität des Enzyms (Mikkelsen et al., 2006, Biochemistry 45,4674-4682).

Nukleinsäuresequenzen und zu diesen korrespondierende Aminosäuresequenzen, codierend ein Protein mit der Aktivität einer Glucan-Wasser-Dikinase sind aus unterschiedlichen Spezies, wie z.B. Kartoffel (WO9711188, GenBank Acc.: AY027522.1, Y09533.1), Weizen (WO0077229, US 6,462,256, GenBank Acc.: AAN93923.1, GenBank Acc.: AR236165.1), Reis (GenBank Acc.: AAR61445.1, US 6620987, GenBank Acc.: AR400814.1), Mais (GenBank Acc.: AAR61444.1, GenBank Acc.: AR400813.1; WO9827212), Soyabohne (GenBank Acc.: AAR61446.1, GenBank Acc.: AR400815.1), *Curcuma longa* (SEQ ID NOs 3 und 4), Citrus (GenBank Acc.: AY094062.1), *Arabidopsis* (GenBank Acc.: AF312027.1), der Grünalge *Ostreococcus tauri* (GenBank Acc.: AY570720.1) und aus Chlamydomonas reinhardtii (US60/701,693, WO2007/018770) beschrieben. Eine synthetische, für die Expression in Mais optimierte GWD-Nukleotidsequenz wurde unter SEQ ID No.1 der WO2005/002359 (korrespondiert zu US2006/0282917 A1) beschrieben.

Vorzugsweise sind im Zusammenhang mit der vorliegenden Erfindung Nukleinsäuremoleküle einzusetzen, die die gesamte codierende Region einer GWD umfassen. Die genannten Nukleinsäuresequenzen und Aminosäuresequenzen codierend ein Protein mit der Aktivität einer Glucan-Wasser-Dikinase sind u.a. veröffentlicht vom NCBI (http://www.ncbi.nlm.nih.gov/entrez/) und sind durch Nennung der Referenzen ausdrücklich in die Beschreibung der vorliegenden Anmeldung aufgenommen.

Im Zusammenhang mit der vorliegenden Erfindung können neben den hier aufgezählten GWD-Genen auch GWD-Gene aus anderen Organismen oder synthetische GWD-Gene eingesetzt werden. Vorzugsweise stammt das GWD-Gen aus *Solanum tuberosum* oder *Curcuma longa* und weist eine Sequenzidentität von mindestens 50%, insbesondere mindestens 60%, vorzugsweise mindestens 70%, besonders bevorzugt von mindestens 80% und ganz besonders bevorzugt von mindestens 90% zu der codierenden Region des unter SEQ ID No. 1 (*Solanum tuberosum*) oder unter SEQ ID No. 3 (Curcuma longa) angebenen Nukleinsäuremoleküls auf. Weiterhin bevorzugt ist die codierende Region der unter SEQ ID No. 1 angegebenen Nukleotidsequenz, besonders bevorzugt die codierende Region der unter SEQ ID No. 3 angegebenen Nukleotidsequenz.

In einer bevorzugten Ausführungsform wird im Zusammenhang mit den erfindungsgemäßen Mais- bzw. Weizen-Pflanzen(zellen) eine GWD der Gattung Curcuma oder Solanum, vorzugsweise der Art *Curcuma longa* oder *Solanum tuberosum* verwendet.

Bevorzugt ist die GWD mit der unter SEQ ID No. 2 angegebenen Aminosäuresequenz, besonders bevorzugt die GWD mit der unter SEQ ID No. 4 angegebenen Aminosäuresequenz.

Unter dem Begriff "Identität" soll im Zusammenhang mit der vorliegenden Erfindung die Anzahl der übereinstimmenden Aminosäuren/Nukleotide (Identität) mit anderen Proteinen/Nukleinsäuren, ausgedrückt in Prozent verstanden werden. Bevorzugt wird die Identität eines Proteins, beispielsweise mit der Aktivität einer GWD durch Vergleich mit der unter SEQ ID NO 2 oder SEQ ID No. 4 angegebenen Aminosäuresequenz bzw. die Identität eines Nukleinsäuremoleküls kodierend ein Protein mit der Aktivität einer GWD durch Vergleich mit der unter SEQ ID NO. 1 oder SEQ ID No. 3 angegebenen kodierenden Nukleinsäuresequenz mit anderen Proteinen/Nukleinsäuren durch Computerprogramme ermittelt. Weisen Sequenzen, die miteinander verglichen werden, unterschiedliche Längen auf, ist die Identität so zu ermitteln, dass die Anzahl an Aminosäuren/Nukleotiden, welche die kürzere Sequenz mit der längeren Sequenz gemeinsam hat, den prozentualen Anteil der Identität bestimmt. Vorzugsweise wird die Identität mittels des bekannten und der Öffentlichkeit zur Verfügung stehenden Computerprogramms ClustalW (Thompson et al., Nukleic Acids Research 22 (1994), 4673-4680) ermittelt. ClustalW wird öffentlich zur Verfügung gestellt von Julie Thompson (Thompson@EMBL-Heidelberg.DE) und Toby Gibson (Gibson@EMBL-Heidelberg.DE), European Molecular Biology Laboratory, Meyerhofstrasse 1, D 69117 Heidelberg, Germany. ClustalW kann ebenfalls von verschiedenen Internetseiten, u.a. beim IGBMC (Institut de Genetique et de Biologie Moleculaire et Cellulaire, B.P.163, 67404 Illkirch Cedex, France; ftp://ftp-igbmc.u-strasbg.fr/pub/) und beim EBI (ftp://ftp.ebi.ac.uk/pub/software/) sowie bei allen gespiegelten Internetseiten des EBI (European Bioinformatics Institute, Wellcome Trust Genome Campus, Hinxton, Cambridge CB10 1SD, UK) heruntergeladen werden.
Vorzugsweise wird das ClustalW Computerprogramm der Version 1.8 benutzt, um die Identität zwischen im Rahmen der vorliegenden Erfindung beschriebenen Proteinen und anderen Proteinen zu bestimmen. Dabei sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP.
Vorzugsweise wird das ClustalW Computerprogramm der Version 1.8 benutzt, um die Identität zwischen z.B. der Nukleotidsequenz der im Rahmen der vorliegenden Erfindung beschriebenen Nukleinsäuremoleküle und der Nukleotidsequenz von anderen Nukleinsäuremolekülen zu bestimmen. Dabei sind folgende Parameter einzustellen: KTUPLE=2, TOPDIAGS=4, PAIRGAP=5, DNAMATRIX:IUB, GAPOPEN=10, GAPEXT=5, MAXDIV=40, TRANSITIONS: unweighted.

Unter einem "heterologen" GWD-Gen bzw. -Protein ist im Zusammenhang mit der vorliegenden Erfindung eine GWD zu verstehen, die natürlicherweise nicht in der Mais- bzw. Weizen-Pflanze(nzelle) vorkommt, sondern deren kodierende DNA-Sequenz beispielsweise mittels gentechnischer Methoden, wie z.B. Transformation der Zelle, in die Zelle eingeführt wird. Dabei kann die kodierende DNA-Sequenz der heterologen GWD beispielsweise aus einer anderen Maissorte stammen als die transformierte Mais-Pflanzenzelle bzw. aus einer anderen Weizensorte als die transformierte Weizen-Pflanzenzelle. Sie steht vorzugsweise nicht unter der Kontrolle ihres eigenen Promotors, sondern unter Kontrolle eines in Bezug auf das jeweilige GWD-Gen heterologen Promotors. Vorzugsweise stammt die heterologe GWD aus einer anderen Pflanzenart als die transformierte erfindungsgemäße Mais-Pflanzenzelle/Mais-Pflanze bzw. Weizen-Pflanzenzelle/Weizen-Pflanze oder die eingesetzte GWD steht unter der Kontrolle eines heterologen Promotors, d.h. eines Promotors, der nicht dem natürlichen GWD-Promotor der jeweiligen GWD entspricht. Besonders bevorzugt stammt die kodierende DNA-Sequenz der heterologen GWD aus einer anderen Pflanzengattung als die transformierte Mais-Pflanzenzelle/Mais-Pflanze bzw. als die transformierte Weizen-Pflanzenzelle/Weizen-Pflanze.

Unter dem Begriff "Pflanzengattung" ist im Zusammenhang mit der vorliegenden Erfindung eine hierarchische Stufe der biologischen Systematik zu verstehen. Eine Gattung enthält eine oder mehrere Arten. Ein Beispiel einer Gattung ist *Triticum* L. (Weizen). Alle Arten innerhalb einer Gattung haben immer einen zweiteiligen (binominalen) Namen, der neben der Gattungsbezeichnung noch ein Art-Epipheton enthält. *Triticum aestivum* L. (der Weichweizen) ist danach eine Art der Gattung *Triticum.*

Unter dem Begriff Mais-Pflanzen(zellen), die eine "amylose extender-Mutation" aufweisen, versteht man im Zusammenhang mit der vorliegenden Erfindung Maispflanzen(zellen), die eine Mutation des Gens des Stärke-Verzweigungsenzym IIb aus Mais (= engl. Starch Branching Enzyme, Abkürzung "BE IIb" oder "SBE IIb") aufweisen, das auch als amylose extender - Gen bezeichnet wird, wobei diese Mutation zu einer Verringerung der SBE IIb - Enzymaktivität im Endosperm dieser Maispflanzen führt im Vergleich zur BE IIb - Aktivität im Endosperm von Mais-Wildtyppflanzen.

Vorzugsweise führt diese Mutation des BE IIb in Mais-Pflanzen(zellen) dazu, dass keine SBE IIb - Aktivität mehr nachweisbar ist (z.B. Fisher et al., Plant Physiol. 110, (1996), 611-619, insbesondere Figur 4; Hedman und Boyer, Biochemical Genetics 21 (11/12), (1983), 1217-1222, insbesondere Tabelle 1).

Unter dem BE IIb - Protein aus Mais soll im Zusammenhang mit der vorliegenden Erfindung ein Verzweigungsenzym der Isoform IIb verstanden werden, das durch das sogenannte amylose extender Gen kodiert wird (Kim et al., Plant Molecular Biology 38, (1998), 945-956). Das Verzweigungsenzym ("Branching Enzyme" = BE) der Isoform IIb ($\alpha$-1,4-Glukan: $\alpha$-1,4-Glukan 6-Glycosyltransferase; E.C. 2.4.1.18) katalysiert eine Transglycosylierungsreaktion, in der $\alpha$-1,4-Verknüpfungen eines $\alpha$-1,4-Glukandonors hydrolysiert und die dabei freigesetzten $\alpha$-1,4-Glukanketten auf eine $\alpha$-1,4-Glukanakzeptorkette transferiert und dabei in $\alpha$-1,6-Verknüpfungen überführt werden.

In seinen biochemischen Eigenschaften unterscheidet sich das BEIIb-Protein aus Mais deutlich vom BEI-Protein aus Mais, die von Fisher et al. (Plant Physiol. 110, (1996), 611-619) in Tabelle 1, Seite 612 zusammengefasst sind. Beispielsweise verzweigt das BEI-Protein Amylose schneller als das BEllb-Protein, wohingegen das BEIIb-Protein Amylopektin mit einer höheren Rate verzweigt als es das BEI-Protein tut (Guan und Preiss, Plant Physiol. 102, (1993), 1269-1273). Die Aminosäuresequenz des BEllb-Protein unterscheidet sich vom BElla-Protein laut Gao et al. (Plant Physiol. 114, (1997), 69-78) vor allem durch eine 49 Aminosäuren lange N-terminale Extension des BElla-Proteins. Das Molekulargewicht des BElla-Proteins bestimmt mittels SDS-PAGE beträgt 89 kD, das des BEllb-Proteins etwas weniger, nämlich 85 kDa (Fisher et al., Plant Physiol. 110, (1996), 611-619).

Die Aminosäuresequenz des BEIlb-Proteins aus Mais weist eine Identität von mindestens 85%, bevorzugt von mindestens 90%, von mindestens 95% und besonders bevorzugt von mindestens 98% mit der unter SEQ ID NO 6 dargestellten Aminosäuresequenz auf.

Unter einem "amylose extender"-Gen (auch "BEIIb"-Gen) aus Mais soll im Zusammenhang mit der vorliegenden Erfindung ein Gen verstanden werden, das ein BEIIb-Protein kodiert. Die Nukleinsäuresequenz des amylose extender-Gens aus Mais weist eine Identität von mindestens 85%, bevorzugt von mindestens 90%, von mindestens 95% und besonders bevorzugt von mindestens 98% mit der unter SEQ ID NO. 5 dargestellten Nukleinsäuresequenz auf.

In einer weiteren Aufführungsform der vorliegenden Erfindung handelt es sich bei der "amylose extender-Mutation" der erfindungsgemäßen Mais-Pflanze(nzelle) um eine dominante Mutation des amylose extender 1 - Lokus, die zur Synthese einer Maisstärke führt, die einen im Vergleich zu Mais-Wildtyp-Pflanzen(-zellen) erhöhten apparenten Amylosegehalt aufweist, der zwischen 35 und 90 Gew.-%, insbesondere zwischen 38-85 Gew.-%, vorzugsweise von 40-80 Gew.-% und besonders bevorzugt zwischen 42-75 Gew.-% aufweist.

Vorzugsweise handelt es sich bei der dominanten Mutation um das Mu-induzierte Allel Ae1-5180 des amylose extender1-Lokus (Stinard et al., Plant Cell 5, (1993), 1555-1566).

In einer besonders bevorzugten Ausführungsform der vorliegenden Erfindung handelt es sich bei der "amylose extender-Mutation" der erfindungsgemäßen Mais-Pflanze(nzelle) um eine Mais-Pflanze(nzelle), die einen homozygot rezessiven "amylose extender"-Genotyp aufweist und eine Maisstärke synthetisiert, die einen apparenten Amylosegehalt zwischen 38-85 Gew.-%, vorzugsweise zwischen 40-80 Gew.-% und besonders bevorzugt zwischen 42-75 Gew.-% aufweist. Der amylose extender 1-(ae1)-Lokus umfasst das Strukturgen, welches das SBE IIb - Protein kodiert (Hedman und Boyer, Biochemical Genetics 20 (5/6), (1982), 483-492).

Amylose extender (ae) - Maismutanten wurden beispielsweise beschrieben bei Vineyard and Bear (Maize Genet Coop Newsletter 26: 5 (1952), die das Referenz-Allel ae1-Ref beschreiben sowie bei Moore und Creech (Genetics 70, (1972), 611-619), Garwood et al. (Cereal Chemistry 53(3), (1976), 355-364) und Hedman und Boyer (Biochemical Genetics 21 (11/12), (1983), 1217-1222).

Unter dem Begriff Weizen-Pflanzen(zellen), die eine "amylose extender-Mutation" aufweisen, versteht man im Zusammenhang mit der vorliegenden Erfindung Weizenpflanzen(zellen), die eine Mutation des Gens des Stärke-Verzweigungsenzyms IIa oder des Stärke-Verzweigungsenzyms IIa (BE IIa-Protein) und des Stärke-Verzweigungsenzyms IIb (BE-IIb-Protein) aus Weizen (= engl. Starch Branching Enzyme, Abkürzung "BE IIa", "BE IIb", "SBE IIa" oder "SBE IIb") aufweisen, wobei diese Mutation zu einer Verringerung der BE IIa- oder diese Mutationen zur Verringerung der BE IIa- und der BE IIb - Enzymaktivität im Endosperm dieser Weizenpflanzen führt/führen im Vergleich zur BE IIa - bzw. zur BElla- und zur BE IIb - Aktivität im Endosperm von Weizen-Wildtyppflanzen und zur Synthese einer Weizenstärke mit einem apparenten Amylosegehalt zwischen 38-85 Gew.-%, vorzugsweise zwischen 40-80 Gew.-% und besonders bevorzugt zwischen 42-75 Gew.-%.

Unter dem BE IIa - Protein aus Weizen soll im Zusammenhang mit der vorliegenden Erfindung ein Verzweigungsenzym der Isoform IIa aus Weizen verstanden werden (Rahman et al., Plant Physiology 125, (2001), 1314-1324), das durch ein BE IIa-Gen aus Weizen kodiert wird. Das Verzweigungsenzym ("Branching Enzyme" = BE) der Isoform IIb ($\alpha$-1,4-Glukan: $\alpha$-1,4-Glukan 6-Glycosyltransferase; E.C. 2.4.1.18) katalysiert eine Transglycosylierungsreaktion, in der $\alpha$-1,4-Verknüpfungen eines $\alpha$-1,4-Glukandonors hydrolysiert und die dabei freigesetzten $\alpha$-1,4-Glukanketten auf eine $\alpha$-1,4-Glukanakzeptorkette transferiert und dabei in $\alpha$-1,6-Verknüpfungen überführt werden.

Die Aminosäuresequenz des BElla-Proteins aus Weizen weist eine Identität von mindestens 85%, bevorzugt von mindestens 90%, von mindestens 95% und besonders bevorzugt von mindestens 98% mit der unter SEQ ID NO 8 dargestellten Aminosäuresequenz auf.

Unter einem "BE IIa-Gen aus Weizen" soll im Zusammenhang mit der vorliegenden Erfindung ein Gen verstanden werden, das ein BElla-Protein aus Weizen kodiert. Die Nukleinsäuresequenz des BE IIa-Gens aus Weizen weist eine Identität von mindestens 85%, bevorzugt von mindestens 90%, von mindestens 95% und besonders bevorzugt von mindestens 98% mit der unter SEQ ID NO. 7 dargestellten Nukleinsäuresequenz auf.

Unter dem BE IIb - Protein aus Weizen soll im Zusammenhang mit der vorliegenden Erfindung ein Verzweigungsenzym der Isoform IIb aus Weizen (Regina et al., Planta 222 (5), (2005), 899-909) verstanden werden, das durch ein BE IIb-Gen aus Weizen kodiert wird. Das Verzweigungsenzym ("Branching Enzyme" = BE) der Isoform IIb ($\alpha$-1,4-Glukan: $\alpha$-1,4-Glukan 6-Glycosyltransferase; E.C. 2.4.1.18) katalysiert eine Transglycosylierungsreaktion, in der $\alpha$-1,4-Verknüpfungen eines $\alpha$-1,4-Glukandonors hydrolysiert und die dabei freigesetzten $\alpha$-1,4-Glukanketten auf eine $\alpha$-1,4-Glukanakzeptorkette transferiert und dabei in $\alpha$-1,6-Verknüpfungen überführt werden.

Die Aminosäuresequenz des BEIlb-Proteins aus Weizen weist eine Identität von mindestens 85%, bevorzugt von mindestens 90%, von mindestens 95% und besonders bevorzugt von mindestens 99% mit der unter SEQ ID NO 10 dargestellten Aminosäuresequenz auf.

Unter einem "BE IIb-Gen aus Weizen" soll im Zusammenhang mit der vorliegenden Erfindung ein Gen verstanden werden, das ein BEIIb-Protein aus Weizen kodiert. Die Nukleinsäuresequenz des BE IIb-Gens aus Weizen weist eine Identität von mindestens 85%, bevorzugt von mindestens 90%, von mindestens 95% und besonders bevorzugt von mindestens 98% mit der unter SEQ ID NO 9 dargestellten Nukleinsäuresequenz auf.

In einer weiteren Ausführungsform der vorliegenden Erfindung ist die erfindungsgemäße Mais- oder Weizen-Pflanze(nzelle), die die erfindungsgemäße Maisstärke bzw. Weizenstärke synthetisiert, genetisch modifiziert, wobei die genetische Modifikation in Bezug auf Mais zu einer nachweisbaren Expression der heterologen GWD und zu einer Verringerung der Aktivität des BEIIb-Proteins führt bzw. in Bezug auf Weizen zu einer nachweisbaren Expression der heterologen GWD und zu einer Verringerung der BE IIa- oder der BE IIa- und der BE IIb - Enzymaktivität führt im Vergleich zu entsprechenden nicht genetisch modifizierten Mais-Wildtyp-Pflanzen(zellen) bzw. im Vergleich zu entsprechenden nicht genetisch modifizierten Weizen-Wildtyp-Pflanzen(zellen).

Die genetische Modifikation kann dabei jede genetische Modifikation sein, die in Bezug auf Mais zu einer nachweisbaren Expression der heterologen GWD und zu einer Verringerung der Aktivität des BEIIb-Proteins führt im Vergleich zu entsprechenden nicht genetisch modifizierten Mais-Wildtyp-Pflanzen(zellen).

In Bezug auf Weizen kann die genetische Modifikation jede genetische Modifikation sein, die zu einer nachweisbaren Expression der heterologen GWD und zu einer Verringerung der BE IIa- oder der BE IIa- und der BE IIb - Enzymaktivität führt im Vergleich zu entsprechenden nicht genetisch modifizierten Weizen-Wildtyp-Pflanzen(zellen).

Der Begriff "nachweisbaren Expression einer heterologen GWD" bedeutet dabei im Rahmen der vorliegenden Erfindung, dass die Expression einer heterologen GWD bestimmt werden kann durch Messung der Menge an Transkripten der heterologen GWD, z.B. durch Northern-Blot-Analyse oder RT-PCR, und dass die Expression der heterologen GWD zu einer Erhöhung des Phosphatgehaltes in C6-Position der Stärke führt im Vergleich zu Stärke aus Wildtyp-Pflanzen(zellen), die keine heterologe GWD exprimieren.

Unter einer Erhöhung des Phosphatgehaltes in C6-Position der Stärke soll im Zusammenhang mit der vorliegenden Erfindung eine Erhöhung des Phosphatgehaltes in C6-Position der Stärke verstanden werden um mindestens 100%, vorzugsweise um mindestens 1000% und besonders bevorzugt um mindestens 1500% im Vergleich zum Phosphatgehalt in C6-Position der Stärke aus entsprechenden Wildtyp-Pflanzen(zellen).

In einer weiteren Ausführungsform der vorliegenden Erfindung resultiert die nachweisbare Expression einer heterologen GWD in der Erhöhung der enzymatischen GWD-Aktivität der jeweiligen erfindungsgemäßen Pflanze(nzelle) im Vergleich zur GWD-Aktivität einer entsprechenden Wildtyp-Pflanze(nzelle) und/oder in einer Erhöhung der Menge an Proteinen mit der Aktivität einer GWD in den erfindungsgemäßen Mais- oder Weizen-Pflanzen(zellen).

Die Bestimmung der Menge an GWD-Protein kann beispielsweise mit Hilfe eines Western-Blots, die Bestimmung der enzymatischen Aktivität einer GWD beispielsweise wie bei Ritte et al. beschrieben (Ritte et al., Planta 216, (2003), 798-801) bestimmt werden.

Der Begriff "Verringerung der Aktivität des BEIIb-Proteins" bedeutet im Rahmen der vorliegenden Erfindung in Bezug auf Mais eine Verringerung der Expression endogener Gene, die BEIIb-Protein kodieren und/oder eine Verringerung der Menge an BEIIb-Protein in den erfindungsgemäßen Mais-Pflanzen(zellen) und/oder vorzugsweise eine Verringerung der enzymatischen Aktivität von BEIIb-Protein in den erfindungsgemäßen Mais-Pflanzen(zellen).

Der Begriff "Verringerung der BE IIa- oder der BE IIa- und der BE IIb - Enzymaktivität" bedeutet dabei im Rahmen der vorliegenden Erfindung in Bezug auf Weizen eine Verringerung der Expression endogener Gene, die BE IIa-Protein und/oder BE IIb-Protein kodieren und/oder eine Verringerung der Menge an BEIIa-und/oder BEIIb-Protein in den erfindungsgemäßen Weizen-Pflanzen(zellen) und/oder vorzugsweise eine Verringerung der enzymatischen Aktivität von BElla- und/oder BEIIb-Protein in den erfindungsgemäßen Weizen-Pflanzen(zellen).

Die Erhöhung/Verringerung der Expression kann beispielsweise bestimmt werden durch Messung der Menge an Transkripten, die Proteine mit der Aktivität einer GWD bzw. eines BEIIb-Proteins bzw. eines BElla-Proteins kodieren. Die Bestimmung kann z.B. durch Northern-Blot-Analyse oder RT-PCR erfolgen.
Die Bestimmung der BEIIb-Aktivität von Maispflanzen erfolgt im Zusammenhang mit der vorliegenden Erfindung vorzugsweise nach Fraktionierung der verschiedenen Isoformen des Verzweigungsenzyms aus Mais, wie bei Fisher et al, Plant Physiology 110, (1996), 611-619), insbesondere wie in den dort beschriebenen Abschnitten "Reagents Used in Assays", "SBE Purification", "SBE Activity Assay" und "SBE Activity Analysis of Selected Alleles".

Die Bestimmung der BEIIa- und der BEIIb-Aktivität von Weizenpflanzen erfolgt im Zusammenhang mit der vorliegenden Erfindung vorzugsweise wie bei Tetlow et al, Plant Cell 16, (2004), 694-708) beschrieben, insbesondere wie in den dort beschriebenen Abschnitten "Enzyme Assays" und "Partial Purification of SBE Activity".

Der Nachweis der Verringerung der enzymatischen Aktivität von BElla- und/oder BEllb-Protein erfolgt im Zusammenhang mit der vorliegenden Erfindung besonders bevorzugt mit Hilfe von Aktivitätsgelen, wie untenstehend unter "Allgemeine Methoden" beschrieben.

Unter einer Verringerung der enzymatischen Aktivität von BEIIb-Proteinen soll im Zusammenhang mit der vorliegenden Erfindung in Bezug auf Mais vorzugsweise eine Verringerung der enzymatischen Aktvität verstanden werden, die zu einem apparenten Amylosegehalt zwischen 35 und 90 Gew.-%, insbesondere zwischen 38-85 Gew.-%, vorzugsweise von 40-80 Gew.-% und besonders bevorzugt zwischen 42-75 Gew.-% führt.

Unter einer "Verringerung der BE IIa- oder der BE IIa- und der BE IIb - Enzymaktivität" soll im Zusammenhang mit der vorliegenden Erfindung in Bezug auf Weizen vorzugsweise eine Verringerung der enzymatischen BElla-Aktvität oder der enzymatischen BEIIa- und BEIIb-Aktivität verstanden werden, die zu einem apparenten Amylosegehalt zwischen 35 und 90 Gew.-%, insbesondere zwischen 38-85 Gew.-%, vorzugsweise zwischen 40-80 Gew.-% und besonders bevorzugt zwischen 42-75 Gew.-% führt.

Der Begriff "Mais-Wildtyp-Pflanzenzelle" bedeutet im Zusammenhang mit der vorliegenden Erfindung, dass es sich um Mais-Pflanzenzellen handelt, die als Ausgangsmaterial für die Herstellung der erfindungsgemäßen Mais-Pflanzenzellen dienten, die die erfindungsgemäße Stärke synthetisieren. Der Begriff "Mais-Wildtyp-Pflanzenzelle" umfasst im Zusammenhang mit der vorliegenden Erfindung keine Mais-Pflanzenzellen von Maismutanten des ae-(amylose extender)-, wx- (waxy)-, du-(dull)-, sh2-(shrunken 2)-, brittle-1 oder des brittle-2- Genotyps oder von Doppel- oder Mehrfachmutanten dieser Genotypen. Der Begriff "Mais-Wildtyp-Pflanze" bedeutet im Zusammenhang mit der vorliegenden Erfindung, dass es sich um Mais-Pflanzen handelt, die als Ausgangsmaterial für die Herstellung der erfindungsgemäßen Mais-Pflanzen dienten, die die erfindungsgemäße Stärke synthetisieren. Der Begriff "Mais-Wildtyp-Pflanze" umfasst im Zusammenhang mit der vorliegenden Erfindung keine Maismutanten des ae-(amylose extender)-, wx- (waxy)-, du-(dull)-, sh2-(shrunken 2)-, brittle-1 oder des brittle-2- Genotyps oder von Doppel- oder Mehrfachmutanten dieser Genotypen.

Vorzugsweise bezieht sich der Begriff "Mais-Wildtyp-Pflanze" auf die Mais-Inzuchtlinie A188, die öffentlich verfügbar ist, beispielsweise über das Maize Genetics Cooperation Stock Center (http://maizecoop.cropsci.uiuc.edu/) bei der University of Illinois, Urbana/ Champaign, USA.

Der Begriff "Weizen-Wildtyp-Pflanzenzelle" bedeutet im Zusammenhang mit der vorliegenden Erfindung, dass es sich um Weizen-Pflanzenzellen handelt, die als Ausgangsmaterial für die Herstellung der erfindungsgemäßen Weizen-Pflanzenzellen dienten, die die erfindungsgemäße Stärke synthetisieren. Der Begriff "Weizen-Wildtyp-Pflanzenzelle" umfasst im Zusammenhang mit der vorliegenden Erfindung keine Weizen-Pflanzenzellen von Weizenmutanten, z.B. des wx- (waxy)- Genotyps. Der Begriff "Weizen-Wildtyp-Pflanze" bedeutet im Zusammenhang mit der vorliegenden Erfindung, dass es sich um Weizen-Pflanzen handelt, die als Ausgangsmaterial für die Herstellung der erfindungsgemäßen Weizen-Pflanzen dienten, die die erfindungsgemäße Weizenstärke synthetisieren. Der Begriff "Weizen-Wildtyp-Pflanzen" umfasst im Zusammenhang mit der vorliegenden Erfindung keine Weizen-Pflanzen von Weizenmutanten, z.B. des wx-(waxy)-Genotyps.

Vorzugsweise bezieht sich der Begriff "Weizen-Wildtyp-Pflanze" auf die Weizensorte FIELDER (Cl 17268), einem "soft white spring wheat", der öffentlich und frei verfügbar ist, beispielsweise über die National Small Grains Collection (NSGC; www.ars-grin.gov) (USDA, ARS, National Small Grains Research Facility, National Small Grains Collection, 1691 S 2700 W, Aberdeen, Idaho 83210; E-mail: nsgc@ars-grin.gov), einer Abteilung des United States Department of Agriculture - Agricultural Research Service (USDA-ARS; http://www.ars.usda.gov). Fielder (test number Cl 17268) ist gleichfalls verfügbar über das Alberta Stock Seed Distribution Committee, Alberta, Kanada.

Der Begriff "entsprechend" bedeutet im Zusammenhang mit der vorliegenden Erfindung, dass beim Vergleich von mehreren Gegenständen die betreffenden Gegenstände, die miteinander verglichen werden, unter gleichen Bedingungen gehalten werden. Im Zusammenhang mit der vorliegenden Erfindung bedeutet der Begriff "entsprechend" im Zusammenhang mit Mais/Weizen-Wildtyp-Pflanzenzelle oder Mais/Weizen-Wildtyp-Pflanze, dass die Pflanzenzellen oder Pflanzen, die miteinander verglichen werden, unter gleichen Kulturbedingungen aufgezogen wurden und dass sie ein gleiches (Kultur)-Alter aufweisen.

In einer weiteren Ausführungsform der vorliegenden Erfindung wird die "amylose extender-Mutation" und/oder die Erhöhung der Aktivität oder Expression einer GWD der erfindungsgemäßen Pflanzen(zellen) durch Mutagenese eines oder mehrerer Gene hervorgerufen. Die Art der Mutation ist dafür unerheblich, solange sie in Bezug auf die erfindungsgemäßen Mais-Pflanzen zu einer Verringerung der BEIIb-Aktivität und/oder zu einer Erhöhung der Expression oder Aktivität einer GWD führt, und in Bezug auf die erfindungsgemäßen Weizen-Pflanzen zu einer Verringerung der BEIIa-und/oder BEIIb-Aktivität und/oder zu einer Erhöhung der Expression oder Aktivität einer GWD führt.

Unter dem Begriff "Mutagenese" ist im Zusammenhang mit der vorliegenden Erfindung jegliche Art von eingeführten Mutationen zu verstehen, wie z.B. Deletionen, Punktmutationen (Nukleotidaustausche), Insertionen, Inversionen, Genkonversionen oder Chromosomentranslokationen.

Eine Mutation, die zur Verringerung der BEIIa- und/oder BEIIb-Aktivität und/oder zu einer Erhöhung der Expression oder Aktivität einer GWD führt, kann spontan in einer Pflanze auftreten, und die entsprechenden Pflanzen können mit Hilfe der unten beschriebenen Methoden selektiert und vermehrt werden.

Eine Mutation, die zur Verringerung der BEIIa- und/oder BEIIb-Aktivität und/oder zu einer Erhöhung der Expression oder Aktivität einer GWD führt, kann auch durch den Einsatz chemischer Agenzien oder energiereicher Strahlung (z.B. Röntgen-, Neutronen-, Gamma-, UV-Strahlung) erzeugt werden.

Agenzien, die zur Erzeugung chemisch induzierter Mutationen eingesetzt werden können und die durch Einwirkung der entsprechenden Mutagene dabei entstehenden Mutationen sind z.B. beschrieben von Ehrenberg und Husain (1981, Mutation Research 86, 1-113) und Müller (1972, Biologisches Zentralblatt 91 (1), 31-48). Die Erzeugung von Reis-Mutanten unter Verwendung von Gamma-Strahlen, Ethyl-Methan-Sulfonat (EMS), N-Methyl-N-nitrosoharnstoff oder Natriumazid ($NaN_3$) ist z.B. beschrieben von Jauhar und Siddiq (1999, Indian Journal of Genetics, 59 (1), 23-28), Rao (1977, Cytologica 42, 443-450), Gupta und Sharma (1990, Oryza 27, 217-219) und Satoh und Omura (1981, Japanese Journal of Breeding 31 (3), 316-326). Die Erzeugung von Weizen-Mutanten unter Verwendung von $NaN_3$ bzw. Maleinsäurehydrazid ist von Arora et al. (1992, Annals of Biology 8 (1), 65-69) beschrieben. Eine Übersicht zur Erzeugung von Weizen-Mutanten unter Verwendung von verschiedenen Arten energiereicher Strahlung und chemischer Agenzien ist von Scarascia-Mugnozza et al. (1993, Mutation Breeding Review 10, 1-28) dargestellt. Svec et al. (1998, Cereal Research Communications 26 (4), 391-396) beschreiben die Anwendung von N-Ethyl-N-nitrosoharnstoff zur Erzeugung von Mutanten in Triticale. Die Verwendung von MMS (Methylmethansulfonsäure) und GammaStrahlung zur Erzeugung von Hirse-Mutanten ist von Shashidhara et al. (1990, Journal of Maharashtra Agricultural Universities 15 (1), 20-23) beschrieben.

Im Zusammenhang mit der vorliegenden Erfindung können erfindungsgemäße Pflanzen(zellen) auch durch die Verwendung der so genannten Insertionsmutagenese (Übersichtsartikel: Thorneycroft et al., 2001, Journal of experimental Botany 52 (361), 1593-1601) hergestellt werden. Unter Insertionsmutagenese ist im Zusammenhang mit der vorliegenden Erfindung insbesondere das Inserieren von Transposons oder so genannter Transfer DNA (T-DNA) in ein Gen oder in die Nähe eines Gens, kodierend ein Protein mit der Aktivität einer GWD oder eines BEIIb oder eines BEIIa zu verstehen, wobei dadurch die Aktivität eines Proteins mit der Aktivität einer GWD in der betreffenden Zelle erhöht wird bzw. die Aktivität des BEIIb-Proteins oder eines BEIIa-Proteins verringert wird.

Bei den Transposons kann es sich dabei sowohl um solche handeln, die in der Zelle natürlicherweise vorkommen (endogene Transposons), als auch um solche, die natürlicherweise nicht in besagter Zelle vorkommen, sondern mittels gentechnischer Methoden, wie z.B. Transformation der Zelle, in die Zelle eingeführt wurden (heterologe Transposons). Die Veränderung der Expression von Genen mittels Transposons ist dem Fachmann bekannt. Eine Übersicht über die Nutzung von endogenen und heterologen Transposons als Werkzeuge in der Pflanzenbiotechnologie ist in Ramachandran und Sundaresan (2001, Plant Physiology and Biochemistry 39, 234-252) dargestellt.

Die T-DNA Insertionsmutagenese beruht darauf, dass bestimmte Abschnitte (T-DNA) von Ti-Plasmiden aus Agrobacterium in das Genom von pflanzlichen Zellen integrieren können. Der Ort der Integration in das pflanzliche Chromosom ist dabei nicht festgelegt, sondern kann an jeder beliebigen Stelle erfolgen. Integriert die T-DNA in einen Abschnitt oder in die Nähe eines Abschnittes des Chromosoms, der eine Genfunktion darstellt, so kann dieses zur Erhöhung oder Verringerung der Genexpression und damit auch zur Änderung der Aktivität eines durch das betreffende Gen kodierten Proteins führen.

Die in das Genom inserierten Sequenzen (insbesondere Transposons oder T-DNA) zeichnen sich dabei dadurch aus, dass sie Sequenzen enthalten, die zu einer Aktivierung von regulatorischen Sequenzen eines Gens führen, das ein Protein mit der Aktivität einer GWD kodiert ("activation tagging"). Bevorzugt zeichnen sich in das Genom inserierten Sequenzen (insbesondere Transposons oder T-DNA) dadurch aus, dass sie in der Nähe von endogenen Nukleinsäuremolekülen im Genom der Pflanzenzelle oder der Pflanze integriert sind, die ein Protein mit der Aktivität einer GWD kodieren.

Erfindungsgemäße Pflanzen(zellen) können z.B. auch mit Hilfe der Methode des so genannten "activation taggings" (siehe z. B. Walden et al., Plant J. (1991), 281-288; Walden et al., Plant Mol. Biol. 26 (1994), 1521-1528) erzeugt werden. Diese Methode beruht auf der Aktivierung endogener Promotoren durch "enhancer"-Sequenzen, wie z.B. dem Enhancer des 35S RNA-Promoters des Blumenkohlmosaikvirus oder dem Octopinsynthase-Enhancers.

Unter dem Begriff "T-DNA activation tagging" soll im Zusammenhang mit der vorliegenden Erfindung ein T-DNA Fragment verstanden werden, das "enhancer"-Sequenzen enthält und durch Integration in das Genom einer Pflanzenzelle zu der Erhöhung der Expression oder Aktivität eines Proteins mit der Aktivität einer GWD führt.

Unter dem Begriff "Genom" soll im Zusammenhang mit der vorliegenden Erfindung die Gesamtheit des in einer pflanzlichen Zelle vorliegenden Erbmaterials verstanden werden. Dem Fachmann ist bekannt, dass neben dem Zellkern auch andere Kompartimente (z.B. Plastiden, Mitochondrien) Erbmaterial enthalten.

Unter dem Begriff "Transposon activation tagging" soll im Zusammenhang mit der vorliegenden Erfindung ein Transposon verstanden werden, das "enhancer"-Sequenzen enthält und durch Integration in das Genom einer Pflanzenzelle zu der Erhöhung der Expression oder Aktivität eines Proteins mit der Aktivität einer GWD führt.

Dem Fachmann ist bekannt, dass es im Falle von Polyploiden Pflanzen, wie z.B. Weizen, es zur Ausprägung des amylose extender Phänotyps unter Umständen notwendig ist, drei nicht funktionale BEIIa oder BEIIa- und BEIIb-Mutationen (auf dem A,B und D Sub-Genom) homozygot vorliegen zu haben.

Alle diese Methoden sind grundsätzlich zur Erzeugung der "amylose extender-Mutation" und/oder der Erhöhung der Expression oder Aktivität einer GWD und somit zur Herstellung der erfindungsgemäßen Pflanzenzellen bzw. der erfindungsgemäßen Pflanzen geeignet.

Das Auffinden von Mutationen in den entsprechenden Genen, insbesondere in Genen, die BEIIa, BEIIb oder eine GWD kodieren, kann mit Hilfe von dem Fachmann bekannten Methoden geschehen. Insbesondere können hierzu Analysen, basierend auf Hybridisierungen mit Sonden ("Southern Blot"), der Amplifikation mittels Polymerasekettenreaktion (PCR), der Sequenzierung betreffender genomischer Nukleinsäure-Fragmente und die Suche nach einzelnen Nukleotidaustauschen angewandt werden. Eine Methode, um Mutationen anhand von Hybridisierungsmustern zu identifizieren, ist z.B. die Suche nach Restriktionsfragment-Längenunterschieden ("Restriction Fragment Length Polymorphism", RFLP) (Nam et al., 1989, The Plant Cell 1, 699-705; Leister and Dean, 1993, The Plant Journal 4 (4), 745-750). Eine auf PCR basierende Methode ist z.B. die Analyse von amplifizierten Fragment-Längenunterschieden ("Amplified Fragment Length Polymorphism", AFLP) (Castiglioni et al., 1998, Genetics 149, 2039-2056; Meksem et al., 2001, Molecular Genetics and Genomics 265, 207-214; Meyer et al., 1998, Molecular and General Genetics 259, 150-160). Auch die Verwendung von mit Restriktionsendonukleasen geschnittenen amplifizierten Fragmenten ("Cleaved Amplified Polymorphic Sequences", CAPS) kann zur Identifizierung von Mutationen herangezogen werden (Konieczny und Ausubel, 1993, The Plant Journal 4, 403-410; Jarvis et al., 1994, Plant Mol. Biol. 24, 685-687; Bachem et al., 1996, The Plant Journal 9 (5), 745-753). Methoden zur Ermittlung von SNPs sind u.a. von Qi et al. (2001, Nukleic Acids Research 29 (22), e116), Drenkard et al. (2000, Plant Physiology 124, 1483-1492) und Cho et al. (1999, Nature Genetics 23, 203-207) beschrieben worden. Insbesondere sind Methoden, die es erlauben, viele Pflanzen innerhalb kurzer Zeit auf Mutationen in bestimmten Genen hin zu untersuchen, geeignet. Solch eine Methode, das sogenannte TILLING ("Targetting Induced Local Lesions In Genomes") ist von McCallum et al. (2000, Plant Physiology 123, 439-442) beschrieben worden.

Alle diese Methoden sind grundsätzlich zur Identifizierung erfindungsgemäßer Pflanzenzellen bzw. der erfindungsgemäßen Pflanzen geeignet.

In einer weiteren Ausführungsform der vorliegenden Erfindung besteht die genetische Modifikation der erfindungsgemäßen Mais/Weizen-Pflanze(nzelle) in der Einführung mindestens eines fremden Nukleinsäuremoleküls in das Genom der Mais/Weizen-Pflanzenzelle bzw. in das Genom der Mais/Weizen-Pflanze.

In diesem Zusammenhang bedeutet der Begriff "genetische Modifikation" in Bezug auf die GWD das Einführen mindestens eines fremden Nukleinsäuremoleküls in das Genom einer Mais- oder Weizen-Pflanze(nzelle), wobei besagtes Einführen dieses Moleküls zur Erhöhung der Expression oder Aktivität eines Proteins mit der Aktivität einer GWD führt. In Bezug auf die amylose extender-Mutation in Mais kann ein fremdes Nukleinsäuremolekül jedes beliebige Nukleinsäuremolekül sein, das in der Pflanzenzelle oder Pflanze eine Verringerung der BEIIb-Aktivität bewirkt. In Bezug auf die amylose extender-Mutation in Weizen kann ein fremdes Nukleinsäuremolekül jedes beliebige Nukleinsäuremolekül sein, das in der Pflanzenzelle oder Pflanze eine Verringerung der BElla- oder der BElla- und der BEIIb-Aktivität bewirkt.

Durch Einführung eines fremden Nukleinsäuremoleküls sind die erfindungsgemäßen Mais- oder Weizen-Pflanzen(zellen) in ihrer genetischen Information verändert. Das Vorhandensein oder die Expression mindestens eines fremden Nukleinsäuremoleküls führt zu einer phänotypischen Veränderung. "Phänotypische Veränderung" bedeutet dabei vorzugsweise eine messbare Veränderung einer oder mehrerer Funktionen der Zellen. Beispielsweise können die genetisch modifizierten Mais- oder Weizen-Pflanzen(zellen) in Bezug auf die GWD aufgrund des Vorhandenseins oder bei Expression eingeführter fremder Nukleinsäuremoleküle eine Erhöhung der Expression oder Aktivität eines Proteins mit der Aktivität einer GWD zeigen. In Bezug auf das BEIIb-Protein weisen die erfindungsgemäßen Mais-Pflanzen(zellen) aufgrund des Vorhandenseins oder der Expression eingeführter fremder Nukleinsäuremoleküle eine Verringerung der BEllb-Aktivität auf. In Bezug auf das BElla- oder das BElla- und das BEllb-Protein weisen die erfindungsgemäßen Weizen-Pflanzen(zellen) aufgrund des Vorhandenseins oder der Expression eingeführter fremder Nukleinsäuremoleküle eine Verringerung der BElla- bzw. der BElla- und der BEIIb-Aktivität auf.

Unter dem Begriff "fremdes Nukleinsäuremolekül" versteht man im Zusammenhang mit der vorliegenden Erfindung ein solches Molekül, das entweder natürlicherweise in entsprechenden Wildtyp-Pflanzenzellen nicht vorkommt, oder das in der konkreten räumlichen Anordnung nicht natürlicherweise in Wildtyp-Pflanzenzellen vorkommt oder das an einem Ort im Genom der Wildtyp-Pflanzenzelle lokalisiert ist, an dem es natürlicherweise nicht vorkommt. Prinzipiell kann ein fremdes Nukleinsäuremolekül in Bezug auf die GWD jedes beliebige Nukleinsäuremolekül sein, das in der Pflanzenzelle oder Pflanze eine Erhöhung der Expression oder Aktivität eines Proteins mit der Aktivität einer GWD bewirkt. In Bezug auf die amylose extender-Mutation in Mais kann ein fremdes Nukleinsäuremolekül jedes beliebige Nukleinsäuremolekül sein, das in der Pflanzenzelle oder Pflanze eine Verringerung der BEIIb-Aktivität bewirkt. In Bezug auf die amylose extender-Mutation in Weizen kann ein fremdes Nukleinsäuremolekül jedes beliebige Nukleinsäuremolekül bzw. Nukleinsäuremoleküle sein, das/die in der Pflanzenzelle oder Pflanze eine Verringerung der BElla-Aktivität oder BElla- und der BEllb-Aktivität bewirkt/bewirken.

Bevorzugt ist das fremde Nukleinsäuremolekül ein rekombinantes Nukleinsäuremolekül, das aus verschiedenen Elementen besteht, deren Kombination oder spezifische räumliche Anordnung natürlicherweise in pflanzlichen Zellen nicht auftritt.

Unter dem Begriff "rekombinantes Nukleinsäuremolekül" soll im Zusammenhang mit der vorliegenden Erfindung ein Nukleinsäuremolekül verstanden werden, welches unterschiedliche Nukleinsäuremoleküle aufweist, die natürlicherweise nicht in einer Kombination vorliegen, wie sie in einem rekombinanten Nukleinsäuremolekül vorliegen. So weisen die rekombinanten Nukleinsäuremoleküle beispielsweise neben Nukleinsäuremolekülen, die ein Protein mit der Aktivität einer GWD und/oder die ein BEIIb-Protein oder Fragmente davon kodieren (z.B. genomische Nukleinsäuremoleküle oder cDNAs), zusätzliche Nukleinsäuresequenzen auf, welche natürlicherweise nicht in Kombination mit diesen Nukleinsäuremolekülen vorliegen. Das rekombinante Nukleinsäuremolekül weist beispielsweise regulatorische Sequenzen auf (z.B. Promotoren, Terminationssignale, Enhancer), vorzugsweise regulatorische Sequenzen, die heterolog sind in Bezug auf das Nukleinsäuremolekül, das die GWD und/oder das BEIIb-Protein oder Fragmente dieses Proteins kodiert. Heterolog bedeutet in diesem Zusammenhang, dass es sich bei der regulatorischen Sequenz nicht um die eigene endogene regulatorische Sequenz des eingesetzten GWD-Gens und/oder BEIIb-Gens und/oder BEIIa-Gens handelt. Weiterhin bevorzugt sind regulatorische Sequenzen, die in pflanzlichem Gewebe, vorzugsweise im Endosperm aktiv sind.

Methoden zur Erzeugung rekombinanter Nukleinsäuremoleküle sind dem Fachmann bekannt und umfassen gentechnische Methoden, wie z.B. die Verbindung von Nukleinsäuremolekülen durch Ligation, genetische Rekombination oder die Neusynthese von Nukleinsäuremolekülen (siehe z.B. Sambrok et al., Molecular Cloning, A Laboratory Manual, 3rd edition (2001) Cold Spring Harbour Laboratory Press, Cold Spring Harbour, NY. ISBN: 0879695773, Ausubel et al., Short Protocols in Molecular Biology, John Wiley & Sons; 5th edition (2002), ISBN: 0471250929).

Bei dem bzw. den zur genetischen Modifikation verwendeten fremden Nukleinsäuremolekül(en) kann es sich um ein zusammengefügtes oder mehrere getrennte Nukleinsäurekonstrukte handeln, insbesondere so genannte Einfach-, Doppel- oder Dreifachkonstrukte. So kann das fremde Nukleinsäuremolekül beispielsweise ein so genanntes "Doppelkonstrukt" sein, worunter man einen einzigen Vektor zur Pflanzentransformation versteht, der sowohl die genetische Information zur Verringerung/Inhibierung der BEIIb-Aktivität als auch zur Erhöhung der Expression oder Aktivität der GWD enthält.

In einer weiteren Ausführungsform der Erfindung wird in das Genom der Pflanzenzelle nicht ein Doppelkonstrukt, sondern es werden nacheinander mehrere unterschiedliche fremde Nukleinsäuremoleküle/Polynukleotide eingeführt, wobei eines dieser fremden Nukleinsäuremoleküle beispielsweise ein DNA-Molekül ist, das die Erhöhung der Expression oder Aktivität der GWD bewirkt, und ein zweites fremdes Nukleinsäuremolekül beispielsweise eine antisense-RNA kodiert, die eine Verringerung der BEIIb-Aktivität vermittelt. Grundsätzlich sind bei der Konstruktion eines solchen zweiten fremden Nukleinsäuremoleküle aber auch Cosuppressions-, Ribozym- oder doppelsträngige RNA-Konstrukte oder in vivo-Mutagenese geeignet, so lange sie zu einer Verringerung der BEIIb-Aktivität führen.

Die fremden Nukleinsäuremoleküle können hierbei zeitgleich ("Cotransformation") oder auch nacheinander, d.h. zeitlich aufeinanderfolgend ("Supertransformation") in das Genom der Pflanzenzelle eingeführt werden.

Die fremden Nukleinsäuremoleküle/Polynukleotide können auch in verschiedene individuelle Pflanzen einer Spezies eingeführt werden. Es können dabei zunächst Pflanzen erzeugt werden, bei welchen die Expression einer heterologen GWD nachweisbar ist oder bei welchen die BEIIb-Aktivität verringert ist. In einer besonders bevorzugten Ausführungsform der vorliegenden Erfindung werden durch anschließendes Kreuzen Mais-Pflanzen erzeugt, bei welchen die Expression einer heterologen GWD nachweisbar und die BEIIb-Aktivität verringert ist. In einer weiteren besonders bevorzugten Ausführungsform der vorliegenden Erfindung werden durch anschließendes Kreuzen Weizen-Pflanzen erzeugt, bei welchen die Expression einer heterologen GWD nachweisbar und die BEIIa- oder die BEIIa- und die BEIIb-Aktivität verringert ist.

Weiterhin kann zur Einführung eines fremden Nukleinsäuremoleküls/Polynukleotids bzw. zur Erzeugung der erfindungsgemäßen Pflanzenzellen oder der erfindungsgemäßen Pflanzen anstelle einer Wildtyp-Pflanzenzelle bzw. -Pflanze eine Mutante, die sich dadurch auszeichnet, dass sie bereits eine verminderte BEIIb-Aktivität oder eine Expression einer heterologen GWD aufweist, herangezogen werden. Beispielsweise ist die Verwendung von Mais amylose extender Mutanten grundsätzlich vorstellbar, in die man ein fremdes Nukleinsäuremolekül einführt, das zu einer Erhöhung der Expression oder Aktivität der GWD führt im Vergleich zu entsprechenden Wildtyp-Pflanzen(zellen).

In einer weiteren Ausführungsform der vorliegenden Erfindung führt das Vorhandensein und/oder die Expression eines oder mehrerer fremder Nukleinsäuremoleküle/Polynukleotide in Bezug auf die erfindungsgemäßen Mais-Pflanzen(zellen) zur Verringerung der BEIIb-Aktivität.

In einer weiteren Ausführungsform der vorliegenden Erfindung führt das Vorhandensein und/oder die Expression eines oder mehrerer fremder Nukleinsäuremoleküle/Polynukleotide in Bezug auf die erfindungsgemäßen Weizen-Pflanzen(zellen) zur Verringerung der BEIIa- oder der BEIIa- und der BEIIb-Aktivität.

Dies kann durch verschiedene, dem Fachmann bekannte Verfahren erzielt werden. Hierzu zählen beispielsweise die Expression einer entsprechenden Antisense-RNA, oder eines doppelsträngigen RNA-Konstruktes, die Bereitstellung von Molekülen oder Vektoren, die einen Cosuppressions-Effekt vermitteln, die Expression eines entsprechend konstruierten Ribozyms, das spezifisch Transkripte spaltet, die BEIIb oder BEIIa (Weizen) kodieren, oder die so genannte "in vivo-Mutagenese". Ferner kann die Verringerung der BEIIb- und/oder der BEIIa-Aktivität auch durch die simultane Expression von Sense- und Antisense-RNA-Molekülen des BEIIb-(amylose extender)- und/oder des BEIIa-Gens hervorgerufen werden. Diese Methoden sind dem Fachmann geläufig.

Darüber hinaus ist bekannt, dass in planta die Bildung von doppelsträngigen RNA-Molekülen von Promotorsequenzen in trans zu einer Methylierung und einer transkriptionellen Inaktivierung homologer Kopien dieses Promotors führen kann (Mette et al., 2000, EMBO J. 19, 5194-5201).

Zur Inhibierung der Genexpression mittels Antisense- oder Cosuppressions-Technologie kann beispielsweise ein DNA-Molekül verwendet werden, das die gesamte für BEIIa bzw. BEIIb kodierende Sequenz umfasst, als auch DNA-Moleküle, die nur Teile der kodierenden Sequenz umfassen, wobei diese Teile lang genug sein müssen, um in den Zellen einen Antisense-Effekt bzw. Cosuppressions-Effekt zu bewirken. Geeignet sind im Allgemeinen Sequenzen mit einer Mindestlänge von 21-23 bp, bevorzugt mit einer Mindestlänge von 50 bp, besonders bevorzugt mit einer Länge von 100-500 bp.

Für Antisense- oder Cosuppressions-Ansätze geeignet, ist auch die Verwendung von Polynukleotidsequenzen, die einen hohen Grad an Identität zu den endogen in der Pflanzenzelle vorkommenden Sequenzen haben, die für BEIIa bzw BEIIb kodieren. Die minimale Identität sollte größer als ca. 90% sein. Die Verwendung von Sequenzen mit Identitäten von mindestens 95%, insbesondere von mindestens 98% ist zu bevorzugen.

Ferner ist zur Erzielung eines Antisense- oder eines Cosuppressions-Effekts auch die Verwendung von Introns denkbar, d.h. von nicht kodierenden Bereichen von Genen, die für BEIIa bzw BEIIb kodieren.

Die Verwendung von Intron-Sequenzen zur Inhibierung der Expression von Genen, die für Proteine der Stärkebiosynthese kodieren, wurde beschrieben in WO 97/04112, WO 97/04113, WO 98/37213, WO 98/37214.

Dem Fachmann ist bekannt, wie er einen Antisense- und einen Cosuppressions-Effekt erzielen kann. Das Verfahren der Cosuppressions-Inhibierung wurde beispielsweise beschrieben von Jorgensen (1990, Trends Biotechnol. 8, 340-344), Niebel et al. (1995, Top. Microbiol. Immunol. 197, 91-103), Flavell et al. (1995, Curr. Top. Microbiol. Immunol. 197, 43-46), Palauqui und Vaucheret (1995, Plant Mol. Biol. 29, 149-159), Vaucheret et al. (1995, Mol. Gen. Genet. 248, 311-317), de Borne et al. (1994, Mol.. Gen. Genet. 243, 613-621).

Auch die Expression von Ribozymen zur Verringerung der Aktivität von bestimmten Enzymen in Zellen ist dem Fachmann bekannt und ist beispielsweise beschrieben in EP-B1 0321201. Die Expression von Ribozymen in pflanzlichen Zellen wurde z.B. beschrieben von Feyter et al. (1996, Mol. Gen. Genet. 250, 329-338).

Ferner kann die Verringerung der BEIIa- bzw. BEIIb-Aktivität in den Pflanzenzellen auch durch die so genannte "in vivo-Mutagenese" erreicht werden, bei der durch Transformation von Zellen ein hybrides RNA-DNA-Oligonukleotid ("Chimeroplast") in Zellen eingeführt wird (Kipp et al., Poster Session beim 5th International Congress of Plant Molecular Biology, 21.-27. September 1997, Singapur; R. A. Dixon und C. J. Arntzen, Meeting report zu Metabolic Engineering in Transgenic Plants, Keystone Symposia, Copper Mountain, CO, USA, 1997, TIBTECH 15, 441-447; WO 95/15972; Kren et al., 1997, Hepatology 25, 1462-1468; Cole-Strauss et al., 1996, Science 273, 1386-1389; Beetham et al., 1999, PNAS 96, 8774-8778).

Ein Teil der DNA-Komponente des RNA-DNA-Oligonukleotids ist homolog zu einer Polynukleotidsequenz eines endogenen BEIIa- bzw. BEIIb-Gens weist jedoch im Vergleich zur Polynukleotidsequenz eines endogenen BEIIa- bzw. BEIIb-Gens eine Mutation auf oder enthält eine heterologe Region, die von den homologen Regionen umschlossen ist. Durch Basenpaarung der homologen Regionen des RNA-DNA-Oligonukleotids und des endogenen Polynukleotids, gefolgt von homologer Rekombination, kann die in der DNA-Komponente des RNA-DNA-Oligonukleotids enthaltene Mutation oder heterologe Region in das Genom einer Pflanzenzelle übertragen werden. Dies führt zu einer Verringerung der Aktivität von BEIIa oder BEIIb.

Ferner kann die Verringerung der BEIIa- bzw. BEIIb-Aktivität in den Pflanzenzellen auch durch die simultane Expression von Sense- und Antisense-RNA-Molekülen des jeweiligen zu reprimierenden Zielgens, vorzugsweise des BEIIa- oder BEIIb-(amylose extender)-Gens hervorgerufen werden.

Dies kann beispielsweise durch die Verwendung von chimären Konstrukten erreicht werden, die "inverted repeats" des jeweiligen Zielgens oder Teilen des Zielgens enthalten. Hierbei kodieren die chimären Konstrukte für Sense- und Antisense-RNA-Moleküle des jeweiligen Zielgens. Sense- und Antisense-RNA werden in planta gleichzeitig als ein RNA-Molekül synthetisiert, wobei Sense- und Antisense-RNA durch einen Spacer voneinander getrennt sein und ein doppelsträngiges RNA-Molekül bilden können (RNAi-Technologie).

Es konnte gezeigt werden, dass die Einführung von inverted-repeat-DNA-Konstrukten in das Genom von Pflanzen eine sehr effiziente Methode ist, um die zu den inverted-repeat-DNA-Konstrukten korrespondierenden Gene zu reprimieren (Waterhouse et al., 1998, Proc. Natl. Acad. Sci. USA 95, 13959-13964; Wang und Waterhouse, 2000, Plant Mol. Biol. 43, 67-82; Singh et al., 2000, Biochemical Society Transactions 28 (6), 925- 927; Liu et al., 2000, Biochemical Society Transactions 28 (6), 927-929; Smith et al., 2000, Nature 407, 319-320; WO 99/53050). Sense- und Antisense-Sequenzen des Zielgens bzw. der Zielgene können auch getrennt voneinander mittels gleicher oder unterschiedlicher Promotoren exprimiert werden (Nap et al, 6th International Congress of Plant Molecular Biology, 18.-24. Juni 2000, Quebec, Poster S7-27, Vortrag Session S7).

Die Verringerung der BEIIa- oder der BEIIb-Aktivität kann somit auch durch die Erzeugung doppelsträngiger RNA-Moleküle des BEIIa- oder BEIIb-Gens erreicht werden. Vorzugsweise werden hierzu "inverted repeats" von DNA-Molekülen, die von BEIIa- oder BEIIb-Genen abgeleitet sind, in das Genom von Pflanzen eingeführt, wobei die zu transkribierenden DNA-Moleküle unter Kontrolle eines Promotors stehen, der die Expression besagter DNA-Moleküle steuert.

Darüber hinaus ist bekannt, dass die Bildung von doppelsträngigen RNA-Molekülen von Promotor-DNA-Molekülen in Pflanzen in trans zu einer Methylierung und einer transkriptionellen Inaktivierung homologer Kopien dieser Promotoren führen kann, die im Folgenden als Zielpromotoren bezeichnet werden sollen (Mette et al., 2000, EMBO J. 19, 5194-5201).

Über die Inaktivierung des Zielpromotors ist es somit möglich, die Genexpression des BEIIa- oder BEIIb-Gens

(Zielgen), das natürlicherweise unter der Kontrolle dieses Zielpromotors steht, zu verringern.

D.h., die DNA-Moleküle, die die Zielpromotoren der zu reprimierenden Gene (Zielgene) umfassen, werden in diesem Fall, im Gegensatz zur ursprünglichen Funktion von Promotoren in Pflanzen, nicht als Steuerelemente zur Expression von Genen oder cDNAs, sondern selbst als transkribierbare DNA-Moleküle verwendet.

Zur Erzeugung der doppelsträngigen Zielpromotor-RNA-Moleküle in planta, die dort als RNA-Haarnadel-Moleküle ("RNA hairpin") vorliegen können, werden vorzugsweise Konstrukte verwendet, die "inverted repeats" der Zielpromotor-DNA-Moleküle enthalten, wobei die Zielpromotor-DNA-Moleküle unter Kontrolle eines Promotors stehen, der die Genexpression besagter Zielpromotor-DNA-Moleküle steuert. Anschließend werden diese Konstrukte in das Genom von Pflanzen eingeführt. Die Expression der "inverted repeats" besagter Zielpromotor-DNA-Moleküle führt in planta zur Bildung doppelsträngiger Zielpromotor-RNA-Moleküle (Mette et al., 2000, EMBO J. 19, 5194-5201). Hierdurch kann der Zielpromotor inaktiviert werden.

Die Verringerung der BElla- oder BEIIb-Aktivität in den Pflanzenzellen kann somit auch durch die Erzeugung doppelsträngiger RNA-Moleküle von Promotorsequenzen des BElla- oder BEllb-Gens erreicht werden. Vorzugsweise werden hierzu "inverted repeats" von Promotor-DNA-Molekülen von BElla- oder BEllb-Promotoren in das Genom von Pflanzen eingeführt, wobei die zu transkribierenden Zielpromotor-DNA-Moleküle (BElla- oder BEIIb-Promotor) unter Kontrolle eines Promotors stehen, der die Expression besagter Zielpromotor-DNA-Moleküle steuert.

Ferner ist dem Fachmann bekannt, dass er eine Verringerung der BElla- oder BEllb-Aktivität durch die Expression von nicht funktionellen Derivaten, insbesondere transdominanten Mutanten, der Enzyme und/oder durch die Expression von Antagonisten/Inhibitoren der Enzyme erreichen kann.

Antagonisten/Inhibitoren der Enzyme können beispielsweise Antikörper, Antikörperfragmente oder Moleküle mit ähnlichen Bindungseigenschaften sein. Beispielsweise wurde ein cytoplasmatischer scFv-Antikörper eingesetzt, um die Aktivität des Phytochrom A-Proteins in gentechnisch veränderten Tabakpflanzen zu modulieren (Owen, 1992, Bio/ Technology 10, 790-794; Review: Franken et al., 1997, Current Opinion in Biotechnology 8, 411-416; Whitelam, 1996, Trends Plant Sci. 1, 268-272).

Geeignete Promotoren im Zusammenhang mit der vorliegenden Erfindung sind konstitutive Promotoren, wie z.B. der Promotor der 35S RNA des Cauliflower Mosaic Virus (Odell et al., 1985, Nature, 313, 810-812), der Ubiquitin-Promotor aus Mais (Christensen et al., Plant Mol. Biol. 18, (1992), 675-689), der Ubiquitin-Promotor aus Reis (Liu et al., Plant Science 165, (2003), der Reis Actin Promoter (Zhang, et al., Plant Cell 3:1150-1160, 1991), der Cassava Vein Mosaic Virus (CVMV) Promotor (Verdaguer et. al., Plant Mol. Biol. 31: 1129-1139), der Mais Histon H3C4 Promotor (US 6,750,378) oder der *Cestrum* YLCV Promotor (Yellow Leaf Curling Virus; WO 01 73087; Stavolone et al., 2003, Plant Mol. Biol. 53, 703-713).

Besonders bevorzugt handelt es sich um gewebespezifische regulatorische Sequenzen, die in Mais- oder Weizengewebe, vorzugsweise im Endosperm von Mais- oder Weizenpflanzen aktiv sind.

Weitere endosperm-spezifische Promotoren in Mais oder Weizen sind der Promotor des 10 kD Zein-Gens aus Mais (Kirihara et al. (1988) Gene 71: 359-370), des 15 kD Zein-Gens aus Mais (Hoffmann et al. (1987) EMBO J. 6: 3213-3221; Schemthaner et al. (1988) EMBO J. 7: 1249-1253; Williamson et al. (1988) Plant Physiol. 88: 1002-1007), des 27 kd Zein-Gens aus Mais (Prat et al. (1987) Gene 52: 51-49; Gallardo et al. (1988) Plant Sci. 54: 211-281), des 19 kD Zein-Gens aus Mais (Marks et al. (1985) J. Biol. Chem. 260: 16451-16459). Die relativen transkriptionalen Aktivitäten dieser Promotoren in Mais sind bei Kodrzyck et al., (1989), Plant Cell 1, 105-114) beschrieben.

Andere, in Verbindung mit der vorliegenden Erfindung denkbare Promotoren sind der Promotor des Sucrose Synthase Gens (Yang, N.-S. and Russel, D. (1990) Proc. Natl. Acad Sci 87: 4144-4148), des waxy-Gens (Unger et al. (1991) Plant Physiol. 96: 124), des sh 2-Gens (Bhave et al. (1990) Plant Cell 2: 581-588, des bt 2-Gens (Bae et al. (1990) Maydica 35: 317-322). Ferner der HMG-Promotor (auch als Weizen Glutenin HMWG-Promotor bezeichnet) aus Weizen (Colot et al., EMBO J. 6, (1987, 3559-3564; Clarke and Appels, Genome 41, (1998), 865-871), der USP-Promotor, der Phaseolinpromotor, Promotoren von Zein-Genen aus Mais (Pedersen et al., Cell 29 (1982), 1015-1026; Quatroccio et al., Plant Mol. Biol. 15 (1990), 81-93), der Glutelin-Promotor (Leisy et al., Plant Mol. Biol. 14 (1990), 41-50; Zheng et al., Plant J. 4 (1993), 357-366; Yoshihara et al., FEBS Lett. 383 (1996), 213-218), der Globulin Promotor (Nakase et al., 1996, Gene 170(2), 223-226) oder der Prolamin Promotor (Qu und Takaiwa, 2004, Plant Biotechnology Journal 2(2), 113-125).

Es können auch Intronsequenzen zwischen dem Promotor und der kodierenden Region vorhanden sein. Solche Intronsequenzen können zur Stabilität der Expression und zu einer erhöhten Expression in Pflanzen führen (Callis et al., 1987, Genes Devel. 1, 1183-1200; Luehrsen, and Walbot, 1991, Mol. Gen. Genet. 225, 81-93; Rethmeier, et al., 1997; Plant Journal. 12(4):895-899; Rose and Beliakoff, 2000, Plant Physiol. 122 (2), 535-542; Vasil et al., 1989, Plant Physiol. 91, 1575-1579; XU et al., 2003, Science in China Series C Vol.46 No.6, 561-569). Geeignete Intronsequenzen sind beispielsweise das erste Intron des sh1-Gens aus Mais (Maas et al. (1991) Plant. Mol. Biol. 16: 199-207, das erste Intron des Poly-Ubiquitin Gens 1 aus Mais, das erste Intron des EPSPS Gens aus Reis oder eines der beiden ersten Introns des PAT1 Gens aus *Arabidopsis,* ferner Introns des Adh-1 oder Bz-1 Gens aus Mais (Callis et al. (1987) Genes Dev. 1: 1183-1200), das Intron 3 des Mais Actin Gens (Luehrsen, K. R. and Walbot, V. (1991) Mol. Gen. Genet. 225: 81-93) oder des Adh1 Introns 6 (Oard et al. (1989) Plant Cell Rep 8: 156-160).

Die vorliegende Erfindung betrifft auch Vermehrungsmaterial erfindungsgemäßer Pflanzen.

Der Begriff "Vermehrungsmaterial" umfasst im Zusammenhang mit der vorliegenden Erfindung bevorzugt endospermhaltige Samen (Körner) der erfindunsgegmäßen Mais- bzw. Weizenpflanzen.

In einer weiteren Ausführungsform betrifft die vorliegende Erfindung ein Verfahren zur Herstellung der erfindungsgemäßen Maisstärke umfassend den Schritt der Extraktion der Stärke aus einer erfindungsgemäßen Mais-Pflanze und/oder aus einer erfindungsgemäßen Mais-Pflanzenzelle.

In einer weiteren Ausführungsform der vorliegenden Erfindung wird die erfindungsgemäße Mais-Stärke aus einer erfindungsgemäßen Mais-Pflanze enthaltend erfindungsgemäße Mais-Pflanzenzellen, aus Vermehrungsmaterial einer erfindungsgemäßen Mais-Pflanze und/oder aus stärkespeichernden Teilen einer erfindungsgemäßen Mais-Pflanze extrahiert.

Unter dem Begriff "stärkespeichernde Teile" sollen im Zusammenhang mit der vorliegenden Erfindung solche Teile einer Pflanze verstanden werden, in welchen Stärke im Gegensatz zu transitorischer Blattstärke zur Überdauerung von längeren Zeiträumen als Depot gespeichert wird. Bevorzugte stärkespeichemde Pflanzenteile sind Mais/Weizen-Körner, besonders bevorzugt sind Mais/Weizen-Körner enthaltend ein Endosperm.

In einer weiteren Ausführungsform betrifft die vorliegende Erfindung ein Verfahren zur Herstellung einer erfindungsgemäßen Weizenstärke umfassend den Schritt der Extraktion der Stärke aus einer erfindungsgemäßen Weizen-Pflanze und/oder aus einer erfindungsgemäßen Weizen-Pflanzenzelle.

In einer weiteren Ausführungsform der vorliegenden Erfindung wird die erfindungsgemäße Weizen-Stärke aus einer erfindungsgemäßen Weizen-Pflanze enthaltend erfindungsgemäße Weizen-Pflanzenzellen, aus Vermehrungsmaterial einer erfindungsgemäßen Weizen-Pflanze und/oder aus stärkespeichernden Teilen einer erfindungsgemäßen Weizen-Pflanze extrahiert.

Vorzugsweise umfasst das erfindungsgemäße Verfahren auch den Schritt des Erntens der kultivierten erfindungsgemäßen Mais- oder Weizen-Pflanzen oder der stärkespeichernden Pflanzenteile und/oder des Vermehrungsmaterials der erfindungsgemäßen Mais- oder Weizen-Pflanzen vor der Extraktion der Stärke. In einer weiteren Ausführungsform umfasst das erfindungsgemäße Verfahren auch den Schritt der Kultivierung der Mais- oder Weizen-Pflanzen vor dem Ernten.

Verfahren zur Extraktion der Stärke aus erfindungsgemäßen Pflanzen oder aus stärkespeichernden Teilen erfindungsgemäßer Pflanzen sind dem Fachmann bekannt. Beispielsweise sind Verfahren zur Extraktion der Stärke aus verschiedenen Stärke speichernden Pflanzen beschrieben, z. B. in Starch: Chemistry and Technology (Hrsg.: Whistler, BeMiller und Paschall (1994), 2. Ausgabe, Academic Press Inc. London Ltd; ISBN 0-12-746270-8; siehe z.B. Kapitel XII, Seite 412-468: Mais und Sorghum Stärken: Herstellung; von Watson; Kapitel XV, Seite 491 bis 506: Weizenstärke: Herstellung, Modifizierung und Verwendungen; von Knight und Oson) oder in Eckhoff et al., Cereal Chem. 73 (1996), 54-57). Die Extraktion von Maisstärke im industriellen Maßstab wird in der Regel durch das so genannte "wet milling" erreicht. Vorrichtungen, die gewöhnlich zur Extraktion von Stärke aus Pflanzenmaterial verwendet werden, sind Separatoren, Dekanter, Hydrocyclone, Sprühtrockner und Wirbelschichttrockner.

In einer weiteren Ausführungsform betrifft die vorliegende Erfindung Maismehl enthaltend die erfindungsgemäße Maisstärke.

In einer weiteren Ausführungsform betrifft die vorliegende Erfindung Weizenmehl enthaltend die erfindungsgemäße Weizenstärke.

Stärkespeichernde Teile von Pflanzen können zu Mehlen verarbeitet werden. Zur Herstellung von Mais- oder Weizenmehlen werden die endospermhaltigen Maiskörner gemahlen und gesiebt. Stärke ist ein Hauptbestandteil des Endosperms. Die erfindungsgemäße Mais/Weizen-Stärke ist neben Proteinen und Lipiden der wesentliche Bestandteil des erfindungsgemäßen Mais/Weizenmehls (ca. 65 bis 75 Gew.-% des Mehl-Trockengewichtes). Die Eigenschaften der erfindungsgemäßen Mais-/Weizen-Mehle werden daher stark durch die in dem Mais- bzw. Weizen-Mehl enthaltene erfindungsgemäße Mais- bzw. Weizenstärke beeinflusst.

Unter dem Begriff "Maismehl" soll im Zusammenhang mit der vorliegenden Erfindung ein durch Mahlen von Maiskörnern erfindungsgemäßer Mais-Pflanzen erhaltenes Pulver verstanden werden, wobei die Maiskörner aus erfindungsgemäßen Mais-Pflanzenzellen bestehen. Gegebenenfalls werden die Maiskörner vor dem Mahlen getrocknet und nach dem Mahlen zerkleinert und/oder gesiebt.

Ein weiterer Gegenstand der vorliegenden Erfindung ist ein Verfahren zur Herstellung des erfindungsgemäßen Mais-Mehls umfassend den Schritt des Mahlens mindestens einer erfindungsgemäßen Mais-Pflanze. In einer weiteren bevorzugten Ausführungsform des erfindungsgemäßen Verfahrens zur Herstellung des erfindungsgemäßen Mais-Mehls werden Mais-Körner gemahlen, die aus erfindungsgemäßen Mais-Pflanzenzellen bestehen.

Unter dem Begriff "Weizenmehl" soll im Zusammenhang mit der vorliegenden Erfindung ein durch Mahlen von Weizenkörnern erhaltenes Pulver verstanden werden, wobei die Weizenkörner erfindungsgemäßen Weizen-Pflanzenzellen enthalten. Gegebenenfalls werden die Weizenkörner vor dem Mahlen getrocknet und nach dem Mahlen zerkleinert und/oder gesiebt.

Ein weiterer Gegenstand der vorliegenden Erfindung ist ein Verfahren zur Herstellung des erfindungsgemäßen Weizen-Mehls umfassend den Schritt des Mahlens mindestens einer erfindungsgemäßen Weizen-Pflanze. In einer weiteren bevorzugten Ausführungsform des erfindungsgemäßen Verfahrens zur Herstellung der erfindungsgemäßen Mehle werden Weizen-Kömer gemahlen, die aus erfindungsgemäßen Weizen-Pflanzenzellen bestehen.

Vorzugsweise umfasst das erfindungsgemäße Verfahren zur Herstellung von erfindungsgemäßen Mais/Weizen-Mehlen auch den Schritt des Erntens der erfindungsgemäßen Mais/Weizen-Pflanzen bzw. der erfindungsgemäßen Mais/Weizen-Körner dieser Mais/Weizen-Pflanzen vor dem Mahlen, vorzugsweise des Waschens der erfindungsgemäßen Mais/Weizen-Pflanzen bzw. der erfindungsgemäßen Mais/Weizen-Körner vor dem Mahlen und ferner den Schritt der Kultivierung der erfindungsgemäßen Mais/Weizen-Pflanzen vor dem Ernten.

In einer weiteren Ausführungsform der vorliegenden Erfindung umfasst das erfindungsgemäße Verfahren zur Herstellung von Mais- oder Weizen-Mehlen eine Prozessierung der erfindungsgemäßen Mais- oder Weizen-Pflanzen vor dem Mahlen.

Die Prozessierung kann dabei z.B. eine Hitzebehandlung und/oder eine Trocknung sein. Die Zerkleinerung der erfindungsgemäßen Mais- oder Weizen-Pflanzen, von stärkespeichernden Teilen oder Körnern solcher Pflanzen vor dem Mahlen kann ebenfalls eine Prozessierung im Sinne der vorliegenden Erfindung darstellen. Das Entfernen von pflanzlichem Gewebe, wie z.B. von Spelzen der Körner, vor dem Mahlen stellt auch eine Prozessierung vor dem Mahlen in Sinne der vorliegenden Erfindung dar.

In einer weiteren Ausführungsform der vorliegenden Erfindung umfasst das erfindungsgemäße Verfahren zur Herstellung von Mais- oder Weizen-Mehlen nach dem Mahlen eine Prozessierung des Mahlgutes. Das Mahlgut kann dabei z.B. nach dem Mahlen gesiebt werden, um z.B. verschiedene Typenmehle herzustellen.

In einer weiteren Ausführungsform betrifft die vorliegende Erfindung die Verwendung von erfindungsgemäßem Maismehl, Mais-Stärke, Weizenmehl oder Weizen-Stärke zur Herstellung eines Lebensmittels.

In einer weiteren Ausführungsform betrifft die vorliegende Erfindung die Verwendung von erfindungsgemäßem Maismehl, Mais-Stärke, Weizenmehl oder Weizen-Stärke als Prebiotikum.

In einer weiteren Ausführungsform betrifft die vorliegende Erfindung eine Zusammensetzung enthaltend die erfindungsgemäße Maisstärke und mindestens einen Nahrungsmittelzusatzstoff.

In einer weiteren Ausführungsform betrifft die vorliegende Erfindung eine Zusammensetzung enthaltend das erfindungsgemäße Maismehl und mindestens einen Nahrungsmittelzusatzstoff.

In einer weiteren Ausführungsform betrifft die vorliegende Erfindung eine Zusammensetzung enthaltend die erfindungsgemäße Weizenstärke und mindestens einen Nahrungsmittelzusatzstoff.

In einer weiteren Ausführungsform betrifft die vorliegende Erfindung eine Zusammensetzung enthaltend das erfindungsgemäße Weizenmehl und mindestens einen Nahrungsmittelzusatzstoff.

Als Nahrungsmittelzusatzstoff sollen im Zusammenhang mit der vorliegenden Erfindung beispielsweise Vitamine (z.B. Vitamin A, B1, B2, B3, B5, B6, B9, B12, C, D, E, F, K), Provitamine, Antioxidantien, Spurenelemente (z. B. Chrom, Eisen, Fluor, Iod, Kobalt, Kupfer, Mangan, Molybdän, Selen, Vanadium, Zink), Mengenelemente (z.B. Calcium, Chlor, Kalium, Magnesium, Phosphor, Schwefel, Natrium), Aromen, Farbstoffe, Öle, Fette, Fettsäuren, insbesondere (mehrfach) ungesättigte Fettsäuren, essentielle Fettsäuren, Kohlenhydrate (z.B. Stärken, Galaktooligosaccharide, Gentiobiose, Tagatose), Ballaststoffe (z.B. Zellulose, Hemizellulose, Pektin, Ligin), Prebiotika (z.B. Oligofruktose, Oligosaccharide, Chitosan, beta Glukane, Arabinogalactan), Probiotika (z.B. Bifidobakterien, Milchsäurebakterien wie z.B. der Gattung Lactobacillus), d.h. nicht-pathogene Mikroorganismen, die dem Lebensmittel lebend oder in Sporenform zugesetzt werden und die Darmflora positiv beeinflussen können.

Die Herstellung der erfindungsgemäßen Zusammensetzungen kann beispielsweise durch einfaches Mischen erfolgen.

In einer weiteren Ausführungsform betrifft die vorliegende Erfindung ein Lebensmittel enthaltend die erfindungsgemäße Maisstärke oder die erfindungsgemäße Weizenstärke.

In einer weiteren Ausführungsform betrifft die vorliegende Erfindung ein Lebensmittel enthaltend das erfindungsgemäße Maismehl oder das erfindungsgemäße Weizenmehl.

In einer weiteren Ausführungsform betrifft die vorliegende Erfindung ein Lebensmittel enthaltend die erfindungsgemäße Zusammensetzung.

Typische Lebensmittel, die unter Verwendung der erfindungsgemäßen Maisstärke, des erfindungsgemäßen Maismehls oder der erfindungsgemäßen Zusammensetzung hergestellt werden können, sind beispielsweise Tortillas, Tortillachips, Backwaren (z.B. Brot, Maisbrot, Brötchen, Kekse, Kuchen, Waffeln, Muffins, Maisfladen), Pfannkuchen, Pizza, Polenta, Teigwaren (z.B. Nudeln), "cornmeal mush" (USA), "porridge" (GB), Eintopfgerichte, Saucen, Maismehlpudding, Milchprodukte (z.B. Joghurt, Quark, Eis), Puddings, Aufstriche (z.B. Butter, Margarine), Getränke, Getränkepulver, Fertiggerichte, (Frühstücks)-Cerealien, Enchelada, Wurstwaren, Fleischwaren, Kindernahrung, Ketchup, Mayonnaise, Barbecue-Saucen und andere.

**Allgemeine Methoden**

Im Folgenden werden Methoden beschrieben, welche zur Durchführung der vorstehend beschriebenen Erfindung verwendet werden können. Diese Methoden stellen konkrete Ausführungsformen der vorliegenden Erfindung dar, beschränken die vorliegende Erfindung jedoch nicht auf diese Methoden.

**1) Pflanzenmaterial und Kultivierung**

| | |
|---|---|
| Maispflanzen: | Zea mays, Varietät A188 |
| | Zea mays, Mutante ae1 (amylose extender) Varietät W64A |
| Weizenpflanzen: | Triticum aestivum, Varietät Fielder |

Die Kulturführung der Maispflanzen im Gewächshaus erfolgte unter nachfolgenden Bedingungen:

| | |
|---|---|
| Substrat: | Sondermischung für Aussaat<br>80% Weißtorf<br>20% Schwarztorf<br>100 kg/$m^3$ Sand<br>40 kg/$m^3$ Feuchtton<br>Struktur: fein<br>pH-Wert: 5,3-6,1<br>Grunddüngung: 2 kg/$m^3$ und 100g/$m^3$ Radigen (Theraflor GmbH; Iserlohn; Germany) (Zusammensetzung: 5% MgO; 2% Fe; 1,5% Cu; 1% Mn; 0,8% Mo; 0,6% B; 0,5% Zn) |
| Töpfe: | 10L Container |
| Standweite: | Max. 6 Pflanzen/$m^2$ |
| Bewässerung: | Pflanzen gleichmäßig feucht halten, aber unbedingt Staunässe sowie Austrocknen des Substrates verhindern |
| Düngung: | 1 TAB Plantosan 4g (Zusammensetzung 15% N; 8% $P_2O_5$; 15% $K_2O$; 2% MgO + Spurenelemente) im 4-Blattstadium<br>1 TAB Plantosan nach weiteren 3 Wochen |
| Temperatur: | Tag 22-25°C/Nacht 16°C |
| Licht: | 18 Stunden, 350-400μEinstein/s/m |
| Luftfeuchte: | 50% rel. |

Die Kulturführung der Weizenpflanzen im Gewächshaus erfolgt unter nachfolgenden Bedingungen:

| | |
|---|---|
| Substrat: | Sondermischung<br>100% Weißtorf<br>200 L/$m^3$ Sand<br>180 kg/$m^3$ Feuchtton<br>20% Perlite<br>Struktur: mittelgrob<br>pH-Wert 5,5 - 6,0<br>Grunddüngung: 2 kg/ $m^3$ 12-14-24 (+3) und 100mg/$m^3$ Radigen (Theraflor GmbH; Iserlohn; Germany) (Zusammensetzung: 5% MgO; 2% Fe; 1,5% Cu; 1% Mn; 0,8% Mo; 0,6% B; 0,5% Zn) |
| Töpfe: | viereckig, 1,6 l Volumen |
| Standweite: | 42 Töpfe/$m^2$ |
| Bewässerung: | Pflanzen gleichmäßig feucht halten, aber unbedingt Staunässe sowie Austrocknen des Substrates verhindern |
| Düngung: | 1g/Pflanze in drei Gaben, Hakaphos (Zusammensetzung 15% N; 10% $P_2O_5$; 15% $K_2O$; 2% MgO) Fa.Compo |
| Temperatur: | Tag 20-25°C / Nacht 16°C |
| Licht: | 18 Stunden, 350-400μEinstein/s/m |
| Luftfeuchte: | rel. 50% |

Pflanzenschutz nach Indikation:

Pirimicarb® (Zeneca), Confidor® (Bayer), Neem Azal® (Trifolio-M GmbH), Vertimec® (Syngenta)

**2) Transformation und Regeneration von Mais- und Weizenpflanzen**

Maispflanzen wurden nach der von Ishida et al. (1996 Nature Biotechnology Vol 14: 745-750) beschriebenen Methode transformiert und regeneriert.
Die Transformation und Regeneration der Weizenpflanzen erfolgte nach der Methode von Jones et al., (Jones H.D., Doherty A., Wu H., 2005. Review of methodologies and a protocol for the Agrobacterium-mediated transformation of wheat. Plant Methods 2005, 1:5 doi:10.1186/1746-4811-1-5).

**3) Herstellung und Identifizierung genetisch modifizierter Maispflanzen, die eine amylose extender Mutation und eine nachweisbare GWD-Expression aufweisen**

Für die Kreuzung der transgenen Maispflanzen mit nachweisbarer GWD-Expression mit der ae1-Mutante der Varietät W64A wurde die ae1-Mutante 7-10 Tage im voraus ausgelegt und die transgenen Linien dann entsprechend nach Erreichen des 2 Blatt Stadiums der Mutante. Dies ist erforderlich, um eine synchrone Entwicklung der Blüten zu bekommen. Die ae1-Mutante wurde als Pollenspender (männlicher Kreuzungspartner) eingesetzt und die Träger mit nachweisbarer GWD-Expression als weiblicher Kreuzungspartner.
Die daraus entstandene F1 Generation wurde anschließend geselbstet und Saatgut selektiert, welches sowohl für die ae1-Mutation als auch für das GWD-Gen homozygot ist.

**4) Erzeugung, Ernte und Prozessierung von Maiskörnern**

Zur Erzeugung von ausreichenden Mengen an Untersuchungsmaterial wurden Maispflanzen unter Gewächshausbedingungen angezogen und nach Erreichen vollständiger Reife *(ca. 40 Tage nach Bestäubung)* die Kolben geerntet. Zum weiteren Trocknen wurden die reifen (d.h. voll entwickelten) Maiskolben für 3-7 Tage bei 37°C gelagert.
Nach der Trocknung (unter 13% Feuchtigkeit, gemessen mit dem Gerät "Grain Moisture Tester Riceter J301" von Fa Kett Electric Laboratory, Tokyo, Japan) wurden die Körner vom Kolben abgenommen und als Ausgangsmaterial für Analysen des ganzen Kornes, wie z.B. Korngewicht, verwendet.

**5) Bestimmung des Phosphatgehaltes der Stärke in C6-Position (C6-P-Gehalt)**

In der Stärke können die Positionen C3 und C6 der Glucoseeinheiten phosphoryliert sein. Zur Bestimmung des C6-P-Gehaltes der Stärke (modifiziert nach Nielsen et al., 1994, Plant Physiol. 105: 111-117) wurden 50 mg Maismehl/Stärke in 500 µl 0,7 M HCl 4 h bei 95°C unter ständigem Schütteln hydrolysiert. Anschließend wurden die Ansätze für 10 min bei 15.500 g zentrifugiert und die Überstände mittels einer Filtermembran (0,45 µM) von Schwebstoffen und Trübungen gereinigt. Von dem klaren Hydrolysat wurden 20 µl mit 180 µl Imidazol-Puffer (300 mM Imidazol, pH 7,4; 7,5 mM $MgCl_2$, 1 mM EDTA und 0,4 mM NADP) gemischt. Die Messung wurde im Photometer bei 340 nm durchgeführt.
Nach Erfassung der Basisabsorption wurde die Enzymreaktion durch die Zugabe von 2 Einheiten (units) Glucose-6-Phosphat Dehydrogenase (von *Leuconostoc mesenteroides,* Boehringer Mannheim) gestartet. Die Absorptionsänderung beruht auf einer äquimolaren Umsetzung von Glucose-6-Phosphat und NADP zu 6-Phosphorglukonat und NADPH, wobei die Bildung des NADPH bei der o. g. Wellenlänge erfasst wird. Die Reaktion wurde bis zum Erreichen eines Plateaus verfolgt. Das Ergebnis dieser Messung liefert den Gehalt an Glucose-6-Phosphat im Hydrolysat. Aus dem identischen Hydrolysat wurde anhand des Gehaltes an freigesetzter Glucose der Grad der Hydrolyse bestimmt. Dieser wird verwendet, um den Gehalt an Glucose-6-Phosphat auf den Anteil hydrolysierter Stärke aus der Menge Frischgewicht zu beziehen. Hierzu wurden 10 µl Hydrolysat mit 10 µl 0,7 M NaOH neutralisiert und nachfolgend 1:100 mit Wasser verdünnt. 4 µl dieser Verdünnung wurden mit 196 µl Messpuffer (100 mM Imidazol pH 6,9; 5 mM $MgCl_2$, 1 mM ATP, 0,4 mM NADP) versetzt und zur Bestimmung der Basisabsorption verwendet. Die Reaktion wurde durch Zugabe von 2 µl Enzym-Mix (Hexokinase 1:10; Glucose-6-Phosphat Dehydrogenase aus Hefe 1:10 in Messpuffer) und bei 340 nm bis zum Plateau verfolgt. Das Messprinzip entspricht dem der ersten Reaktion.
Das Ergebnis dieser Messung liefert die Menge an Glucose (in mg), die im Verlauf der Hydrolyse aus der im Ausgangsmaterial vorhandenen Stärke freigesetzt wurde.
Nachfolgend wird das Ergebnis beider Messungen in Relation gesetzt, um so den Gehalt an Glucose-6-Phosphat pro mg hydrolysierter Stärke auszudrücken. Anders als bei einem Bezug der Menge an Glucose-6-Phosphat auf das Frischgewicht der Probe wird durch diese Berechnung die Menge an Glucose-6-Phosphat lediglich auf den Teil der Stärke bezogen, welcher vollständig zu Glucose hydrolysiert wurde und somit auch als Quelle für das Glucose-6-Phosphat anzusehen ist.

### 6) Bestimmung des Quellvermögens (SP, Swelling Power) und der Heißwasser-Löslichkeit

Das Quellvermögen und die Heißwasser-Löslichkeit einer 3%-(w/v)-igen Stärkesuspension bei 90°C wurden nach der Methode von Leach et al. (Cereal Chemistry 36, (1959), 534-544) ermittelt.
Hierzu wurde die Stärkesuspension für 30 Minuten unter ständigem Schütteln (200rpm) bei 90°C inkubiert und nach Abkühlen auf Raumtemperatur für 15 Minuten bei 700 x g zentrifugiert. Der resultierende Überstand wurde vollständig entfernt und zur Ermittlung der Menge an löslichen Bestandteilen für 48 Stunden bei 37°C vollständig getrocknet und nachfolgend gewogen. Der Rückstand der Zentrifugation repräsentiert das gequollene Stärkegel, dessen Gewicht gravimetrisch bestimmt wird. Es ergeben sich gemäß folgende Formeln:

Quellvermögen (g/g) = Gewicht des Gels / (Gewicht der eingewogene Stärke – lösliche Bestandteile)

Heißwasser-Löslichkeit bei 90°C in % = Gewicht getrockneter Überstand / Gewicht der eingewogenen Stärke x 100

### 7) Bestimmung des Gehaltes an apparenter Amlyose

Die Bestimmung des Gehaltes an apparenter Amylose erfolgte in Anlehnung an die Methode von Juliano (1971, Cereal Science Today 16 (10): 334-340).

Für jede Probe wurden zweimal 50 mg Mais- bzw. Weizenmehl oder Mais- bzw. Weizen-Stärke in 100 ml Erlenmeyerkolben eingewogen und aufeinander folgend mit 1 ml 95% Ethanol und 9 ml 1M NaOH befeuchtet.

Parallel werden zur Anfertigung einer Standardkurve Kolben mit definierten Mengen reiner Amylose in der gleichen Weise behandelt wie die Mehl/Stärkeproben. Die Kolben wurden zur Durchmischung kurz geschwenkt und anschließend für 20 Minuten im kochenden Wasserbad unter leichtem Schütteln inkubiert. Nach 5-10 Minuten Abkühlen bei Raumtemperatur (RT) wurde das Volumen mit Wasser auf 100 ml aufgefüllt.

Ein Aliquot von 100 µl wurde mit 1 ml Messlösung (10 mM Essigsäure, 0,004% (w/v) $I_2$; 0,04% (w/v) KI) versetzt, gut durchmischt und die Absorption bei 620nm gegen einen entsprechenden Blindwert bestimmt. Die Berechnung des Amylose-Gehaltes erfolgte mit Hilfe der Amylose-Standards, die zur Erstellung einer Eichkurve verwendet werden.

### 8) Analyse von Mais- oder Weizenstärke mittels Rapid Visco Analyser (RVA)

Das Prinzip dieser Analyse beruht darauf, dass eine Suspension aus Wasser und Maisstärke einem definierten Temperatur- und Scher-Protokol unterzogen und dabei kontinuierlich die Viskosität der Suspension aufgezeichnet wird. Als Messgerät dient ein RVA Super3 der Firma Newport Scientific (Macclesfield, UK) mit der entsprechenden Software "Thermocline for Windows", Version 2.3.

Für die Analyse wurden 2,5 g Mais/Weizen-Stärke (Einwaage als reines Trockengewicht des Probenmaterials, korrigiert auf 0% Feuchte) in ein Messgefäß eingewogen, mit 25 ml Wasser versetzt und das Messgefäß nach Einsetzen eines Rührers in das Gerät eingespannt.

Das folgende Temperatur- und Scher-Profil wurde appliziert:

| Zeit | Art | Wert |
|---|---|---|
| 00:00:00 | Temp | 50°C |
| 00:00:00 | Speed | 960 rpm |
| 00:00:10 | Speed | 160 rpm |
| 00:01:00 | Temp | 50°C |
| 00:04:45 | Temp | 95°C |
| 00:07:15 | Temp | 95°C |

(fortgesetzt)

| Zeit | Art | Wert |
|---|---|---|
| 00:11:00 | Temp | 50°C |
| 00:12:30 | End of test | |

Nach Beendigung der Messung wurden die folgenden Parameter ermittelt:

| | |
|---|---|
| Peak Viskosität | (höchste Viskosität zwischen 2 und 8 Minuten Messzeit) |
| Trough Viskosität | (geringste Viskosität zwischen 6 und 12 Minuten Messzeit) |
| Final Viskosität | (Viskosität am Ende der Messung nach 12,5 Minuten) |

Breakdown = Peak - Trough
Setback = Final - Trough

| | |
|---|---|
| Pasting Temperatur | (Temperatur, bei der sich in einem Zeitintervall von 0,5 Minuten die Viskosität um mehr als 50cP ändert) |
| Peak time | (Zeit, bei der die Peak Viskosität erreicht ist) |

**9) Extraktion von Maisstärke**

Die Extraktion von Maisstärke erfolgte in Anlehnung an die bei "corn refiners association" (http://www.corn.org/) beschriebene Methode der Nassstärkeextraktion. 10-50g Maiskörner wurden eingewogen und zum Aufbrechen der Proteinmatrix im Überschuss mit 0,2%iger schwefliger Säure drei Tage bei 50 °C inkubiert. Die Körner wurden anschließend mit Wasser gewaschen und kurz abgetrocknet. Die Zerkleinerung erfolgte in einer Retsch Ultrazentrifugalmühle ZM100 mit 2 mm Sieb (Retsch, Haan, Deutschland). Das zerkleinerte Material wurde in ein Becherglas überführt, mit 20 %iger NaCl-Lösung versetzt und mindestens für 30 min stehen gelassen. Hierbei sedimentiert die Stärke und die Lipid-Körper schwimmen auf. Die obere Schicht (Keime) wurde abgegossen und das Sediment im restlichen Überstand erneut suspendiert. Anschließend wurde in mehreren Siebschritten die Stärke weiter aufgereinigt. Zuerst wurde die Probe durch ein 500µm Prüfsieb (DIN 4188), anschließend durch ein 200µm Retsch Analysensieb (DIN 4188) und zuletzt durch ein 125µm Sieb (Iso 3310-1) gegeben und mit NaCl (2-3 l) mit Hilfe eines Drucksprühgerätes nachgespült, bis die Tropfen unter dem Sieb keine Stärke mehr enthielten. Diese vorgereinigte Stärke wurde über Nacht bei Raumtemperatur sedimentiert und im Anschluss der Überstand bis auf ca. 5mm über dem Sediment abgegossen. Die Stärke wurde in Zentrifugenbecher überführt und in einer Heraeus Varifuge (Heraeus, Hanau, Deutschland) bei Raumtemperatur mit 3500 rpm für 10 min zentrifugiert. Anschließend wurde die oben liegende Stärke-Protein-Schicht (meist farblich anders) abgekratzt und verworfen.

Im weiterem folgten mehrere Waschschritte, zunächst mit 0,2M Natrium-Acetat pH 4,6 (Zentrifugation s.o. 5 min), wobei nach jedem Waschschritt die Stärke-Protein-Schicht erneut abgekratzt wurde. Im Anschluss hieran wurde ein Verdau in 0,2M Natrium-Acetat pH 4,6 mit je 1% Bromelain (Fa. AppliChem, Darmstadt, Deutschland) und 1% Pepsin (Fa. AppliChem, Darmstadt, Deutschland) angesetzt und bei 37°C für eine Stunde auf dem Rotator (Vertikal Schüttler; Fröbel, Lindau, Deutschland) inkubiert. Anschließend wurde zentrifugiert (s.o.) und der Überstand verworfen. Die Stärke-Protein-Schicht wurde erneut verworfen und das Pellet in Leitungswasser resuspendiert und zentrifugiert (s.o. 3000 rpm). Hierauf folgte eine erneute mechanische Trennung der auf dem Pellet befindlichen, meist klar abgegrenzten Proteinschicht. Es folgten weitere vier Waschschritte mit Wasser wie oben beschrieben. Anschließend wurde das Pellet viermal mit 80 % technischem Ethanol gewaschen und zentrifugiert (s.o. 3000 rpm). Schließlich wurde einmal mit Aceton gewaschen, um die Stärke zu entfetten, und zwei Tage unter dem Abzug bei Raumtemperatur getrocknet.

**10) Nachweis der Verzweigungsenzym-Aktivität mittels Aktivitätsgel**

Der Nachweis der verschiedenen Verzweigungs-Enzym-Aktivitäten in unreifen Maiskörnern erfolgte mittels Aktivitätsgelen (Zymogrammen, siehe auch Figur 2), bei denen Proteinextrakte unter nativen Bedingungen in einem Polyacrylamidgel aufgetrennt und nachfolgend mit entsprechenden Substraten inkubiert werden. Das entstandene Reaktionsprodukt (Stärke) wurde mittels Lugol'scher Lösung (2% (w/v) KI; 0,2% (w/v) $I_2$) im Gel angefärbt.

Einzelne unreife Maiskörner (ca. 15 Tage nach der Blüte - gemessen ab dem Tag des Blühbeginns) wurden in flüssigem Stickstoff schock gefroren und in 150-200 µl kaltem Extraktionspuffer (50 mM Tris/HCl pH 7,6, 2,5 mM EDTA, 2 mM DTT, 4 mM PMSF, 0,1% (w/v) Glykogen, 10% (v/v) Glyzerin) homogenisiert. Nach Zentrifugation (15 min, 13000 g, 4°C) wurde der klare Überstand in ein frisches Reaktionsgefäß überführt und ein Aliquot des Extraktes zur Bestimmung des Proteingehaltes nach Bradford (1976, Anal Biochem 72: 248-254) verwendet.

Die Auftrennung der Proteinextrakte erfolgte mittels eines kontinuierlichen 7,5% Polyacrylamid-Geles (7,5% AA/BAA 37,5:1; 25 mM Tris/HCl pH 7,6, 192 mM Glycin, 0,1 % (w/v) APS, 0,05% (v/v) TEMED) unter Verwendung von einfach konzentriertem Laufpuffer (25 mM Tris/HCl, 192 mM Glycin). Vor Beladung der Gele erfolgt ein Vorlauf zur Entfernung von Radikalen für 30 Minuten bei 8mA und 4°C. Für jede Probe wurden 2 µg Protein aufgetragen und die Elektrophorese für 2 - 2,5 Stunden bei 8mA und 4°C durchgeführt. Anschließend wurden die Gele in Waschpuffer (0.1M Natriumcitrat pH 7,0) fünfmal für 5 Minuten auf Eis gewaschen. Nachfolgend wurden die Gele in 15 ml Inkubationspuffer (0.1M Natriumcitrat pH 7,0, 50mM Glucose-1-Phosphat, 1mM AMP, 0,95Units/ml Phosphorylase A aus Kaninchen) über Nacht bei Raumtemperatur unter ständigem Schütteln inkubiert. Die Anfärbung der gebildeten Stärke erfolgte mittels Lugol'scher Lösung.

**11) Nachweis der Expression der GWD aus *Curcuma longa* mittels quantitativer PCR**

RNA wurde aus Blattproben präpariert, die direkt nach der Probennahme in flüssigem Stickstoff eingefroren wurden. Nach Homogenisierung mit einer 4 mm Stahlkugel (Retsch-Mühle, 30 Hz, 45 sec) wurde mit dem "SV 96 Total RNA Isolation System" von Promega die RNA nach Protokoll No. 294 (Promega) präpariert. Die RNA wurde mit je 10 µl "RQ1 RNase-Free DNase" (Promega) nach Herstellervorschrift behandelt.

Die quantitative RT-PCR wurde mit den Reagenzien des "Access RT-PCR System" von Promega durchgeführt. Das Amplikon für R1 aus *Curcuma longa* hatte eine Länge von 105 bp Basenpaaren. Als Amplifikationsprimer wurden Cl_R1-F1 (TggATAAATACAAAgAgTgAAgCAg = SEQ ID No. 11) und Cl_R1-R1 (ggACATTgAAggTgTTgTAAAgg = SEQ ID No. 12) verwendet. Als Taqman-Sonde wurde das zweifach fluoreszenzmarkierte Oligonukleotid Cl_R1-FAM (FAMcttcgtcgtccaaacaagaccacag -TAMRA = SEQ ID NO. 13) verwendet.

Das Amplikon für Catalase aus Mais hatte eine Länge von 73 Basenpaaren. Für die Amplifikation wurden die Primer Zm_Cat-F1 (GTGGGAGCAACTCCAGCTT = SEQ ID No. 14), Zm_Cat-R1 (CGGTGAGGGCAGAGTTGTT= SEQ Id No. 15) und die zweifach fluoreszenzmarkierte TaqMan-Sonde Cat-VIC (VIC-ACTCCGGCGCCCCCGT -TAMRA 0 SEQ ID No. 16) eingesetzt.

Im 30 µl-Reaktionsansatz befanden sich Puffer (1-fach), 3 mM $MgSO_4$, je 500 nM Amplifikationsprimer, 100 nM Sonde Cl_R1-FAM bzw. Cat-VIC, je 0,2 mM desoxy-Ribonukleotide, je 0,6 µl reverse Transkriptase und Tfl-Polymerase (entsprechend Protokoll 294). Jeder Ansatz enthielt 350 ng Gesamt-RNA.

Die Reaktionsbedingungen für die RT-PCR waren: 30 min 55 °C, 2 min 94 °C, 40 x (15 sec 94 °C, 1 min 60 °C). Das Fluoreszenzsignal wurde vom Gerät ABI Prism 7700 (Applied Biosystems) jeweils während der kombinierten Annealing/Extensionphase aufgenommen.

Als Kontrollen wurden jeweils Ansätze ohne reverse Trankriptase im Ansatz mitgeführt.

Die Berechnung der relativen Expression wurde nach M. W. Pfaffl, A new mathematical model for relative quantification in real-time RT-PCR, Nukleic Acids Research 2001, Vol. 29, No. 9 00 durchgeführt. Die Expression von R1 aus *Curcuma longa* wurde auf die Expression von Catalase normiert.

**12) Bestimmung der Kopienzahl für GWD aus Curcuma mittels PCR**

DNA wurde aus Blattproben präpariert, die direkt nach der Probennahme in flüssigem Stickstoff eingefroren wurden. Nach Homogenisierung mit einer 4 mm Stahlkugel (Retsch-Mühle, 30 Hz, 45 sec) wurde mit dem Qiagen Kit "DNeasy plant Mini kit" präpariert. Die quantitative PCR wurde mit folgenden Amplicons durchgeführt:

Das Amplikon für R1 aus *Curcuma longa* hatte eine Länge von 105 bp Basenpaaren. Als Amplifikationsprimer wurden Cl_R1-F1 (TggATAAATACAAAgAgTgAAgCAg = SEQ ID No. 11) und Cl_R1-R1 (ggACATTgAAggTgTTg-TAAAgg = SEQ ID No. 12) verwendet. Als Taqman-Sonde wurde das zweifach fluoreszenzmarkierte Oligonukleotid Cl_R1-FAM (FAM- cttcgtcgtccaaacaagaccacag -TAMRA = SEQ ID No. 13) verwendet.

Das Amplikon für Catalase aus Mais hatte eine Länge von 73 Basenpaaren. Für die Amplifikation wurden die Primer Zm_Cat-F1 (GTGGGAGCAACTCCAGCTT=SEQ ID No. 14), Zm_Cat-R1 (CGGTGAGGGCAGAGTTGTT= SEQ ID No. 15) und die zweifach fluoreszenzmarkierte TaqMan-Sonde Cat-VIC (VIC-ACTCCGGCGCCCCCGT -TAMRA = SEQ ID No. 16) eingesetzt.

Für die Reaktionen (30 µl) wurde der HotStar Mastermix von Qiagen verwendet. Im Reaktionsansatz befanden sich Mastermix (1-fach) bei dem die Endkonzentration von $MgCl^{2+}$, auf 3 mM eingestellt wurde. Die Endkonzentrationen der Amplifikationsprimer betrugen je 300 nM und die der Sonde Cl_R1-FAM bzw. Cat-VIC 100 nM. Nach Herstellerangaben betrug die Endkonzentration der desoxy-Nukleotide 200 µM und die Enzymkonzentration 1,5 Units Taq-Polymerase/pro 30 µl-Ansatz. Es wurden jeweils zwei Ansätze mit 50 ng bzw. 5 ng DNA als Template pro Ansatz analysiert.

Die Reaktionsbedingungen für die PCR waren: 2 min 94 °C, 40 x (15 sec 94 °C, 1 min 60 °C). Das Fluoreszenzsignal wurde vom Gerät ABI Prism 7700 (Applied Biosystems) jeweils während der kombinierten Annealing/Extensionphase aufgenommen.

Die Berechnung der relativen Kopienzahlen wurde nach M. W. Pfaffl, A new mathematical model for relative quantification in real-time RT-PCR, Nukleic Acids Research 2001, Vol. 29, No. 9 00 durchgeführt. Die DNA-Menge wurde über das Amplifikationssignal für Catalase normiert.

**Beispiel 1**

**Herstellung eines Konstruktes zur Transformation von Maispflanzen, die eine nachweisbare GWD-Genexpression zeigen**

Als Ausgangsplasmid zur Herstellung des Plasmides pSC16, welches zur Transformation von Maispflanzen verwendet wurde, diente das Plasmid pMZ12. Dieses Plasmid enthält den *ColE1* Origon des Plasmides pBR322 (Bolivar et al, 1977, Gene 2, 95-113) und einen bakteriellen Selektionsmarker, der eine Resistenz gegenüber dem Antibiotikum Gentamycin vermittelt (Wohlleben et al., 1989, MGG 217, 202-208). Weiterhin enthält dieses Plasmid eine rechte und eine linke T-DNA Border Sequenz. Zwischen diesen T-DNA Border Sequenzen enthält das Plasmid ein *bar*-Gen aus *Streptomyces hygroscopicus* (White et al., 1990, NAR 18, 1062; EMBL Acc.: X17220), welches Resistenz gegenüber dem Herbizid Glufosinat vermittelt. Die Expression des *bar*-Gens wird durch den Promotor des actin-Gens aus Reis (McElroy et al., 1990, Plant Cell 2, 163.171) initiiert. Zur Stabilisierung der Expression des *bar*-Gens ist zwischen dem *actin* Promotor und der das *bar*-Protein codierenden Sequenz das 1. Intron des actin-Gens aus Reis (McElroy et al., 1990, Plant Cell 2, 163.171) eingefügt. Nach der das *bar*-Protein codierenden Sequenz folgt das Polyadenylierungssignal des Nopalinsynthase Gens aus *Agrobacterium tumefaciens* (Depicker et al., 1982, J Mol. Appl. Gent. 1, 561-573).

In das Plasmid pMZ12 wurde der Ubiquitinpromotor aus *Zea mays* (Christensen et al. 1992, Plant Mol. Bio 18, 675-689), gefolgt vom 1. Intron des Ubiquitin Gens aus Zea *mays* (Christensen et al. 1992, Plant Mol. Bio 18, 675-689), gefolgt von der codierenden Sequenz des GWD-(R1)-Gens aus *Curcuma longa* (siehe SEQ ID NO 3) unter Zuhilfenahme des Gateway systems von Invitrogen und sogenannten attB Erkennungssequenzen (Hartley J.L., Temple G.F., Brasch M.A. (2000). DNA cloning using in vitro site-specific recombination. Genome Research, 10, 1788-1795), gefolgt von dem Polyadenylierungssignal des Nopalinsynthase Gens aus *Agrobacterium tumefaciens* (Depicker et al., 1982, J Mol. Appl. Gent. 1, 561-573) zusätzlich zu der Selektionskassette zwischen die linke und rechte T-DNA Border Sequenz eingefügt. Das resultierende Plasmid wurde als pSC16 bezeichnet.

**Beispiel 2**

**Herstellung und Analyse von Maispflanzen, die eine nachweisbare GWD-Expression aufweisen**

Zehn Tage nach Pollination wurden unreife Embryonen von Maispflanzen (Varietät A188) isoliert und mit Hilfe von *Agrobacterium tumefaciens*, enthaltend das Plasmid pSC16 als Cointegrat, nach der bei Ishida et al. (1996, Nature Biotechnology 14, 745-750) beschriebenen Methode transformiert. Aus dieser Transformation hervorgegangene so genannte T0 Pflanzen wurden im Gewächshaus angezogen. Die erhaltenden Pflanzen erhielten die Bezeichnung SC16-X, wobei X unabhängige aus der Transformation hervorgegangene Pflanzen bezeichnet.

Aus der Transformation mit dem Expressionsvektor pSC16 hervorgegangene Maispflanzen (T0 Pflanzen) wurden im Gewächshaus in Erde kultiviert und mit Wildtyp (Varietät A188) Pollen bestäubt. Aus einzelnen, reifen Körnern (T1-Samen) wurde Mehl hergestellt. Dazu wurden einzelne Körner in einer Kugelmühle (Firma Retsch, Haan, Deutschland, Modell MM300) für 30 Sekunden bei einer Frequenz von 30 Hertz zerkleinert. Anschließend erfolgte eine Bestimmung des Stärkephosphatgehaltes in der C6-Position von Glucosemolekülen der Stärke wie unter Allgemeine Methoden beschrieben.

Für ausgewählte Pflanzen wurden folgende Ergebnisse erhalten:

| Bezeichnung der Pflanze | nmol C6-Phosphat pro mg Stärke |
|---|---|
| SC16-1 | 1,9 |
| SC16-2 | 1,4 |
| Sc16-3 | 1,6 |
| WT A188 | 0,1 |

Wie aus der Tabelle hervorgeht, konnten unabhängige Linien identifiziert werden, die im Vergleich zu entsprechenden nicht genetisch modifizierten Wildtyp-Pflanzen (A188) einen erhöhten Phosphatgehalt in C6-Position der Glucosemonomere der Stärke aufweisen. Die Erhöhung des Phosphatgehaltes in Linien mit der Bezeichnung pSC16 ist auf die nachweisbare Expression eines GWD-Gens aus *Curcuma longa* (SEQ ID NO. 3) zurückzuführen.

**Beispiel 3**

**Herstellung von Pflanzen, die sowohl das Amylose-Extender Merkmal als auch eine nachweisbare Expression einer Glucan-Wasser Dikinase aufweisen**

Jeweils 30 T1 Samen von Pflanzen verschiedener Linien mit der Bezeichnung SC16-1-X, die eine nachweisbare Expression eines GWD-Gens aus *Curcuma longa* aufweisen im Vergleich zu den Mais-Wildtyppflanzen der Varietät A188, wurden im Gewächshaus kultiviert und die daraus resultierenden Pflanzen mit einer 0,5% Basta® (Bayer CropScience)-Lösung besprüht. Etwa die Hälfte der behandelten Pflanzen der Linien pSC16-1-X reagierte sensitiv auf die Behandlung mit Basta®, was darauf schließen ließ, dass sie kein gegen Basta® Resistenz vermittelndes gar-Gen enthielten. Bei den resistenten Pflanzen ließ sich demnach darauf schließen, dass die T-DANN(s) an einem Ort im Genom integriert war bzw. an Orten im Genom integriert waren, die so dicht beieinander liegen, dass sie nicht segregieren. Diese Pflanzen wurden geselbstet und daraus resultierende T1S1-Samen von T1 Pflanzen, die resistent gegenüber der Behandlung mit Basta® waren, wurden erneut im Gewächshaus ausgelegt, eine Behandlung mit Basta®, wie soeben beschrieben, durchgeführt, geselbstet und resultierende T1 S2 Samen erzeugt. Anschließend wurde die gleiche Behandlung mit Basta® mit T1 S2 Pflanzen dieser Linien durchgeführt: Es konnten dabei verschiedene T1 S2 Pflanzen identifiziert werden, bei welchen alle Nachkommen resistent gegenüber Basta® waren. Dieses ließ darauf schließen, dass die Ausgangs-T1S2 Pflanzen homozygot für die integrierte T-DNA waren. T1S2 Samen von homozygoten Pflanzen der Linie SC16-1 wurden erneut ausgelegt und verschiedene Pflanzen jeweils mit Pollen der Amylose-Extender Mutante ae1 der Varietät W64A bestäubt (siehe auch Allgemeine Methoden), die vom "Maize Genetics Cooperation Stock Center", Illinois, USA bezogen wurde (http://maizecoop.cropsci.uiuc.edu/). Die daraus entstandenen Kreuzungsnachkommen wurden mit SC16 x ae1 bezeichnet.

**Beispiel 4**

**Analyse von Pflanzen, die sowohl das Amylose-Extender Merkmal als auch eine nachweisbare Expression einer Glucan-Wasser-Dikinase aufweisen**

Von den aus der in Beispiel 3 beschriebenen Kreuzung hervorgegangenen Pflanzen SC16 x ae1 wurden jeweils F1-Samen geerntet, erneut ausgelegt und geselbstet. F1S1 Samen und Pflanzen von F1 Pflanzen dieser Kreuzung wurden hinsichtlich der Kopienzahl des eingebrachten GWD-Gens aus *Curcuma longa* und des Amylose-Gehaltes untersucht. Samen, die einen Amylosegehalt von mindestens 40% aufwiesen (siehe Allgemeine Methoden), wurden kultiviert und die F1S1 Pflanzen, die dann eine Kopienzahl von 2 für das GWD-Transgen aus Curcuma (siehe Allgemeine Methoden) enthielten, wurden zur Produktion von doppelt homozygoten F1 S2-Samen im Gewächshaus verwendet. Der Nachweis der reduzierten Verzweigungsenzymaktivität erfolgte mit Hilfe von Aktivitätsgelen (siehe Figur 2 und "Allgemeine Methoden":

| Bezeichnung der Pflanze | Nr. des homozygoten Samens | % Amylose |
|---|---|---|
| SC16 × ae1 | 1 | 51,1 |
| | 2 | 49,4 |
| | 3 | 45,9 |
| | 4 | 49,8 |
| | 5 | 52,4 |
| SC 16 -1 (Mutter) | 1 | 25,5 |
| | 2 | 26,0 |
| ae1 (Vater) | 1 | 52,4 |
| | 2 | 51,9 |
| WT A188 | 1 | 26,0 |
| | 2 | 26,5 |

| Bezeichnung der Pflanze | Nr. der homozygoten Pflanze | Kopienzahl |
|---|---|---|
| SC16 × ae1 | 1 | 2 |
| | 2 | 2 |
| | 3 | 2 |
| | 4 | 2 |
| | 5 | 2 |
| SC 16 -1 (Mutter) | 1 | 2 |
| | 2 | 2 |
| ae1 (Vater) | 1 | 0 |
| | 2 | 0 |
| WT A188 | 1 | 0 |
| | 2 | 0 |

**Beispiel 5**

**Analyse der Mais-Stärke aus Mais-Körnern von doppelt homozygoten Pflanzen der Kreuzung SC16 x ae1**

1. Amylose-Gehalt

Die Ermittlung des Amylose-Gehaltes von Stärken, hergestellt aus doppelt homozygoten Samen der Kreuzung SC16 x ae1, den Elternlinien und vom Wildtyp-Varietät A188 erfolgte wie unter Allgemeine Methoden beschrieben. Es wurden folgende Ergebnisse erhalten:

| Bezeichnung der Pflanze | Amylose (% Trockengewicht) |
|---|---|
| SC16 × ae1 | 43,1 |
| SC 16 | 25,0 |
| ae1 | 52,9 |
| WT A188 | 27,0 |

2. Analyse der Viskositätseigenschaften von Maisstärke mittels Rapid Visco Analyzer (RVA)

Die Viskositätseigenschaften 10% (w/v)-iger Suspensionen verschiedener Maisstärken wurden mit Hilfe der Methode "Analyse von Maismehl mittels RVA" (AACC-Methode 61-02) untersucht:

| Probe: | Wildtyp (A188) | SC16 | ae1 | SC16 x ae1 | Amylogel® | Hylon ® VII |
|---|---|---|---|---|---|---|
| Peak-Viskosität (cP) | 1844 | 2069 | 2 | 1594 | 40 | 2 |
| Trough (cP) | 329 | 525 | 0 | 1517 | 32 | 0 |
| Breakdown (cP) | 1515 | 1544 | 2 | 77 | 8 | 2 |
| Endviskosität(cP) (12,5 Min.) | 705 | 1088 | 0 | 1580 | 42 | 0 |
| Setback (cP) (12,5 Minuten) | 376 | 563 | 0 | 63 | 10 | 0 |
| Peak Time (min) | 3,94 | 3,94 | 2,02 | 6,55 | 6,58 | 2,02 |
| Verkleisterungstemperatur (°C) | 73,25 | 72,35 | Error | 85,35 | Error | Error |

Die Ergebnisse zeigen ein deutlich verändertes RVA-Profil (siehe Figur 1) der erfindungsgemäßen Stärke der Kreuzung SC16 x ae1 im Vergleich zu den Elternlinien, vor allem zu Amylose-Extender (ae1), und im Vergleich zu herkömmlicher Hochamylose-Maisstärke (Amylogel®, Hylon® VII). Die Stärke der Amylose-Extender Linie sowie die kommerziell erhältlichen Mais-Hochamylosestärken (Amylogel®, Hylon® VII) verkleistern nicht in signifikantem Umfang, so dass auch keine Verkleisterungstemperatur in der RVA-Analyse bestimmt werden kann (ERROR). Die Stärkeprobe der Kreuzung SC16 x ae1 verkleistert bei einer deutlich höheren Temperatur als die SC16- oder Wildtyp-Stärke. Die Endviskosität der erfindungsgemäßen Stärke ist größer als bei SC16- oder Wildtyp-Stärken und zeigt ein sehr geringes Setback.

3. Quellvermögen und Heißwasser-Löslichkeit

Die Ermittlung des Quellvermögens und der Heißwasser-Löslichkeit von Stärken, hergestellt aus doppelt homozygoten Samen der Kreuzung SC16 x ae1, den Elternlinien sowie von Wildtyp-Pflanzen erfolgte wie unter Allgemeine Methoden beschrieben. Es wurden folgende Ergebnisse erhalten:

| Bezeichnung der Pflanze | Quellvermögen von Stärke [g/g] | Löslichkeit in % |
|---|---|---|
| SC16 x ae1 | 12,6 | 12,4 |
| SC 16 | 27,2 | 7,1 |
| ae1 | 5,4 | 6,9 |
| WT A188 | 23,2 | 20,9 |
| Hylon VII ® | 5,0 | 6,4 |
| Amylogel® | 5,2 | 5,6 |

Die Ergebnisse stellen den Mittelwert aus zwei unabhängigen Messungen dar. Sie zeigen, dass sowohl die Stärke der Amylose-Extender Mutante (ae) als auch die Handelsprodukte (Hylon VII ®, Amylogel®) ein deutlich geringeres Quellvermögen und eine deutlich geringere Löslichkeit aufweisen als die erfindungsgemäßen Stärken der Kreuzung SC16 x ae1.

4. Phosphatgehalt an in C6-Position der Glukosemonomere der Stärke

Die Ermittlung des C6-Phosphat Gehaltes von Stärken, hergestellt aus doppelt homozygoten Samen der Kreuzung SC16 x ae1, den Elternlinien und vom Wildtyp-Varietät A188 erfolgte wie unter Allgemeine Methoden beschrieben. Es wurden folgende Ergebnisse (Mittelwert von zwei Messungen) erhalten:

| Bezeichnung der Pflanze | nmol C6-Phosphat / mg Stärke |
|---|---|
| SC16 x ae1 | 20,9 |
| SC 16 -1 (Mutter) | 4,3 |
| ae1 (Vater) | 1,0 |
| WT A188 | 0,1 |

Insgesamt zeigt die Kombination SC16 x ae1 einen signifikanten Anstieg des Phosphatgehaltes in C6-Position.

## Patentansprüche

1. Maisstärke, die einen apparenten Amylosegehalt zwischen 35 Gew.-% und 90 Gew.-% aufweist und die in einer wässrigen Suspension in der RVA-Analyse eine messbare Verkleisterungstemperatur zeigt.

2. Maisstärke nach Anspruch 1, die einen apparenten Amylosegehalt zwischen 38 Gew.-% und 85 Gew.-% aufweist.

3. Maisstärke nach Anspruch 1, die einen apparenten Amylosegehalt zwischen 40 Gew.-% und 80 Gew.-% aufweist.

4. Maisstärke nach Anspruch 1, die einen apparenten Amylosegehalt zwischen 42 Gew.-% und 75 Gew.-% aufweist.

5. Maisstärke nach Anspruch 1, die einen apparenten Amylosegehalt zwischen 50 Gew.-% und 70 Gew.-% aufweist.

6. Maisstärke nach einem der Ansprüche 1 bis 5 , wobei die wässrige Suspension eine 10% ige (w/v) Suspension der Maisstärke nach einem der Ansprüche 1 bis 5 in Wasser ist.

7. Maisstärke nach einem der Ansprüche 1 bis 6, die in der RVA-Analyse eine Verkleisterungstemperatur von mindestens 75° C aufweist.

8. Maisstärke nach einem der Ansprüche 1 bis 6, die in der RVA-Analyse eine Verkleisterungstemperatur von mindestens 80° C aufweist.

9. Maisstärke nach einem der Ansprüche 1 bis 6, die in der RVA-Analyse eine Verkleisterungstemperatur zwischen 78°C und 95°C aufweist.

10. Maisstärke nach einem der Ansprüche 1 bis 9, die in einer wässrigen Suspension in der RVA-Analyse eine Endviskosität zwischen 1100 und 2000 Centi Poise aufweist.

11. Maisstärke nach einem der Ansprüche 1 bis 10, die in einer wässrigen Suspension in der RVA-Analyse eine Peak-Viskosität zwischen 1200 und 2000 Centi Poise aufweist.

12. Maisstärke nach einem der Ansprüche 1 bis 11, die in einer wässrigen Suspension in der RVA-Analyse ein Setback zwischen -100 und 350 Centi Poise aufweist.

13. Maisstärke nach einem der Ansprüche 1 bis 12, die eine native Mais-Stärke ist.

14. Mais-Pflanzenzelle, die ein heterologes GWD-Gen exprimiert und eine amylose extender-Mutation aufweist.

15. Mais-Pflanze enthaltend Mais-Pflanzenzellen nach Anspruch 14.

16. Mais-Pflanze nach Anspruch 15, die eine Maisstärke nach einem der Ansprüche 1 bis 13 synthetisiert.

17. Vermehrungsmaterial einer Mais-Pflanze nach Anspruch 15 oder 16 enthaltend Mais-Pflanzenzellen nach Anspruch 14.

18. Verfahren zur Herstellung einer Maisstärke nach einem der Ansprüche 1 bis 13 umfassend den Schritt der Extraktion der Maisstärke aus Mais-Pflanzen nach Anspruch 15 oder 16 oder aus Vermehrungsmaterial nach Anspruch 17.

19. Maismehl enthaltend Maisstärke nach einem der Ansprüche 1 bis 13.

**20.** Verfahren zur Herstellung von Maismehl nach Anspruch 19 umfassend den Schritt des Mahlens einer Mais-Pflanze nach Anspruch 15 oder 16 oder von Vermehrungsmaterial nach Anspruch 17.

**21.** Zusammensetzung enthaltend Maisstärke nach einem der Ansprüche 1 bis 13 und mindestens einen Zusatzstoff.

**22.** Zusammensetzung nach Anspruch 21, wobei der Zusatzstoff ein Nahrungsmittelzusatzstoff ist.

**23.** Zusammensetzung enthaltend Maismehl nach Anspruch 19 und mindestens einen Zusatzstoff.

**24.** Zusammensetzung nach Anspruch 23, wobei der Zusatzstoff ein Nahrungsmittelzusatzstoff ist.

**25.** Lebensmittel enthaltend Maisstärke nach einem der Ansprüche 1 bis 13 oder Maismehl nach Anspruch 19.

**26.** Lebensmittel enthaltend eine Zusammensetzung nach einem der Ansprüche 21 bis 24.

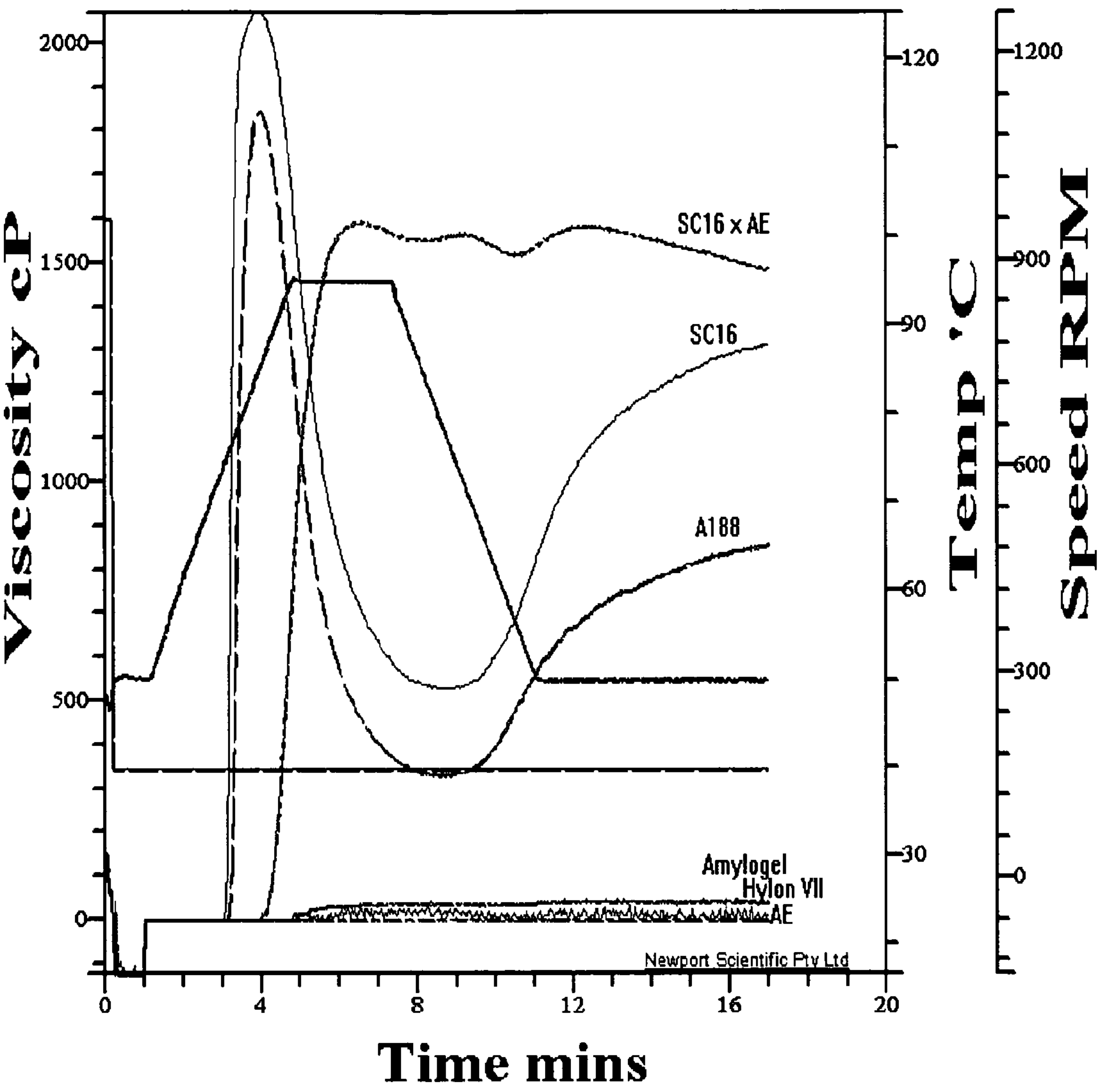
Viscosity cP
2000
1500
1000
500
0
Temp 'C
120
90
60
30
Speed RPM
1200
900
600
300
0
SC16 x AE
SC16
A188
Amylogel
Hylon VII
AE
Newport Scientific Pty Ltd
0
4
8
12
16
20
Time mins

Figur 1

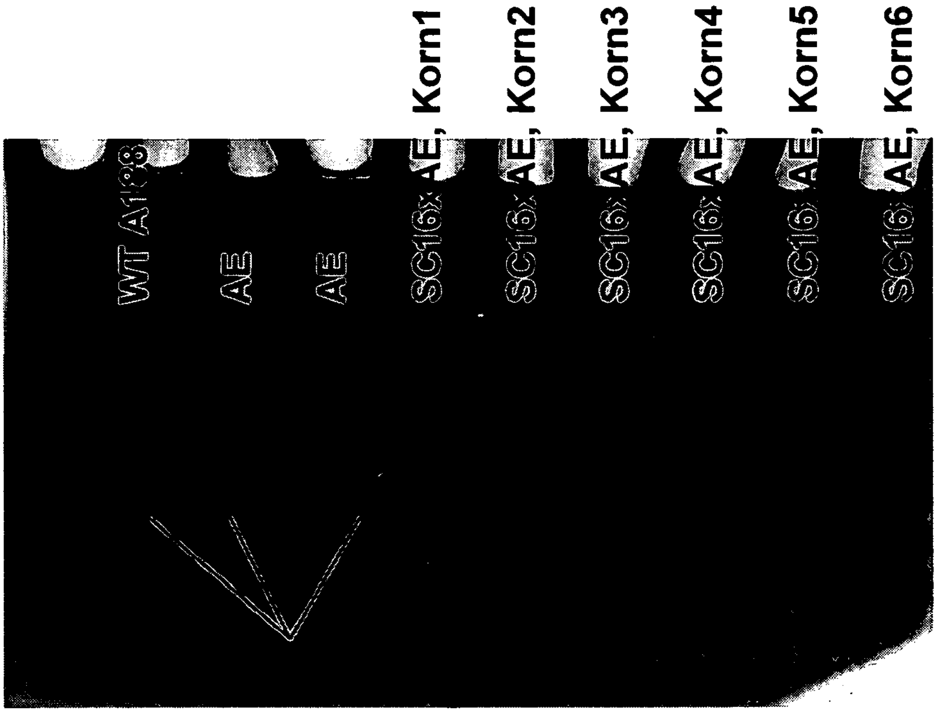
WT A188
AE
AE
SC16x AE, Korn1
SC16x AE, Korn2
SC16x AE, Korn3
SC16x AE, Korn4
SC16x AE, Korn5
SC16x AE, Korn6
2 µg/ Slot

Figur 2

Europäisches Patentamt

# EUROPÄISCHER RECHERCHENBERICHT

Nummer der Anmeldung
EP 07 07 5524

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2005/002359 A (SYNGENTA PARTICIPATIONS AG [CH]; LANAHAN MICHAEL B [US]; BASU SHIP S [) 13. Januar 2005 (2005-01-13) |  | INV. C12N15/82 C08B30/00 |
| A | WO 03/071860 A (BAYER CROPSCIENCE GMBH [DE]; FROHBERG CLAUS [DE]; BAEUERLEIN MICHAEL [) 4. September 2003 (2003-09-04) |  |  |
| A | WO 02/101059 A (BAYER CROPSCIENCE GMBH [DE]) 19. Dezember 2002 (2002-12-19) |  |  |
| A | WO 02/34923 A (AVENTIS CROPSCIENCE GMBH [DE] BAYER CROPSCIENCE GMBH [DE]) 2. Mai 2002 (2002-05-02) |  |  |
| A | US 2003/150023 A1 (KLUCINEC JEFFREY D [US] ET AL) 7. August 2003 (2003-08-07) |  |  |
| A | BLENNOW ET AL: "Structure function relationships of transgenic starches with engineered phosphate substitution and starch branching" INTERNATIONAL JOURNAL OF BIOLOGICAL MACROMOLECULES, BUTTERWORTH & CO., GUILDFORD, GB, Bd. 36, Nr. 3, August 2005 (2005-08), Seiten 159-168, XP005001313 ISSN: 0141-8130 |  | RECHERCHIERTE SACHGEBIETE (IPC) C12N C08B |
| A | KIM KYUNG-NAM ET AL: "Molecular cloning and characterization of the amylose-extender gene encoding starch branching enzyme IIB in maize" PLANT MOLECULAR BIOLOGY, Bd. 38, Nr. 6, Dezember 1998 (1998-12), Seiten 945-956, XP002464580 ISSN: 0167-4412 |  |  |

Der vorliegende Recherchenbericht wurde für alle Patentansprüche erstellt

| Recherchenort | Abschlußdatum der Recherche | Prüfer |
|---|---|---|
| München | 15. Januar 2008 | Kania, Thomas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

3
EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 07 07 5524

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-01-2008

| Im Recherchenbericht angeführtes Patentdokument | Datum der Veröffentlichung | Mitglied(er) der Patentfamilie | Datum der Veröffentlichung |
|---|---|---|---|
| WO 2005002359 A | 13-01-2005 | BR PI0410544 A | 20-06-2006 |
| | | CA 2526480 A1 | 13-01-2005 |
| | | CN 1826412 A | 30-08-2006 |
| | | EP 1629102 A2 | 01-03-2006 |
| WO 03071860 A | 04-09-2003 | AU 2003222749 A1 | 09-09-2003 |
| | | DE 10208132 A1 | 11-09-2003 |
| | | EP 1481070 A2 | 01-12-2004 |
| | | US 2006150278 A1 | 06-07-2006 |
| WO 02101059 A | 19-12-2002 | CA 2465884 A1 | 19-12-2002 |
| | | EP 1483390 A2 | 08-12-2004 |
| | | JP 2004537990 T | 24-12-2004 |
| | | US 2003126633 A1 | 03-07-2003 |
| | | US 2007174932 A1 | 26-07-2007 |
| WO 0234923 A | 02-05-2002 | AU 2171602 A | 06-05-2002 |
| | | CA 2421679 A1 | 02-05-2002 |
| | | CN 1541273 A | 27-10-2004 |
| | | EP 1328651 A2 | 23-07-2003 |
| | | JP 2004512048 T | 22-04-2004 |
| | | PL 365536 A1 | 10-01-2005 |
| | | US 2003110534 A1 | 12-06-2003 |
| US 2003150023 A1 | 07-08-2003 | US 2006216402 A1 | 28-09-2006 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9711188 A
- WO 9827212 A
- WO 0077229 A, Weizen
- US 6462256 B
- AR 236165
- US 6620987 B
- AR 400814
- AR 400813
- AR 400815
- US 60701693 B
- WO 2007018770 A
- WO 2005002359 A
- US 20060282917 A1
- WO 9704112 A
- WO 9704113 A
- WO 9837213 A
- WO 9837214 A
- EP 0321201 B1
- WO 9515972 A
- WO 9953050 A
- US 6750378 B
- WO 0173087 A


### In der Beschreibung aufgeführte Nicht-Patentliteratur


- Methoden basieren auf dem Iodbindevermögen der Amylose, das potentiometrisch. Edinburgh University Press, 51-66
- **LARSON et al.** *Analytical Chemistry,* 1953, vol. 25 (5), 802-804
- **MORRISON ; LAIGNELET.** *J. Cereal Sc.,* 1983, vol. 1, 9-20
- **KUGIMIYA ; DONOVAN.** *Journal of Food Science,* 1981, vol. 46, 765-770
- **SIEVERT ; HOLM.** *Starch/Stärke,* 1993, vol. 45 (4), 136-139
- **GÉRARD et al.** *Carbohydrate Polymers,* 2001, vol. 44, 19-27
- **VINEYARD ; BEAR.** *Maize Genet Coop Newsletter,* 1952, vol. 26, 5
- **MOORE ; CREECH.** *Genetics,* 1972, vol. 70, 611-619
- **GARWOOD et al.** *Cereal Chemistry,* 1976, vol. 53 (3), 355-364
- **HEDMAN ; BOYER.** *Biochemical Genetics,* 1983, vol. 21 (11/12), 1217-1222
- **FISHER et al.** *Plant Physiol.,* 1996, vol. 110, 611-619
- **SHI et al.** *Journal of Cereal Science,* 1998, vol. 27, 289-299
- **SENTI ; RUSSELL.** *Tappi,* 1960, vol. 43 (4), 343-349
- **VON LEACH et al.** *Cereal Chemistry,* 1959, vol. 36, 534-544
- **NIELSEN et al.** *Plant Physiol.,* 1994, vol. 105, 111-117
- **ORTHOEFER.** Corn, Chemistry and Technology. 1987, 479-499
- **RITTE et al.** *PNAS,* 2002, vol. 99, 7166-7171
- **KÖTTING et al.** *Plant Physiol.,* 2005, vol. 137, 2424-252
- **BAUNSGAARD et al.** *Plant Journal,* 2005, vol. 41, 595-605
- **RITTE et al.** *PNAS,* 2005, vol. 99, 7166-7171
- **LORBERTH et al.** *Nature Biotechnology,* 1998, vol. 16, 473-477
- **RITTE et al.** *Planta,* 2003, vol. 216, 798-801
- **TIEN-SHIN YU et al.** *Plant Cell,* 2001, vol. 13, 1907-1918
- **MIKKELSEN et al.** *Biochemical Journal,* 2004, vol. 377, 525-532
- **MIKKELSEN ; BLENNOW.** *Biochemical Journal,* 2005, vol. 385, 355-361
- **MIKKELSEN et al.** *Biochemistry,* 2006, vol. 45, 4674-4682
- **THOMPSON et al.** *Nukleic Acids Research,* 1994, vol. 22, 4673-4680
- **HEDMAN ; BOYER.** *Biochemical Genetics,* 1983, vol. 21 (11-12), 1217-1222
- **KIM et al.** *Plant Molecular Biology,* 1998, vol. 38, 945-956
- **VON FISHER et al.** *Plant Physiol.,* 1996, vol. 110, 611-619
- **GUAN ; PREISS.** *Plant Physiol.,* 1993, vol. 102, 1269-1273
- **VOM BELLA-PROTEIN LAUT GAO et al.** *Plant Physiol.,* 1997, vol. 114, 69-78
- **STINARD et al.** *Plant Cell,* 1993, vol. 5, 1555-1566

- **HEDMAN ; BOYER.** *Biochemical Genetics,* 1982, vol. 20 (5/6), 483-492
- *Maize Genet Coop Newsletter,* 1952, vol. 26, 5
- **RAHMAN et al.** *Plant Physiology,* 2001, vol. 125, 1314-1324
- **REGINA et al.** *Planta,* 2005, vol. 222 (5), 899-909
- **FISHER et al.** *Plant Physiology,* 1996, vol. 110, 611-619
- **TETLOW et al.** *Plant Cell,* 2004, vol. 16, 694-708
- **VON EHRENBERG ; HUSAIN.** *Mutation Research,* 1981, vol. 86, 1-113
- **MÜLLER.** *Biologisches Zentralblatt,* 1972, vol. 91 (1), 31-48
- **VON JAUHAR ; SIDDIQ.** *Indian Journal of Genetics,* 1999, vol. 59 (1), 23-28
- **RAO.** *Cytologica,* 1977, vol. 42, 443-450
- **GUPTA ; SHARMA.** *Oryza,* 1990, vol. 27, 217-219
- **SATOH ; OMURA.** *Japanese Journal of Breeding,* 1981, vol. 31 (3), 316-326
- **VON ARORA et al.** *Annals of Biology,* 1992, vol. 8 (1), 65-69
- **VON SCARASCIA-MUGNOZZA et al.** *Mutation Breeding Review,* 1993, vol. 10, 1-28
- **SVEC et al.** *Cereal Research Communications,* 1998, vol. 26 (4), 391-396
- **VON SHASHIDHARA et al.** *Journal of Maharashtra Agricultural Universities,* 1990, vol. 15 (1), 20-23
- **THORNEYCROFT et al.** *Journal of experimental Botany,* 2001, vol. 52 (361), 1593-1601
- **RAMACHANDRAN ; SUNDARESAN.** *Plant Physiology and Biochemistry,* 2001, vol. 39, 234-252
- **B. WALDEN et al.** *Plant J.,* 1991, 281-288
- **WALDEN et al.** *Plant Mol. Biol.,* 1994, vol. 26, 1521-1528
- **NAM et al.** *The Plant Cell,* 1989, vol. 1, 699-705
- **LEISTER ; DEAN.** *The Plant Journal,* 1993, vol. 4 (4), 745-750
- **CASTIGLIONI et al.** *Genetics,* 1998, vol. 149, 2039-2056
- **MEKSEM et al.** *Molecular Genetics and Genomics,* 2001, vol. 265, 207-214
- **MEYER et al.** *Molecular and General Genetics,* 1998, vol. 259, 150-160
- **KONIECZNY ; AUSUBEL.** *The Plant Journal,* 1993, vol. 4, 403-410
- **JARVIS et al.** *Plant Mol. Biol.,* 1994, vol. 24, 685-687
- **BACHEM et al.** *The Plant Journal,* 1996, vol. 9 (5), 745-753
- **VON QI et al.** *Nukleic Acids Research,* 2001, vol. 29 (22), 116
- **DRENKARD et al.** *Plant Physiology,* 2000, vol. 124, 1483-1492
- **CHO et al.** *Nature Genetics,* 1999, vol. 23, 203-207
- **VON MCCALLUM et al.** *Plant Physiology,* 2000, vol. 123, 439-442
- **SAMBROK et al.** Molecular Cloning, A Laboratory Manual. Cold Spring Harbour Laboratory Press, 2001
- **AUSUBEL et al.** Short Protocols in Molecular Biology. John Wiley & Sons, 2002
- **METTE et al.** *EMBO J.,* 2000, vol. 19, 5194-5201
- **VON JORGENSEN.** *Trends Biotechnol.,* 1990, vol. 8, 340-344
- **NIEBEL et al.** *Top. Microbiol. Immunol.,* 1995, vol. 197, 91-103
- **FLAVELL et al.** *Curr. Top. Microbiol. Immunol.,* 1995, vol. 197, 43-46
- **PALAUQUI ; VAUCHERET.** *Plant Mol. Biol.,* 1995, vol. 29, 149-159
- **VAUCHERET et al.** *Mol. Gen. Genet.,* 1995, vol. 248, 311-317
- **DE BORNE et al.** *Mol.. Gen. Genet.,* 1994, vol. 243, 613-621
- **VON FEYTER et al.** *Mol. Gen. Genet.,* 1996, vol. 250, 329-338
- **KIPP et al.** *Poster Session beim 5th International Congress of Plant Molecular Biology,* 21. September 1997
- **R. A. DIXON ; C. J. ARNTZEN.** Meeting report zu Metabolic Engineering in Transgenic Plants. *TIBTECH,* 1997, vol. 15, 441-447
- **KREN et al.** *Hepatology,* 1997, vol. 25, 1462-1468
- **COLE-STRAUSS et al.** *Science,* 1996, vol. 273, 1386-1389
- **BEETHAM et al.** *PNAS,* 1999, vol. 96, 8774-8778
- **WATERHOUSE et al.** *Proc. Natl. Acad. Sci. USA,* 1998, vol. 95, 13959-13964
- **WANG ; WATERHOUSE.** *Plant Mol. Biol.,* 2000, vol. 43, 67-82
- **SINGH et al.** *Biochemical Society Transactions,* 2000, vol. 28 (6), 925-927
- **LIU et al.** *Biochemical Society Transactions,* 2000, vol. 28 (6), 927-929
- **SMITH et al.** *Nature,* 2000, vol. 407, 319-320
- **NAP et al.** *6th International Congress of Plant Molecular Biology,* 18. Juni 2000
- **OWEN.** *Bio/Technology,* 1992, vol. 10, 790-794
- **FRANKEN et al.** *Current Opinion in Biotechnology,* 1997, vol. 8, 411-416
- **WHITELAM.** *Trends Plant Sci.,* 1996, vol. 1, 268-272
- **ODELL et al.** *Nature,* 1985, vol. 313, 810-812

- **CHRISTENSEN et al.** *Plant Mol. Biol.,* 1992, vol. 18, 675-689
- **LIU et al.** *Plant Science,* 2003, 165
- **ZHANG et al.** *Plant Cell,* 1991, vol. 3, 1150-1160
- **VERDAGUER.** *Plant Mol. Biol.,* vol. 31, 1129-1139
- **STAVOLONE et al.** *Plant Mol. Biol.,* 2003, vol. 53, 703-713
- **KIRIHARA et al.** *Gene,* 1988, vol. 71, 359-370
- **HOFFMANN et al.** *EMBO J.,* 1987, vol. 6, 3213-3221
- **SCHEMTHANER et al.** *EMBO J.,* 1988, vol. 7, 1249-1253
- **WILLIAMSON et al.** *Plant Physiol.,* 1988, vol. 88, 1002-1007
- **PRAT et al.** *Gene,* 1987, vol. 52, 51-49
- **GALLARDO et al.** *Plant Sci.,* 1988, vol. 54, 211-281
- **MARKS et al.** *J. Biol. Chem.,* 1985, vol. 260, 16451-16459
- **KODRZYCK et al.** *Plant Cell,* 1989, vol. 1, 105-114
- **YANG, N.-S. ; RUSSEL, D.** *Proc. Natl. Acad Sci,* 1990, vol. 87, 4144-4148
- **UNGER et al.** *Plant Physiol.,* 1991, vol. 96, 124
- **BHAVE et al.** *Plant Cell,* 1990, vol. 2, 581-588
- **BAE et al.** *Maydica,* 1990, vol. 35, 317-322
- **COLOT et al.** *EMBO J.,* 1987, vol. 6, 3559-3564
- **CLARKE ; APPELS.** *Genome,* 1998, vol. 41, 865-871
- **PEDERSEN et al.** *Cell,* 1982, vol. 29, 1015-1026
- **QUATROCCIO et al.** *Plant Mol. Biol.,* 1990, vol. 15, 81-93
- **LEISY et al.** *Plant Mol. Biol.,* 1990, vol. 14, 41-50
- **ZHENG et al.** *Plant J.,* 1993, vol. 4, 357-366
- **YOSHIHARA et al.** *FEBS Lett.,* 1996, vol. 383, 213-218
- **NAKASE et al.** *Gene,* 1996, vol. 170 (2), 223-226
- **QU ; TAKAIWA.** *Plant Biotechnology Journal,* 2004, vol. 2 (2), 113-125
- **CALLIS et al.** *Genes Devel.,* 1987, vol. 1, 1183-1200
- **LUEHRSEN ; WALBOT.** *Mol. Gen. Genet.,* 1991, vol. 225, 81-93
- **RETHMEIER et al.** *Plant Journal.,* 1997, vol. 12 (4), 895-899
- **ROSE ; BELIAKOFF.** *Plant Physiol.,* 2000, vol. 122 (2), 535-542
- **VASIL et al.** *Plant Physiol.,* 1989, vol. 91, 1575-1579
- **XU et al.** *Science in China Series C,* 2003, vol. 46 (6), 561-569
- **MAAS et al.** *Plant. Mol. Biol.,* 1991, vol. 16, 199-207
- **CALLIS et al.** *Genes Dev.,* 1987, vol. 1, 1183-1200
- **LUEHRSEN, K. R. ; WALBOT, V.** *Mol. Gen. Genet.,* 1991, vol. 225, 81-93
- **OARD et al.** *Plant Cell Rep,* 1989, vol. 8, 156-160
- Mais und Sorghum Stärken: Herstellung. Starch: Chemistry and Technology. Academic Press Inc. London Ltd, 1994, 412-468
- **VON WATSON.** STARCH: CHEMISTRY AND TECHNOLOGY. 491-506
- **VON KNIGHT ; OSON.** *Weizenstärke: Herstellung, Modifizierung und Verwendungen*
- **ECKHOFF et al.** *Cereal Chem.,* 1996, vol. 73, 54-57
- **VON ISHIDA et al.** *Nature Biotechnology,* 1996, vol. 14, 745-750
- **JONES H.D. ; DOHERTY A. ; WU H.** Review of methodologies and a protocol for the Agrobacterium-mediated transformation of wheat. *Plant Methods 2005,* 2005, vol. 1, 5
- **VON JULIANO.** *Cereal Science Today,* 1971, vol. 16 (10), 334-340
- **BRADFORD.** *Anal Biochem,* 1976, vol. 72, 248-254
- **M. W. PFAFFL.** A new mathematical model for relative quantification in real-time RT-PCR. *Nukleic Acids Research,* 2001, vol. 29 (9 00
- **BOLIVAR et al.** *Gene,* 1977, vol. 2, 95-113
- **WOHLLEBEN et al.** *MGG,* 1989, vol. 217, 202-208
- **WHITE et al.** *NAR,* 1990, vol. 18, 1062
- **MCELROY et al.** *Plant Cell,* 1990, vol. 2, 163.171
- **DEPICKER et al.** *J Mol. Appl. Gent.,* 1982, vol. 1, 561-573
- **CHRISTENSEN et al.** *Plant Mol. Bio,* 1992, vol. 18, 675-689
- **HARTLEY J.L. ; TEMPLE G.F. ; BRASCH M.A.** DNA cloning using in vitro site-specific recombination. *Genome Research,* 2000, vol. 10, 1788-1795
- **LSHIDA et al.** *Nature Biotechnology,* 1996, vol. 14, 745-750

SEQUENCE LISTING

<110> Bayer CropScience AG

<120> Stärken mit hohem Amylosegehalt und verbesserten Verarbeitungseigenschaften

<130> BCS 07-5006 EP

<160> 16

<170> PatentIn version 3.3

<210> 1
<211> 4851
<212> DNA
<213> Solanum tuberosum

<220>
<221> transit_peptide
<222> (1)..(77)

<220>
<221> CDS
<222> (105)..(4499)

<300>
<308> EMBL / Y09533
<309> 1998-07-30
<313> (1)..(4499)

<400> 1

```
catcttcatc gaatttctcg aagcttcttc gctaatttcc tggtttcttc actcaaaatc      60

gacgtttcta gctgaacttg agtgaattaa gccagtggga ggat atg agt aat tcc      116
                                                 Met Ser Asn Ser
                                                 1

tta ggg aat aac ttg ctg tac cag gga ttc cta acc tca aca gtg ttg       164
Leu Gly Asn Asn Leu Leu Tyr Gln Gly Phe Leu Thr Ser Thr Val Leu
5                   10                  15                  20

gaa cat aaa agt aga atc agt cct cct tgt gtt gga ggc aat tct ttg       212
Glu His Lys Ser Arg Ile Ser Pro Pro Cys Val Gly Gly Asn Ser Leu
                25                  30                  35

ttt caa caa caa gtg atc tcg aaa tca cct tta tca act gag ttt cga       260
Phe Gln Gln Gln Val Ile Ser Lys Ser Pro Leu Ser Thr Glu Phe Arg
            40                  45                  50
```

```
ggt aac agg tta aag gtg cag aaa aag aaa ata cct atg gaa aag aag      308
Gly Asn Arg Leu Lys Val Gln Lys Lys Lys Ile Pro Met Glu Lys Lys
        55                  60                  65

cgt gct ttt tct agt tct cct cat gct gta ctt acc act gat acc tct      356
Arg Ala Phe Ser Ser Ser Pro His Ala Val Leu Thr Thr Asp Thr Ser
    70                  75                  80

tct gag cta gca gaa aag ttc agt cta ggg ggg aat att gag cta cag      404
Ser Glu Leu Ala Glu Lys Phe Ser Leu Gly Gly Asn Ile Glu Leu Gln
85                  90                  95                  100

gtt gat gtt agg cct ccc act tca ggt gat gtg tcc ttt gtg gat ttt      452
Val Asp Val Arg Pro Pro Thr Ser Gly Asp Val Ser Phe Val Asp Phe
                105                 110                 115

caa gta aca aat ggt agt gat aaa ctg ttt ttg cac tgg ggg gca gta      500
Gln Val Thr Asn Gly Ser Asp Lys Leu Phe Leu His Trp Gly Ala Val
            120                 125                 130

aaa ttc ggg aaa gaa aca tgg tct ctt ccg aat gat cgt cca gat ggg      548
Lys Phe Gly Lys Glu Thr Trp Ser Leu Pro Asn Asp Arg Pro Asp Gly
        135                 140                 145

acc aaa gtg tac aag aac aaa gca ctt aga act cca ttt gtt aaa tct      596
Thr Lys Val Tyr Lys Asn Lys Ala Leu Arg Thr Pro Phe Val Lys Ser
    150                 155                 160

ggc tct aac tcc atc ctg aga ctg gag ata cga gac act gct atc gaa      644
Gly Ser Asn Ser Ile Leu Arg Leu Glu Ile Arg Asp Thr Ala Ile Glu
165                 170                 175                 180

gct att gag ttt ctc ata tac gat gaa gcc cac gat aaa tgg ata aag      692
Ala Ile Glu Phe Leu Ile Tyr Asp Glu Ala His Asp Lys Trp Ile Lys
                185                 190                 195

aat aat ggt ggt aat ttt cgt gtc aaa ttg tca aga aaa gag ata cga      740
Asn Asn Gly Gly Asn Phe Arg Val Lys Leu Ser Arg Lys Glu Ile Arg
            200                 205                 210

ggc cca gat gtt tct gtt cct gag gag ctt gta cag atc caa tca tat      788
Gly Pro Asp Val Ser Val Pro Glu Glu Leu Val Gln Ile Gln Ser Tyr
        215                 220                 225

ttg agg tgg gag agg aag gga aaa cag aat tac ccc cct gag aaa gag      836
Leu Arg Trp Glu Arg Lys Gly Lys Gln Asn Tyr Pro Pro Glu Lys Glu
    230                 235                 240

aag gag gaa tat gag gct gct cga act gtg cta cag gag gaa ata gct      884
```

```
Lys Glu Glu Tyr Glu Ala Ala Arg Thr Val Leu Gln Glu Glu Ile Ala
245                 250                 255                 260

cgt ggt gct tcc ata cag gac att cga gca agg cta aca aaa act aat       932
Arg Gly Ala Ser Ile Gln Asp Ile Arg Ala Arg Leu Thr Lys Thr Asn
                265                 270                 275

gat aaa agt caa agc aaa gaa gag cct ctt cat gta aca aag agt gat       980
Asp Lys Ser Gln Ser Lys Glu Glu Pro Leu His Val Thr Lys Ser Asp
            280                 285                 290

ata cct gat gac ctt gcc caa gca caa gct tac att agg tgg gag aaa      1028
Ile Pro Asp Asp Leu Ala Gln Ala Gln Ala Tyr Ile Arg Trp Glu Lys
        295                 300                 305

gca gga aag ccg aac tat cct cca gaa aag caa att gaa gaa ctc gaa      1076
Ala Gly Lys Pro Asn Tyr Pro Pro Glu Lys Gln Ile Glu Glu Leu Glu
    310                 315                 320

gaa gca aga aga gaa ttg caa ctt gag ctt gag aaa ggc att acc ctt      1124
Glu Ala Arg Arg Glu Leu Gln Leu Glu Leu Glu Lys Gly Ile Thr Leu
325                 330                 335                 340

gat gag ttg cgg aaa acg att aca aaa ggg gag ata aaa act aag gtg      1172
Asp Glu Leu Arg Lys Thr Ile Thr Lys Gly Glu Ile Lys Thr Lys Val
                345                 350                 355

gaa aag cac ctg aaa aga agt tct ttt gcc gtt gaa aga atc caa aga      1220
Glu Lys His Leu Lys Arg Ser Ser Phe Ala Val Glu Arg Ile Gln Arg
            360                 365                 370

aag aag aga gac ttt ggg cat ctt att aat aag tat act tcc agt cct      1268
Lys Lys Arg Asp Phe Gly His Leu Ile Asn Lys Tyr Thr Ser Ser Pro
        375                 380                 385

gca gta caa gta caa aag gtc ttg gaa gaa cca cca gcc tta tct aaa      1316
Ala Val Gln Val Gln Lys Val Leu Glu Glu Pro Pro Ala Leu Ser Lys
    390                 395                 400

att aag ctg tat gcc aag gag aag gag gag cag att gat gat ccg atc      1364
Ile Lys Leu Tyr Ala Lys Glu Lys Glu Glu Gln Ile Asp Asp Pro Ile
405                 410                 415                 420

cta aat aaa aag atc ttt aag gtc gat gat ggg gag cta ctg gta ctg      1412
Leu Asn Lys Lys Ile Phe Lys Val Asp Asp Gly Glu Leu Leu Val Leu
                425                 430                 435

gta gca aag tcc tct ggg aag aca aaa gta cat cta gct aca gat ctg      1460
Val Ala Lys Ser Ser Gly Lys Thr Lys Val His Leu Ala Thr Asp Leu
            440                 445                 450
```

aat cag cca att act ctt cac tgg gca tta tcc aaa agt cct gga gag 1508
Asn Gln Pro Ile Thr Leu His Trp Ala Leu Ser Lys Ser Pro Gly Glu
455 460 465

tgg atg gta cca cct tca agc ata ttg cct cct ggg tca att att tta 1556
Trp Met Val Pro Pro Ser Ser Ile Leu Pro Pro Gly Ser Ile Ile Leu
470 475 480

gac aag gct gcc gaa aca cct ttt tca gcc agt tct tct gat ggt cta 1604
Asp Lys Ala Ala Glu Thr Pro Phe Ser Ala Ser Ser Ser Asp Gly Leu
485 490 495 500

act tct aag gta caa tct ttg gat ata gta att gaa gat ggc aat ttt 1652
Thr Ser Lys Val Gln Ser Leu Asp Ile Val Ile Glu Asp Gly Asn Phe
505 510 515

gtg ggg atg cca ttt gtt ctt ttg tct ggt gaa aaa tgg att aag aac 1700
Val Gly Met Pro Phe Val Leu Leu Ser Gly Glu Lys Trp Ile Lys Asn
520 525 530

caa ggg tcg gat ttc tat gtt ggc ttc agt gct gca tcc aaa tta gca 1748
Gln Gly Ser Asp Phe Tyr Val Gly Phe Ser Ala Ala Ser Lys Leu Ala
535 540 545

ctc aag gct gct ggg gat ggc agt gga act gca aag tct tta ctg gat 1796
Leu Lys Ala Ala Gly Asp Gly Ser Gly Thr Ala Lys Ser Leu Leu Asp
550 555 560

aaa ata gca gat atg gaa agt gag gct cag aag tca ttt atg cac cgg 1844
Lys Ile Ala Asp Met Glu Ser Glu Ala Gln Lys Ser Phe Met His Arg
565 570 575 580

ttt aat att gca gct gac ttg ata gaa gat gcc act agt gct ggt gaa 1892
Phe Asn Ile Ala Ala Asp Leu Ile Glu Asp Ala Thr Ser Ala Gly Glu
585 590 595

ctt ggt ttt gct gga att ctt gta tgg atg agg ttc atg gct aca agg 1940
Leu Gly Phe Ala Gly Ile Leu Val Trp Met Arg Phe Met Ala Thr Arg
600 605 610

caa ctg ata tgg aac aaa aac tat aac gta aaa cca cgt gaa ata agc 1988
Gln Leu Ile Trp Asn Lys Asn Tyr Asn Val Lys Pro Arg Glu Ile Ser
615 620 625

aag gct cag gac aga ctt aca gac ttg ttg cag aat gct ttc acc agt 2036
Lys Ala Gln Asp Arg Leu Thr Asp Leu Leu Gln Asn Ala Phe Thr Ser
630 635 640

cac cct cag tac cgt gaa att ttg cgg atg att atg tca act gtt gga 2084

```
His Pro Gln Tyr Arg Glu Ile Leu Arg Met Ile Met Ser Thr Val Gly
645                 650                 655                 660

cgt gga ggt gaa ggg gat gta gga cag cga att agg gat gaa att ttg      2132
Arg Gly Gly Glu Gly Asp Val Gly Gln Arg Ile Arg Asp Glu Ile Leu
                665                 670                 675

gtc atc cag agg aac aat gac tgc aag ggt ggt atg atg caa gaa tgg      2180
Val Ile Gln Arg Asn Asn Asp Cys Lys Gly Gly Met Met Gln Glu Trp
            680                 685                 690

cat cag aaa ttg cat aat aat act agt cct gat gat gtt gtg atc tgt      2228
His Gln Lys Leu His Asn Asn Thr Ser Pro Asp Asp Val Val Ile Cys
        695                 700                 705

cag gca tta att gac tac atc aag agt gat ttt gat ctt ggt gtt tat      2276
Gln Ala Leu Ile Asp Tyr Ile Lys Ser Asp Phe Asp Leu Gly Val Tyr
    710                 715                 720

tgg aaa acc ctg aat gag aac gga ata aca aaa gag cgt ctt ttg agt      2324
Trp Lys Thr Leu Asn Glu Asn Gly Ile Thr Lys Glu Arg Leu Leu Ser
725                 730                 735                 740

tat gac cgt gct atc cat tct gaa cca aat ttt aga gga gat caa aag      2372
Tyr Asp Arg Ala Ile His Ser Glu Pro Asn Phe Arg Gly Asp Gln Lys
                745                 750                 755

ggt ggt ctt ttg cgt gat tta ggt cac tat atg aga aca ttg aag gca      2420
Gly Gly Leu Leu Arg Asp Leu Gly His Tyr Met Arg Thr Leu Lys Ala
            760                 765                 770

gtt cat tca ggt gca gat ctt gag tct gct att gca aac tgc atg ggc      2468
Val His Ser Gly Ala Asp Leu Glu Ser Ala Ile Ala Asn Cys Met Gly
        775                 780                 785

tac aaa act gag gga gaa ggc ttt atg gtt gga gtc cag ata aat cct      2516
Tyr Lys Thr Glu Gly Glu Gly Phe Met Val Gly Val Gln Ile Asn Pro
    790                 795                 800

gta tca ggc ttg cca tct ggc ttt cag gac ctc ctc cat ttt gtc tta      2564
Val Ser Gly Leu Pro Ser Gly Phe Gln Asp Leu Leu His Phe Val Leu
805                 810                 815                 820

gac cat gtg gaa gat aaa aat gtg gaa act ctt ctt gag aga ttg cta      2612
Asp His Val Glu Asp Lys Asn Val Glu Thr Leu Leu Glu Arg Leu Leu
                825                 830                 835

gag gct cgt gag gag ctt agg ccc ttg ctt ctc aaa cca aac aac cgt      2660
Glu Ala Arg Glu Glu Leu Arg Pro Leu Leu Leu Lys Pro Asn Asn Arg
            840                 845                 850
```

```
cta aag gat ctg ctg ttt ttg gac ata gca ctt gat tct aca gtt aga      2708
Leu Lys Asp Leu Leu Phe Leu Asp Ile Ala Leu Asp Ser Thr Val Arg
            855                 860                 865

aca gca gta gaa agg gga tat gaa gaa ttg aac aac gct aat cct gag      2756
Thr Ala Val Glu Arg Gly Tyr Glu Glu Leu Asn Asn Ala Asn Pro Glu
        870                 875                 880

aaa atc atg tac ttc atc tcc ctc gtt ctt gaa aat ctc gca ctc tct      2804
Lys Ile Met Tyr Phe Ile Ser Leu Val Leu Glu Asn Leu Ala Leu Ser
885                 890                 895                 900

gtg gac gat aat gaa gat ctt gtt tat tgc ttg aag gga tgg aat caa      2852
Val Asp Asp Asn Glu Asp Leu Val Tyr Cys Leu Lys Gly Trp Asn Gln
                905                 910                 915

gct ctt tca atg tcc aat ggt ggg gac aac cat tgg gct tta ttt gca      2900
Ala Leu Ser Met Ser Asn Gly Gly Asp Asn His Trp Ala Leu Phe Ala
            920                 925                 930

aaa gct gtg ctt gac aga acc cgt ctt gca ctt gca agc aag gca gag      2948
Lys Ala Val Leu Asp Arg Thr Arg Leu Ala Leu Ala Ser Lys Ala Glu
            935                 940                 945

tgg tac cat cac tta ttg cag cca tct gcc gaa tat cta gga tca ata      2996
Trp Tyr His His Leu Leu Gln Pro Ser Ala Glu Tyr Leu Gly Ser Ile
        950                 955                 960

ctt ggg gtg gac caa tgg gct ttg aac ata ttt act gaa gaa att ata      3044
Leu Gly Val Asp Gln Trp Ala Leu Asn Ile Phe Thr Glu Glu Ile Ile
965                 970                 975                 980

cgt gct gga tca gca gct tca tta tcc tct ctt ctt aat aga ctc gat      3092
Arg Ala Gly Ser Ala Ala Ser Leu Ser Ser Leu Leu Asn Arg Leu Asp
                985                 990                 995

ccc gtg ctt cgg  aaa act gca aat cta  gga agt tgg cag att  atc       3137
Pro Val Leu Arg  Lys Thr Ala Asn Leu  Gly Ser Trp Gln Ile  Ile
            1000                 1005                 1010

agt cca gtt gaa  gcc gtt gga tat gtt  gtc gtt gtg gat gag  ttg       3182
Ser Pro Val Glu  Ala Val Gly Tyr Val  Val Val Val Asp Glu  Leu
            1015                 1020                 1025

ctt tca gtt cag  aat gaa atc tac gag  aag ccc acg atc tta  gta       3227
Leu Ser Val Gln  Asn Glu Ile Tyr Glu  Lys Pro Thr Ile Leu  Val
            1030                 1035                 1040

gca aaa tct gtt  aaa gga gag gag gaa  att cct gat ggt gct  gtt       3272
```

```
Ala Lys Ser Val  Lys Gly Glu Glu Glu  Ile Pro Asp Gly Ala  Val
            1045                 1050                 1055

gcc ctg ata aca  cca gac atg cca gat  gtt ctt tca cat gtt  tct      3317
Ala Leu Ile Thr  Pro Asp Met Pro Asp  Val Leu Ser His Val  Ser
            1060                 1065                 1070

gtt cga gct aga  aat ggg aag gtt tgc  ttt gct aca tgc ttt  gat      3362
Val Arg Ala Arg  Asn Gly Lys Val Cys  Phe Ala Thr Cys Phe  Asp
            1075                 1080                 1085

ccc aat ata ttg  gct gac ctc caa gca  aag gaa gga agg att  ttg      3407
Pro Asn Ile Leu  Ala Asp Leu Gln Ala  Lys Glu Gly Arg Ile  Leu
            1090                 1095                 1100

ctc tta aag cct  aca cct tca gac ata  atc tat agt gag gtg  aat      3452
Leu Leu Lys Pro  Thr Pro Ser Asp Ile  Ile Tyr Ser Glu Val  Asn
            1105                 1110                 1115

gag att gag ctc  caa agt tca agt aac  ttg gta gaa gct gaa  act      3497
Glu Ile Glu Leu  Gln Ser Ser Ser Asn  Leu Val Glu Ala Glu  Thr
            1120                 1125                 1130

tca gca aca ctt  aga ttg gtg aaa aag  caa ttt ggt ggt tgt  tac      3542
Ser Ala Thr Leu  Arg Leu Val Lys Lys  Gln Phe Gly Gly Cys  Tyr
            1135                 1140                 1145

gca ata tca gca  gat gaa ttc aca agt  gaa atg gtt gga gct  aaa      3587
Ala Ile Ser Ala  Asp Glu Phe Thr Ser  Glu Met Val Gly Ala  Lys
            1150                 1155                 1160

tca cgt aat att  gca tat ctg aaa gga  aaa gtg cct tcc tcg  gtg      3632
Ser Arg Asn Ile  Ala Tyr Leu Lys Gly  Lys Val Pro Ser Ser  Val
            1165                 1170                 1175

gga att cct acg  tca gta gct ctt cca  ttt gga gtc ttt gag  aaa      3677
Gly Ile Pro Thr  Ser Val Ala Leu Pro  Phe Gly Val Phe Glu  Lys
            1180                 1185                 1190

gta ctt tca gac  gac ata aat cag gga  gtg gca aaa gag ttg  caa      3722
Val Leu Ser Asp  Asp Ile Asn Gln Gly  Val Ala Lys Glu Leu  Gln
            1195                 1200                 1205

att ctg atg aaa  aaa cta tct gaa gga  gac ttc agc gct ctt  ggt      3767
Ile Leu Met Lys  Lys Leu Ser Glu Gly  Asp Phe Ser Ala Leu  Gly
            1210                 1215                 1220

gaa att cgc aca  acg gtt tta gat ctt  tca gca cca gct caa  ttg      3812
Glu Ile Arg Thr  Thr Val Leu Asp Leu  Ser Ala Pro Ala Gln  Leu
            1225                 1230                 1235
```

```
gtc aaa gag ctg  aag gag aag atg cag  ggt tct ggc atg cct  tgg       3857
Val Lys Glu Leu  Lys Glu Lys Met Gln  Gly Ser Gly Met Pro  Trp
            1240                 1245                 1250

cct ggt gat gaa  ggt cca aag cgg tgg  gaa caa gca tgg atg  gcc       3902
Pro Gly Asp Glu  Gly Pro Lys Arg Trp  Glu Gln Ala Trp Met  Ala
            1255                 1260                 1265

ata aaa aag gtg  tgg gct tca aaa tgg  aat gag aga gca tac  ttc       3947
Ile Lys Lys Val  Trp Ala Ser Lys Trp  Asn Glu Arg Ala Tyr  Phe
            1270                 1275                 1280

agc aca agg aag  gtg aaa ctg gat cat  gac tat ctg tgc atg  gct       3992
Ser Thr Arg Lys  Val Lys Leu Asp His  Asp Tyr Leu Cys Met  Ala
            1285                 1290                 1295

gtc ctt gtt caa  gaa ata ata aat gct  gat tat gca ttt gtc  att       4037
Val Leu Val Gln  Glu Ile Ile Asn Ala  Asp Tyr Ala Phe Val  Ile
            1300                 1305                 1310

cac aca acc aac  cca tct tcc gga gac  gac tca gaa ata tat  gcc       4082
His Thr Thr Asn  Pro Ser Ser Gly Asp  Asp Ser Glu Ile Tyr  Ala
            1315                 1320                 1325

gag gtg gtc agg  ggc ctt ggg gaa aca  ctt gtt gga gct tat  cca       4127
Glu Val Val Arg  Gly Leu Gly Glu Thr  Leu Val Gly Ala Tyr  Pro
            1330                 1335                 1340

gga cgt gct ttg  agt ttt atc tgc aag  aaa aag gat ctc aac  tct       4172
Gly Arg Ala Leu  Ser Phe Ile Cys Lys  Lys Lys Asp Leu Asn  Ser
            1345                 1350                 1355

cct caa gtg tta  ggt tac cca agc aaa  ccg atc ggc ctt ttc  ata       4217
Pro Gln Val Leu  Gly Tyr Pro Ser Lys  Pro Ile Gly Leu Phe  Ile
            1360                 1365                 1370

aaa aga tct atc  atc ttc cga tct gat  tcc aat ggg gaa gat  ttg       4262
Lys Arg Ser Ile  Ile Phe Arg Ser Asp  Ser Asn Gly Glu Asp  Leu
            1375                 1380                 1385

gaa ggt tat gcc  ggt gct ggc ctc tac  gac agt gta cca atg  gat       4307
Glu Gly Tyr Ala  Gly Ala Gly Leu Tyr  Asp Ser Val Pro Met  Asp
            1390                 1395                 1400

gag gag gaa aaa  gtt gta att gat tac  tct tcc gac cca ttg  ata       4352
Glu Glu Glu Lys  Val Val Ile Asp Tyr  Ser Ser Asp Pro Leu  Ile
            1405                 1410                 1415

act gat ggt aac  ttc cgc cag aca atc  ctg tcc aac att gct  cgt       4397
```

```
Thr Asp Gly Asn  Phe Arg Gln Thr Ile  Leu Ser Asn Ile Ala  Arg
            1420                 1425                 1430

gct gga cat gct  atc gag gag cta tat  ggc tct cct caa gac  att      4442
Ala Gly His Ala  Ile Glu Glu Leu Tyr  Gly Ser Pro Gln Asp  Ile
            1435                 1440                 1445

gag ggt gta gtg  agg gat gga aag att  tat gtc gtt cag aca  aga      4487
Glu Gly Val Val  Arg Asp Gly Lys Ile  Tyr Val Val Gln Thr  Arg
            1450                 1455                 1460

cca cag atg tga ttatattctc gttgtatgtt gttcagagaa gaccacagat         4539
Pro Gln Met


gtgatcatat tctcattgta tcagatctgt gaccacttac ctgatacctc ccatgaagtt   4599

acctgtatga ttatacgtga tccaaagcca tcacatcatg ttcaccttca gctattggag   4659

gagaagtgag aagtaggaat tgcaatatga ggaataataa gaaaaacttt gtaaaagcta   4719

aattagctgg gtatgatata gggagaaatg tgtaaacatt gtactatata tagtatatac   4779

acacgcatta tgtattgcat tatgcactga ataatatcgc agcatcaaag aagaaatcct   4839

ttgggtggtt tc                                                       4851


<210>  2
<211>  1464
<212>  PRT
<213>  Solanum tuberosum

<400>  2

Met Ser Asn Ser Leu Gly Asn Asn Leu Leu Tyr Gln Gly Phe Leu Thr
1               5                   10                  15


Ser Thr Val Leu Glu His Lys Ser Arg Ile Ser Pro Pro Cys Val Gly
            20                  25                  30


Gly Asn Ser Leu Phe Gln Gln Gln Val Ile Ser Lys Ser Pro Leu Ser
        35                  40                  45


Thr Glu Phe Arg Gly Asn Arg Leu Lys Val Gln Lys Lys Ile Pro
    50                  55                  60
```

```
Met Glu Lys Lys Arg Ala Phe Ser Ser Ser Pro His Ala Val Leu Thr
65                  70                  75                  80

Thr Asp Thr Ser Ser Glu Leu Ala Glu Lys Phe Ser Leu Gly Gly Asn
                85                  90                  95

Ile Glu Leu Gln Val Asp Val Arg Pro Pro Thr Ser Gly Asp Val Ser
            100                 105                 110

Phe Val Asp Phe Gln Val Thr Asn Gly Ser Asp Lys Leu Phe Leu His
        115                 120                 125

Trp Gly Ala Val Lys Phe Gly Lys Glu Thr Trp Ser Leu Pro Asn Asp
    130                 135                 140

Arg Pro Asp Gly Thr Lys Val Tyr Lys Asn Lys Ala Leu Arg Thr Pro
145                 150                 155                 160

Phe Val Lys Ser Gly Ser Asn Ser Ile Leu Arg Leu Glu Ile Arg Asp
                165                 170                 175

Thr Ala Ile Glu Ala Ile Glu Phe Leu Ile Tyr Asp Glu Ala His Asp
            180                 185                 190

Lys Trp Ile Lys Asn Asn Gly Gly Asn Phe Arg Val Lys Leu Ser Arg
        195                 200                 205

Lys Glu Ile Arg Gly Pro Asp Val Ser Val Pro Glu Glu Leu Val Gln
    210                 215                 220

Ile Gln Ser Tyr Leu Arg Trp Glu Arg Lys Gly Lys Gln Asn Tyr Pro
225                 230                 235                 240

Pro Glu Lys Glu Lys Glu Glu Tyr Glu Ala Ala Arg Thr Val Leu Gln
                245                 250                 255

Glu Glu Ile Ala Arg Gly Ala Ser Ile Gln Asp Ile Arg Ala Arg Leu
```

260 265 270

Thr Lys Thr Asn Asp Lys Ser Gln Ser Lys Glu Glu Pro Leu His Val
275 280 285

Thr Lys Ser Asp Ile Pro Asp Asp Leu Ala Gln Ala Gln Ala Tyr Ile
290 295 300

Arg Trp Glu Lys Ala Gly Lys Pro Asn Tyr Pro Pro Glu Lys Gln Ile
305 310 315 320

Glu Glu Leu Glu Glu Ala Arg Arg Glu Leu Gln Leu Glu Leu Glu Lys
325 330 335

Gly Ile Thr Leu Asp Glu Leu Arg Lys Thr Ile Thr Lys Gly Glu Ile
340 345 350

Lys Thr Lys Val Glu Lys His Leu Lys Arg Ser Ser Phe Ala Val Glu
355 360 365

Arg Ile Gln Arg Lys Lys Arg Asp Phe Gly His Leu Ile Asn Lys Tyr
370 375 380

Thr Ser Ser Pro Ala Val Gln Val Gln Lys Val Leu Glu Glu Pro Pro
385 390 395 400

Ala Leu Ser Lys Ile Lys Leu Tyr Ala Lys Glu Lys Glu Glu Gln Ile
405 410 415

Asp Asp Pro Ile Leu Asn Lys Lys Ile Phe Lys Val Asp Asp Gly Glu
420 425 430

Leu Leu Val Leu Val Ala Lys Ser Ser Gly Lys Thr Lys Val His Leu
435 440 445

Ala Thr Asp Leu Asn Gln Pro Ile Thr Leu His Trp Ala Leu Ser Lys
450 455 460

```
Ser Pro Gly Glu Trp Met Val Pro Pro Ser Ser Ile Leu Pro Pro Gly
465                 470                 475                 480

Ser Ile Ile Leu Asp Lys Ala Ala Glu Thr Pro Phe Ser Ala Ser Ser
                485                 490                 495

Ser Asp Gly Leu Thr Ser Lys Val Gln Ser Leu Asp Ile Val Ile Glu
            500                 505                 510

Asp Gly Asn Phe Val Gly Met Pro Phe Val Leu Leu Ser Gly Glu Lys
        515                 520                 525

Trp Ile Lys Asn Gln Gly Ser Asp Phe Tyr Val Gly Phe Ser Ala Ala
    530                 535                 540

Ser Lys Leu Ala Leu Lys Ala Ala Gly Asp Gly Ser Gly Thr Ala Lys
545                 550                 555                 560

Ser Leu Leu Asp Lys Ile Ala Asp Met Glu Ser Glu Ala Gln Lys Ser
                565                 570                 575

Phe Met His Arg Phe Asn Ile Ala Ala Asp Leu Ile Glu Asp Ala Thr
            580                 585                 590

Ser Ala Gly Glu Leu Gly Phe Ala Gly Ile Leu Val Trp Met Arg Phe
        595                 600                 605

Met Ala Thr Arg Gln Leu Ile Trp Asn Lys Asn Tyr Asn Val Lys Pro
    610                 615                 620

Arg Glu Ile Ser Lys Ala Gln Asp Arg Leu Thr Asp Leu Leu Gln Asn
625                 630                 635                 640

Ala Phe Thr Ser His Pro Gln Tyr Arg Glu Ile Leu Arg Met Ile Met
                645                 650                 655

Ser Thr Val Gly Arg Gly Gly Glu Gly Asp Val Gly Gln Arg Ile Arg
```

```
            660                 665                 670

Asp Glu Ile Leu Val Ile Gln Arg Asn Asn Asp Cys Lys Gly Gly Met
        675                 680                 685

Met Gln Glu Trp His Gln Lys Leu His Asn Asn Thr Ser Pro Asp Asp
    690                 695                 700

Val Val Ile Cys Gln Ala Leu Ile Asp Tyr Ile Lys Ser Asp Phe Asp
705                 710                 715                 720

Leu Gly Val Tyr Trp Lys Thr Leu Asn Glu Asn Gly Ile Thr Lys Glu
                725                 730                 735

Arg Leu Leu Ser Tyr Asp Arg Ala Ile His Ser Glu Pro Asn Phe Arg
            740                 745                 750

Gly Asp Gln Lys Gly Gly Leu Leu Arg Asp Leu Gly His Tyr Met Arg
        755                 760                 765

Thr Leu Lys Ala Val His Ser Gly Ala Asp Leu Glu Ser Ala Ile Ala
    770                 775                 780

Asn Cys Met Gly Tyr Lys Thr Glu Gly Glu Gly Phe Met Val Gly Val
785                 790                 795                 800

Gln Ile Asn Pro Val Ser Gly Leu Pro Ser Gly Phe Gln Asp Leu Leu
                805                 810                 815

His Phe Val Leu Asp His Val Glu Asp Lys Asn Val Glu Thr Leu Leu
            820                 825                 830

Glu Arg Leu Leu Glu Ala Arg Glu Glu Leu Arg Pro Leu Leu Leu Lys
        835                 840                 845

Pro Asn Asn Arg Leu Lys Asp Leu Leu Phe Leu Asp Ile Ala Leu Asp
    850                 855                 860
```

```
Ser Thr Val Arg Thr Ala Val Glu Arg Gly Tyr Glu Glu Leu Asn Asn
865                 870                 875                 880

Ala Asn Pro Glu Lys Ile Met Tyr Phe Ile Ser Leu Val Leu Glu Asn
                885                 890                 895

Leu Ala Leu Ser Val Asp Asp Asn Glu Asp Leu Val Tyr Cys Leu Lys
            900                 905                 910

Gly Trp Asn Gln Ala Leu Ser Met Ser Asn Gly Gly Asp Asn His Trp
        915                 920                 925

Ala Leu Phe Ala Lys Ala Val Leu Asp Arg Thr Arg Leu Ala Leu Ala
    930                 935                 940

Ser Lys Ala Glu Trp Tyr His His Leu Leu Gln Pro Ser Ala Glu Tyr
945                 950                 955                 960

Leu Gly Ser Ile Leu Gly Val Asp Gln Trp Ala Leu Asn Ile Phe Thr
                965                 970                 975

Glu Glu Ile Ile Arg Ala Gly Ser Ala Ala Ser Leu Ser Ser Leu Leu
            980                 985                 990

Asn Arg Leu Asp Pro Val Leu Arg  Lys Thr Ala Asn Leu  Gly Ser Trp
        995                 1000                1005

Gln Ile  Ile Ser Pro Val Glu  Ala Val Gly Tyr Val  Val Val Val
    1010                1015                1020

Asp Glu  Leu Leu Ser Val Gln  Asn Glu Ile Tyr Glu  Lys Pro Thr
    1025                1030                1035

Ile Leu  Val Ala Lys Ser Val  Lys Gly Glu Glu Glu  Ile Pro Asp
    1040                1045                1050

Gly Ala  Val Ala Leu Ile Thr  Pro Asp Met Pro Asp  Val Leu Ser
```

1055 1060 1065

His Val Ser Val Arg Ala Arg Asn Gly Lys Val Cys Phe Ala Thr
1070 1075 1080

Cys Phe Asp Pro Asn Ile Leu Ala Asp Leu Gln Ala Lys Glu Gly
1085 1090 1095

Arg Ile Leu Leu Leu Lys Pro Thr Pro Ser Asp Ile Ile Tyr Ser
1100 1105 1110

Glu Val Asn Glu Ile Glu Leu Gln Ser Ser Ser Asn Leu Val Glu
1115 1120 1125

Ala Glu Thr Ser Ala Thr Leu Arg Leu Val Lys Lys Gln Phe Gly
1130 1135 1140

Gly Cys Tyr Ala Ile Ser Ala Asp Glu Phe Thr Ser Glu Met Val
1145 1150 1155

Gly Ala Lys Ser Arg Asn Ile Ala Tyr Leu Lys Gly Lys Val Pro
1160 1165 1170

Ser Ser Val Gly Ile Pro Thr Ser Val Ala Leu Pro Phe Gly Val
1175 1180 1185

Phe Glu Lys Val Leu Ser Asp Asp Ile Asn Gln Gly Val Ala Lys
1190 1195 1200

Glu Leu Gln Ile Leu Met Lys Lys Leu Ser Glu Gly Asp Phe Ser
1205 1210 1215

Ala Leu Gly Glu Ile Arg Thr Thr Val Leu Asp Leu Ser Ala Pro
1220 1225 1230

Ala Gln Leu Val Lys Glu Leu Lys Glu Lys Met Gln Gly Ser Gly
1235 1240 1245

```
Met Pro  Trp Pro Gly Asp Glu  Gly Pro Lys Arg Trp  Glu Gln Ala
    1250                 1255                 1260

Trp Met  Ala Ile Lys Lys Val  Trp Ala Ser Lys Trp  Asn Glu Arg
    1265                 1270                 1275

Ala Tyr  Phe Ser Thr Arg Lys  Val Lys Leu Asp His  Asp Tyr Leu
    1280                 1285                 1290

Cys Met  Ala Val Leu Val Gln  Glu Ile Ile Asn Ala  Asp Tyr Ala
    1295                 1300                 1305

Phe Val  Ile His Thr Thr Asn  Pro Ser Ser Gly Asp  Asp Ser Glu
    1310                 1315                 1320

Ile Tyr  Ala Glu Val Val Arg  Gly Leu Gly Glu Thr  Leu Val Gly
    1325                 1330                 1335

Ala Tyr  Pro Gly Arg Ala Leu  Ser Phe Ile Cys Lys  Lys Lys Asp
    1340                 1345                 1350

Leu Asn  Ser Pro Gln Val Leu  Gly Tyr Pro Ser Lys  Pro Ile Gly
    1355                 1360                 1365

Leu Phe  Ile Lys Arg Ser Ile  Ile Phe Arg Ser Asp  Ser Asn Gly
    1370                 1375                 1380

Glu Asp  Leu Glu Gly Tyr Ala  Gly Ala Gly Leu Tyr  Asp Ser Val
    1385                 1390                 1395

Pro Met  Asp Glu Glu Glu Lys  Val Val Ile Asp Tyr  Ser Ser Asp
    1400                 1405                 1410

Pro Leu  Ile Thr Asp Gly Asn  Phe Arg Gln Thr Ile  Leu Ser Asn
    1415                 1420                 1425

Ile Ala  Arg Ala Gly His Ala  Ile Glu Glu Leu Tyr  Gly Ser Pro
```

```
    1430                1435                1440

Gln Asp  Ile Glu Gly Val Val  Arg Asp Gly Lys Ile  Tyr Val Val
    1445                1450                1455

Gln Thr  Arg Pro Gln Met
    1460


<210>  3
<211>  4443
<212>  DNA
<213>  Curcuma longa


<220>
<221>  CDS
<222>  (1)..(4443)

<400>  3
atg aac aat tgt gtt gga cat acc tta cct cag caa gct ctg ttt cgg      48
Met Asn Asn Cys Val Gly His Thr Leu Pro Gln Gln Ala Leu Phe Arg
1               5                   10                  15

cct tct gtt gta gaa cgc cat aat aca gct tgc caa cgt tct tct gga      96
Pro Ser Val Val Glu Arg His Asn Thr Ala Cys Gln Arg Ser Ser Gly
            20                  25                  30

aac att ttg tgc act gtt cca tca gca tca aag gca gaa gat gtg cca     144
Asn Ile Leu Cys Thr Val Pro Ser Ala Ser Lys Ala Glu Asp Val Pro
        35                  40                  45

tct ctt aaa cct ttc ctt tca agt aga ttc ctg ggg aag act ccc tat     192
Ser Leu Lys Pro Phe Leu Ser Ser Arg Phe Leu Gly Lys Thr Pro Tyr
    50                  55                  60

gca gga aaa gga aac cca tta aag aaa aat tta aga aca gtt acc atg     240
Ala Gly Lys Gly Asn Pro Leu Lys Lys Asn Leu Arg Thr Val Thr Met
65                  70                  75                  80

agc cct caa gct tta ttg gca gca gat cct gct tca gag ctt gct aga     288
Ser Pro Gln Ala Leu Leu Ala Ala Asp Pro Ala Ser Glu Leu Ala Arg
                85                  90                  95

aaa ttc aag ctg gac acc aat tcc gaa ttg gag gtt act att tgt aag     336
Lys Phe Lys Leu Asp Thr Asn Ser Glu Leu Glu Val Thr Ile Cys Lys
            100                 105                 110
```

```
ccc aca tct gag tct cct atg caa att gat ttt caa gta acc aat gtc      384
Pro Thr Ser Glu Ser Pro Met Gln Ile Asp Phe Gln Val Thr Asn Val
        115                 120                 125

agt ggt tcc ttg gtg ctt cat tgg ggt gta att ctc caa aca aga aga      432
Ser Gly Ser Leu Val Leu His Trp Gly Val Ile Leu Gln Thr Arg Arg
    130                 135                 140

gaa tgg tct ctt cct tct cat tat cct gaa gga aca aaa gta tac aaa      480
Glu Trp Ser Leu Pro Ser His Tyr Pro Glu Gly Thr Lys Val Tyr Lys
145                 150                 155                 160

aat caa gct ctc aga act cct ttt act aaa gtt ggc tcg act tgt tca      528
Asn Gln Ala Leu Arg Thr Pro Phe Thr Lys Val Gly Ser Thr Cys Ser
                165                 170                 175

ctg aga tta gag att gat gat cct gaa ata gaa ata gtt gag ttt ctt      576
Leu Arg Leu Glu Ile Asp Asp Pro Glu Ile Glu Ile Val Glu Phe Leu
            180                 185                 190

ata ctg gat gag gca gaa aac aaa tgg tac aaa cat aat ggc cag aat      624
Ile Leu Asp Glu Ala Glu Asn Lys Trp Tyr Lys His Asn Gly Gln Asn
        195                 200                 205

ttt caa gtt cat ttg ttg aaa caa ggc tat caa aat caa cat gtt tca      672
Phe Gln Val His Leu Leu Lys Gln Gly Tyr Gln Asn Gln His Val Ser
    210                 215                 220

gtc tct gga aat cca aat atc att gta cct gaa gac ctt gtg cag att      720
Val Ser Gly Asn Pro Asn Ile Ile Val Pro Glu Asp Leu Val Gln Ile
225                 230                 235                 240

caa gcc ttt ctt agg tgg gaa aga aag ggt agg cag aca tat aca cct      768
Gln Ala Phe Leu Arg Trp Glu Arg Lys Gly Arg Gln Thr Tyr Thr Pro
                245                 250                 255

gat caa gaa aag gag gag tat gaa gca gct aga atg gag ctg ata gaa      816
Asp Gln Glu Lys Glu Glu Tyr Glu Ala Ala Arg Met Glu Leu Ile Glu
            260                 265                 270

gaa ata agt aga ggt atg cct gta gag gag ctt cga tcc aag ttg aca      864
Glu Ile Ser Arg Gly Met Pro Val Glu Glu Leu Arg Ser Lys Leu Thr
        275                 280                 285

gag aaa cca gaa gtc aaa tct gga agt aga gaa gag aaa acc cac aga      912
Glu Lys Pro Glu Val Lys Ser Gly Ser Arg Glu Glu Lys Thr His Arg
    290                 295                 300

gta caa agt cac aaa ggt ggg atc tca gat gat ctt gtg caa ata caa      960
Val Gln Ser His Lys Gly Gly Ile Ser Asp Asp Leu Val Gln Ile Gln
```

```
305                 310                 315                 320

gca ttc atc cga tgg gag aaa gct ggg aaa cca aac tac cct cca gag      1008
Ala Phe Ile Arg Trp Glu Lys Ala Gly Lys Pro Asn Tyr Pro Pro Glu
                325                 330                 335

aag caa ctt atg gag ttt gag gaa gca agg aaa gag ctg cag ctt gag      1056
Lys Gln Leu Met Glu Phe Glu Glu Ala Arg Lys Glu Leu Gln Leu Glu
            340                 345                 350

ttt gat aaa ggt act tct ctg gct gaa cta cgg gaa aag atc atg aag      1104
Phe Asp Lys Gly Thr Ser Leu Ala Glu Leu Arg Glu Lys Ile Met Lys
        355                 360                 365

ggg gat ata tca act aaa gtt ttg aag caa ctg aag gtt gaa aag tat      1152
Gly Asp Ile Ser Thr Lys Val Leu Lys Gln Leu Lys Val Glu Lys Tyr
    370                 375                 380

ttc agc aac aaa aga att cag cgg aag gaa agg gac atc atg gaa att      1200
Phe Ser Asn Lys Arg Ile Gln Arg Lys Glu Arg Asp Ile Met Glu Ile
385                 390                 395                 400

ttg aat aaa aaa gtt gca gaa act cta gat gaa aaa tct tct caa ata      1248
Leu Asn Lys Lys Val Ala Glu Thr Leu Asp Glu Lys Ser Ser Gln Ile
                405                 410                 415

gtc act cct cct aca gtg cta gaa ctc ttg gct aag tct ata cat gag      1296
Val Thr Pro Pro Thr Val Leu Glu Leu Leu Ala Lys Ser Ile His Glu
            420                 425                 430

cag gat ggt gaa tca gtt ctg cat cag aaa atc tat aag ctg gat aat      1344
Gln Asp Gly Glu Ser Val Leu His Gln Lys Ile Tyr Lys Leu Asp Asn
        435                 440                 445

aag aat ctt ctg gta cta gta acc aaa cct ttt gaa agg aca aaa gtt      1392
Lys Asn Leu Leu Val Leu Val Thr Lys Pro Phe Glu Arg Thr Lys Val
    450                 455                 460

tat ttg gct aca gat caa agt gaa cca ctt att tta cac tgg gga tta      1440
Tyr Leu Ala Thr Asp Gln Ser Glu Pro Leu Ile Leu His Trp Gly Leu
465                 470                 475                 480

tca agg aaa tca aga gag tgg atg gta ccc cct aca agt tct att cct      1488
Ser Arg Lys Ser Arg Glu Trp Met Val Pro Pro Thr Ser Ser Ile Pro
                485                 490                 495

cca ggt tca gta ttg cta gaa gag tct tgt gaa acc cct ttt act aag      1536
Pro Gly Ser Val Leu Leu Glu Glu Ser Cys Glu Thr Pro Phe Thr Lys
            500                 505                 510
```

```
ggt tta atg gta gat cag tat tat cag gcc att caa ata gag att gat      1584
Gly Leu Met Val Asp Gln Tyr Tyr Gln Ala Ile Gln Ile Glu Ile Asp
        515                 520                 525

ggg ggt gat tat gct gga att ccc ttc gtt ctt cgt tca gac gat aaa      1632
Gly Gly Asp Tyr Ala Gly Ile Pro Phe Val Leu Arg Ser Asp Asp Lys
    530                 535                 540

tgg ata aag aat agt ggt ttg gac ttt tac att gag ttg gac gat aga      1680
Trp Ile Lys Asn Ser Gly Leu Asp Phe Tyr Ile Glu Leu Asp Asp Arg
545                 550                 555                 560

agt att agg aag gct cct ggt gat gga agc ggc att gca aaa tca ttg      1728
Ser Ile Arg Lys Ala Pro Gly Asp Gly Ser Gly Ile Ala Lys Ser Leu
                565                 570                 575

ctt gac aag att gct gac ctg gag acc gag gct caa aaa tct ttt atg      1776
Leu Asp Lys Ile Ala Asp Leu Glu Thr Glu Ala Gln Lys Ser Phe Met
            580                 585                 590

cac agg ttt agt att gca gca gat ctc act gag caa gct aga ggc tct      1824
His Arg Phe Ser Ile Ala Ala Asp Leu Thr Glu Gln Ala Arg Gly Ser
        595                 600                 605

ggc cat cta ggg ctt gtt ggc att ctt gtt tgg atg aga ttc atg gca      1872
Gly His Leu Gly Leu Val Gly Ile Leu Val Trp Met Arg Phe Met Ala
    610                 615                 620

atg aga caa ctc att tgg aat aaa aac tac aat gtc aag cca cgt gag      1920
Met Arg Gln Leu Ile Trp Asn Lys Asn Tyr Asn Val Lys Pro Arg Glu
625                 630                 635                 640

att agt aaa gct cag gat agg ctc aca gat ctt ctt cag gac ata tat      1968
Ile Ser Lys Ala Gln Asp Arg Leu Thr Asp Leu Leu Gln Asp Ile Tyr
                645                 650                 655

aaa gac ttc ccc cag tat aga gag atc ttg agg atg atc atg gct act      2016
Lys Asp Phe Pro Gln Tyr Arg Glu Ile Leu Arg Met Ile Met Ala Thr
            660                 665                 670

gtt ggt agg ggc ggt gaa ggt gat gtt ggt cag cgt atc cga gat gaa      2064
Val Gly Arg Gly Gly Glu Gly Asp Val Gly Gln Arg Ile Arg Asp Glu
        675                 680                 685

ata tta gtt ata cag aga aac aat gac tgc aag gga gga atg atg gag      2112
Ile Leu Val Ile Gln Arg Asn Asn Asp Cys Lys Gly Gly Met Met Glu
    690                 695                 700

gaa tgg cat cag aag cta cat aac aac act agc cca gat gat gtt gtg      2160
Glu Trp His Gln Lys Leu His Asn Asn Thr Ser Pro Asp Asp Val Val
```

```
705                 710                 715                 720

ata tgc cag gca ctt att gat tat gtt aaa agt gat ttt gac atc agt      2208
Ile Cys Gln Ala Leu Ile Asp Tyr Val Lys Ser Asp Phe Asp Ile Ser
                725                 730                 735

gtg tac tgg gac agt ttg aat aaa aat gga ata acc aag gaa cgt ttg      2256
Val Tyr Trp Asp Ser Leu Asn Lys Asn Gly Ile Thr Lys Glu Arg Leu
            740                 745                 750

ttg agc tat gat cgt gct att cat tct gaa cca agt ttc agg aga gat      2304
Leu Ser Tyr Asp Arg Ala Ile His Ser Glu Pro Ser Phe Arg Arg Asp
        755                 760                 765

cag aaa gaa ggt ctt tta cgt gat cta gga aac tac atg agg acg ttg      2352
Gln Lys Glu Gly Leu Leu Arg Asp Leu Gly Asn Tyr Met Arg Thr Leu
    770                 775                 780

aag gca gtt cac tct ggt gca gat ctc gag tct gcc att gct acg tgt      2400
Lys Ala Val His Ser Gly Ala Asp Leu Glu Ser Ala Ile Ala Thr Cys
785                 790                 795                 800

atg ggt tac aaa tct gag cgt caa ggc ttt atg gtt ggc gtt caa ata      2448
Met Gly Tyr Lys Ser Glu Arg Gln Gly Phe Met Val Gly Val Gln Ile
                805                 810                 815

aac ccg ata ggg gga ttg cca tct gga ttc cct ggt cta atg aaa ttc      2496
Asn Pro Ile Gly Gly Leu Pro Ser Gly Phe Pro Gly Leu Met Lys Phe
            820                 825                 830

att cta aaa cat gtt gaa gat aaa aat gtg gag cct ttg ata gag ggg      2544
Ile Leu Lys His Val Glu Asp Lys Asn Val Glu Pro Leu Ile Glu Gly
        835                 840                 845

ttg ctg gag gca cga gtg gaa ctt aga cca ttg ctt ctt agc tct cat      2592
Leu Leu Glu Ala Arg Val Glu Leu Arg Pro Leu Leu Leu Ser Ser His
    850                 855                 860

gaa cgg ctg aag gat ctt att ttt ttg gat atc gcc ctt gat tct act      2640
Glu Arg Leu Lys Asp Leu Ile Phe Leu Asp Ile Ala Leu Asp Ser Thr
865                 870                 875                 880

gtc agg aca gct gtt gag aga gga tat gag gaa ttg agt aat gcg gag      2688
Val Arg Thr Ala Val Glu Arg Gly Tyr Glu Glu Leu Ser Asn Ala Glu
                885                 890                 895

cca gag aaa ctt att tac ctt att atg ctg ctg ctt gag aat ctt gca      2736
Pro Glu Lys Leu Ile Tyr Leu Ile Met Leu Leu Leu Glu Asn Leu Ala
            900                 905                 910
```

```
ttg tct aca gat gat aat gag gac ctc ata tat tgc ttg aag gga tgg     2784
Leu Ser Thr Asp Asp Asn Glu Asp Leu Ile Tyr Cys Leu Lys Gly Trp
        915                 920                 925

aaa cat tcg atg gag atg tgt aag caa aaa gat gat caa tgg gca cta     2832
Lys His Ser Met Glu Met Cys Lys Gln Lys Asp Asp Gln Trp Ala Leu
    930                 935                 940

ttt gct aag tca ttt ctt gac aga acc cgt ctg gct cta tca agc aag     2880
Phe Ala Lys Ser Phe Leu Asp Arg Thr Arg Leu Ala Leu Ser Ser Lys
945                 950                 955                 960

gca gaa tac tac cat caa att ttg caa cct tca gct gaa tac ctt gga     2928
Ala Glu Tyr Tyr His Gln Ile Leu Gln Pro Ser Ala Glu Tyr Leu Gly
                965                 970                 975

tca ttg ctt gat gtt gat gca ggg gcg gta agc ata ttc aca gaa gaa     2976
Ser Leu Leu Asp Val Asp Ala Gly Ala Val Ser Ile Phe Thr Glu Glu
            980                 985                 990

atc ata cgt gct gga tca gca gct  tct tta tct gca ctt  ctt cag cga   3024
Ile Ile Arg Ala Gly Ser Ala Ala  Ser Leu Ser Ala Leu  Leu Gln Arg
        995                 1000                 1005

ctt gac  cct ctt ctt cgg aaa  gtt gca cat ttg gga  agc tgg cag      3069
Leu Asp  Pro Leu Leu Arg Lys  Val Ala His Leu Gly  Ser Trp Gln
    1010                 1015                 1020

gtc ata  agc cct gtt gaa gtt  gct gga tat gtt gaa  att gta gaa      3114
Val Ile  Ser Pro Val Glu Val  Ala Gly Tyr Val Glu  Ile Val Glu
    1025                 1030                 1035

gaa ttg  ctt gct gtc cag aat  aaa tca tat aca caa  tca aca att      3159
Glu Leu  Leu Ala Val Gln Asn  Lys Ser Tyr Thr Gln  Ser Thr Ile
    1040                 1045                 1050

ttg gtt  gca aaa cat gta agg  gga gaa gag gaa ata  cca gat ggc      3204
Leu Val  Ala Lys His Val Arg  Gly Glu Glu Glu Ile  Pro Asp Gly
    1055                 1060                 1065

aca gtt  gct gtt tta aca cct  gat atg cca gat gtt  cta tct cat      3249
Thr Val  Ala Val Leu Thr Pro  Asp Met Pro Asp Val  Leu Ser His
    1070                 1075                 1080

gtc tct  gtg cga gct aga aat  agc aag gta tgt ttt  gct acc tgc      3294
Val Ser  Val Arg Ala Arg Asn  Ser Lys Val Cys Phe  Ala Thr Cys
    1085                 1090                 1095

ttt gat  gac aat atc ctg gat  gag ttt cgg aga aat  gca gga aag      3339
Phe Asp  Asp Asn Ile Leu Asp  Glu Phe Arg Arg Asn  Ala Gly Lys
```

```
    1100                1105                1110

ctt ttt  cat cta aag ccc aca  tca gat gat att gta  tat agt aaa       3384
Leu Phe  His Leu Lys Pro Thr  Ser Asp Asp Ile Val  Tyr Ser Lys
    1115                1120                1125

ata gaa  aaa act gaa cct gaa  gat gtg ggt cca gtt  caa gct gga       3429
Ile Glu  Lys Thr Glu Pro Glu  Asp Val Gly Pro Val  Gln Ala Gly
    1130                1135                1140

gat gag  caa tca ctg cca tct  gtg aca ttg gtt agg  aag cac ttc       3474
Asp Glu  Gln Ser Leu Pro Ser  Val Thr Leu Val Arg  Lys His Phe
    1145                1150                1155

agc ggc  aag tac acc ata tca  gct gaa gaa ttt acc  aat gaa atg       3519
Ser Gly  Lys Tyr Thr Ile Ser  Ala Glu Glu Phe Thr  Asn Glu Met
    1160                1165                1170

gtt ggt  gct aaa tca cgg aat  atc tca ttt cta aaa  gga aag gtt       3564
Val Gly  Ala Lys Ser Arg Asn  Ile Ser Phe Leu Lys  Gly Lys Val
    1175                1180                1185

cct tca  tgg gtg ggc att ccc  aca tca gtc gct cta  cca ttt gga       3609
Pro Ser  Trp Val Gly Ile Pro  Thr Ser Val Ala Leu  Pro Phe Gly
    1190                1195                1200

gtt ttt  gaa gaa gtt ctg tca  aat gac ata aac aag  gaa att gcc       3654
Val Phe  Glu Glu Val Leu Ser  Asn Asp Ile Asn Lys  Glu Ile Ala
    1205                1210                1215

agc cag  ctg cag tta ctg aaa  gag aag ttg gct atc  gga gaa ttc       3699
Ser Gln  Leu Gln Leu Leu Lys  Glu Lys Leu Ala Ile  Gly Glu Phe
    1220                1225                1230

aat gca  ctt ctc gac ata aga  aag atg atc ttg cag  cta gca tct       3744
Asn Ala  Leu Leu Asp Ile Arg  Lys Met Ile Leu Gln  Leu Ala Ser
    1235                1240                1245

cca att  gag ttg gta caa gag  cta aag gga aaa atg  cag gca tca       3789
Pro Ile  Glu Leu Val Gln Glu  Leu Lys Gly Lys Met  Gln Ala Ser
    1250                1255                1260

gga atg  cca tgg cct ggt gat  gag ggt gaa gat cgg  tgg gaa ctt       3834
Gly Met  Pro Trp Pro Gly Asp  Glu Gly Glu Asp Arg  Trp Glu Leu
    1265                1270                1275

gct tgg  atg gca ata aaa aga  gtt tgg gct tca aag  tgg aat gag       3879
Ala Trp  Met Ala Ile Lys Arg  Val Trp Ala Ser Lys  Trp Asn Glu
    1280                1285                1290
```

```
aga gca  tat ttc agc aca agg  aaa gtc aag ttg gat  cat gac tat        3924
Arg Ala  Tyr Phe Ser Thr Arg  Lys Val Lys Leu Asp  His Asp Tyr
    1295                 1300                 1305

ttg tgc  atg gct gtc ttg gtt  caa gaa atc att agt  gct gat tat        3969
Leu Cys  Met Ala Val Leu Val  Gln Glu Ile Ile Ser  Ala Asp Tyr
    1310                 1315                 1320

gca ttt  gtc atc cac act aca  aac cca tca tct gga  gac tca tct        4014
Ala Phe  Val Ile His Thr Thr  Asn Pro Ser Ser Gly  Asp Ser Ser
    1325                 1330                 1335

gaa ata  tat gcc gag gtg gtg  aaa gga ctc gga gaa  act ctt gtt        4059
Glu Ile  Tyr Ala Glu Val Val  Lys Gly Leu Gly Glu  Thr Leu Val
    1340                 1345                 1350

gga gcc  tat cca ggc cgg gca  ttg agc ttc gtc tgt  aat aag aac        4104
Gly Ala  Tyr Pro Gly Arg Ala  Leu Ser Phe Val Cys  Asn Lys Asn
    1355                 1360                 1365

aat ctg  aac tcg cca aag gta  ctt ggt ttc cca agc  aag cct att        4149
Asn Leu  Asn Ser Pro Lys Val  Leu Gly Phe Pro Ser  Lys Pro Ile
    1370                 1375                 1380

ggc ctc  ttc atc aaa cga tca  att atc ttc aga tct  gat tct aat        4194
Gly Leu  Phe Ile Lys Arg Ser  Ile Ile Phe Arg Ser  Asp Ser Asn
    1385                 1390                 1395

ggt gaa  gat tta gaa ggt tat  gca ggt gct ggt ctt  tat gac agt        4239
Gly Glu  Asp Leu Glu Gly Tyr  Ala Gly Ala Gly Leu  Tyr Asp Ser
    1400                 1405                 1410

gtg ccc  atg gat gag gaa gag  aaa gtg gta ctc gac  tat gta gct        4284
Val Pro  Met Asp Glu Glu Glu  Lys Val Val Leu Asp  Tyr Val Ala
    1415                 1420                 1425

gac ccg  tta atc atg gat aag  aac ttc cgt aat tca  ctg ctc tcc        4329
Asp Pro  Leu Ile Met Asp Lys  Asn Phe Arg Asn Ser  Leu Leu Ser
    1430                 1435                 1440

agc att  gct cga gca ggt tat  gcg atc gag gag ctc  tat ggc tct        4374
Ser Ile  Ala Arg Ala Gly Tyr  Ala Ile Glu Glu Leu  Tyr Gly Ser
    1445                 1450                 1455

cca cag  gac att gaa ggt gtt  gta aag gat ggt aaa  atc ttc gtc        4419
Pro Gln  Asp Ile Glu Gly Val  Val Lys Asp Gly Lys  Ile Phe Val
    1460                 1465                 1470

gtc caa  aca aga cca cag atg  tga                                    4443
Val Gln  Thr Arg Pro Gln Met
```

1475 1480

<210> 4
<211> 1480
<212> PRT
<213> Curcuma longa

<400> 4

```
Met Asn Asn Cys Val Gly His Thr Leu Pro Gln Gln Ala Leu Phe Arg
1               5                   10                  15

Pro Ser Val Val Glu Arg His Asn Thr Ala Cys Gln Arg Ser Ser Gly
            20                  25                  30

Asn Ile Leu Cys Thr Val Pro Ser Ala Ser Lys Ala Glu Asp Val Pro
        35                  40                  45

Ser Leu Lys Pro Phe Leu Ser Ser Arg Phe Leu Gly Lys Thr Pro Tyr
    50                  55                  60

Ala Gly Lys Gly Asn Pro Leu Lys Lys Asn Leu Arg Thr Val Thr Met
65                  70                  75                  80

Ser Pro Gln Ala Leu Leu Ala Ala Asp Pro Ala Ser Glu Leu Ala Arg
                85                  90                  95

Lys Phe Lys Leu Asp Thr Asn Ser Glu Leu Glu Val Thr Ile Cys Lys
            100                 105                 110

Pro Thr Ser Glu Ser Pro Met Gln Ile Asp Phe Gln Val Thr Asn Val
        115                 120                 125

Ser Gly Ser Leu Val Leu His Trp Gly Val Ile Leu Gln Thr Arg Arg
    130                 135                 140

Glu Trp Ser Leu Pro Ser His Tyr Pro Glu Gly Thr Lys Val Tyr Lys
145                 150                 155                 160
```

```
Asn Gln Ala Leu Arg Thr Pro Phe Thr Lys Val Gly Ser Thr Cys Ser
                165                 170                 175

Leu Arg Leu Glu Ile Asp Asp Pro Glu Ile Glu Ile Val Glu Phe Leu
            180                 185                 190

Ile Leu Asp Glu Ala Glu Asn Lys Trp Tyr Lys His Asn Gly Gln Asn
        195                 200                 205

Phe Gln Val His Leu Leu Lys Gln Gly Tyr Gln Asn Gln His Val Ser
    210                 215                 220

Val Ser Gly Asn Pro Asn Ile Ile Val Pro Glu Asp Leu Val Gln Ile
225                 230                 235                 240

Gln Ala Phe Leu Arg Trp Glu Arg Lys Gly Arg Gln Thr Tyr Thr Pro
                245                 250                 255

Asp Gln Glu Lys Glu Glu Tyr Glu Ala Ala Arg Met Glu Leu Ile Glu
            260                 265                 270

Glu Ile Ser Arg Gly Met Pro Val Glu Glu Leu Arg Ser Lys Leu Thr
        275                 280                 285

Glu Lys Pro Glu Val Lys Ser Gly Ser Arg Glu Glu Lys Thr His Arg
    290                 295                 300

Val Gln Ser His Lys Gly Gly Ile Ser Asp Asp Leu Val Gln Ile Gln
305                 310                 315                 320

Ala Phe Ile Arg Trp Glu Lys Ala Gly Lys Pro Asn Tyr Pro Pro Glu
                325                 330                 335

Lys Gln Leu Met Glu Phe Glu Glu Ala Arg Lys Glu Leu Gln Leu Glu
            340                 345                 350

Phe Asp Lys Gly Thr Ser Leu Ala Glu Leu Arg Glu Lys Ile Met Lys
        355                 360                 365
```

```
Gly Asp Ile Ser Thr Lys Val Leu Lys Gln Leu Lys Val Glu Lys Tyr
    370                 375                 380

Phe Ser Asn Lys Arg Ile Gln Arg Lys Glu Arg Asp Ile Met Glu Ile
385                 390                 395                 400

Leu Asn Lys Lys Val Ala Glu Thr Leu Asp Glu Lys Ser Ser Gln Ile
                405                 410                 415

Val Thr Pro Pro Thr Val Leu Glu Leu Leu Ala Lys Ser Ile His Glu
            420                 425                 430

Gln Asp Gly Glu Ser Val Leu His Gln Lys Ile Tyr Lys Leu Asp Asn
        435                 440                 445

Lys Asn Leu Leu Val Leu Val Thr Lys Pro Phe Glu Arg Thr Lys Val
    450                 455                 460

Tyr Leu Ala Thr Asp Gln Ser Glu Pro Leu Ile Leu His Trp Gly Leu
465                 470                 475                 480

Ser Arg Lys Ser Arg Glu Trp Met Val Pro Pro Thr Ser Ser Ile Pro
                485                 490                 495

Pro Gly Ser Val Leu Leu Glu Glu Ser Cys Glu Thr Pro Phe Thr Lys
            500                 505                 510

Gly Leu Met Val Asp Gln Tyr Tyr Gln Ala Ile Gln Ile Glu Ile Asp
        515                 520                 525

Gly Gly Asp Tyr Ala Gly Ile Pro Phe Val Leu Arg Ser Asp Asp Lys
    530                 535                 540

Trp Ile Lys Asn Ser Gly Leu Asp Phe Tyr Ile Glu Leu Asp Asp Arg
545                 550                 555                 560
```

```
Ser Ile Arg Lys Ala Pro Gly Asp Gly Ser Gly Ile Ala Lys Ser Leu
                565                 570                 575

Leu Asp Lys Ile Ala Asp Leu Glu Thr Glu Ala Gln Lys Ser Phe Met
            580                 585                 590

His Arg Phe Ser Ile Ala Ala Asp Leu Thr Glu Gln Ala Arg Gly Ser
        595                 600                 605

Gly His Leu Gly Leu Val Gly Ile Leu Val Trp Met Arg Phe Met Ala
    610                 615                 620

Met Arg Gln Leu Ile Trp Asn Lys Asn Tyr Asn Val Lys Pro Arg Glu
625                 630                 635                 640

Ile Ser Lys Ala Gln Asp Arg Leu Thr Asp Leu Leu Gln Asp Ile Tyr
                645                 650                 655

Lys Asp Phe Pro Gln Tyr Arg Glu Ile Leu Arg Met Ile Met Ala Thr
            660                 665                 670

Val Gly Arg Gly Gly Glu Gly Asp Val Gly Gln Arg Ile Arg Asp Glu
        675                 680                 685

Ile Leu Val Ile Gln Arg Asn Asn Asp Cys Lys Gly Gly Met Met Glu
    690                 695                 700

Glu Trp His Gln Lys Leu His Asn Asn Thr Ser Pro Asp Asp Val Val
705                 710                 715                 720

Ile Cys Gln Ala Leu Ile Asp Tyr Val Lys Ser Asp Phe Asp Ile Ser
                725                 730                 735

Val Tyr Trp Asp Ser Leu Asn Lys Asn Gly Ile Thr Lys Glu Arg Leu
            740                 745                 750

Leu Ser Tyr Asp Arg Ala Ile His Ser Glu Pro Ser Phe Arg Arg Asp
        755                 760                 765
```

```
Gln Lys Glu Gly Leu Leu Arg Asp Leu Gly Asn Tyr Met Arg Thr Leu
    770                 775                 780

Lys Ala Val His Ser Gly Ala Asp Leu Glu Ser Ala Ile Ala Thr Cys
785                 790                 795                 800

Met Gly Tyr Lys Ser Glu Arg Gln Gly Phe Met Val Gly Val Gln Ile
                805                 810                 815

Asn Pro Ile Gly Gly Leu Pro Ser Gly Phe Pro Gly Leu Met Lys Phe
            820                 825                 830

Ile Leu Lys His Val Glu Asp Lys Asn Val Glu Pro Leu Ile Glu Gly
        835                 840                 845

Leu Leu Glu Ala Arg Val Glu Leu Arg Pro Leu Leu Leu Ser Ser His
    850                 855                 860

Glu Arg Leu Lys Asp Leu Ile Phe Leu Asp Ile Ala Leu Asp Ser Thr
865                 870                 875                 880

Val Arg Thr Ala Val Glu Arg Gly Tyr Glu Glu Leu Ser Asn Ala Glu
                885                 890                 895

Pro Glu Lys Leu Ile Tyr Leu Ile Met Leu Leu Leu Glu Asn Leu Ala
            900                 905                 910

Leu Ser Thr Asp Asp Asn Glu Asp Leu Ile Tyr Cys Leu Lys Gly Trp
        915                 920                 925

Lys His Ser Met Glu Met Cys Lys Gln Lys Asp Asp Gln Trp Ala Leu
    930                 935                 940

Phe Ala Lys Ser Phe Leu Asp Arg Thr Arg Leu Ala Leu Ser Ser Lys
945                 950                 955                 960
```

Ala Glu Tyr Tyr His Gln Ile Leu Gln Pro Ser Ala Glu Tyr Leu Gly
965 970 975

Ser Leu Leu Asp Val Asp Ala Gly Ala Val Ser Ile Phe Thr Glu Glu
980 985 990

Ile Ile Arg Ala Gly Ser Ala Ala Ser Leu Ser Ala Leu Leu Gln Arg
995 1000 1005

Leu Asp Pro Leu Leu Arg Lys Val Ala His Leu Gly Ser Trp Gln
1010 1015 1020

Val Ile Ser Pro Val Glu Val Ala Gly Tyr Val Glu Ile Val Glu
1025 1030 1035

Glu Leu Leu Ala Val Gln Asn Lys Ser Tyr Thr Gln Ser Thr Ile
1040 1045 1050

Leu Val Ala Lys His Val Arg Gly Glu Glu Glu Ile Pro Asp Gly
1055 1060 1065

Thr Val Ala Val Leu Thr Pro Asp Met Pro Asp Val Leu Ser His
1070 1075 1080

Val Ser Val Arg Ala Arg Asn Ser Lys Val Cys Phe Ala Thr Cys
1085 1090 1095

Phe Asp Asp Asn Ile Leu Asp Glu Phe Arg Arg Asn Ala Gly Lys
1100 1105 1110

Leu Phe His Leu Lys Pro Thr Ser Asp Asp Ile Val Tyr Ser Lys
1115 1120 1125

Ile Glu Lys Thr Glu Pro Glu Asp Val Gly Pro Val Gln Ala Gly
1130 1135 1140

Asp Glu Gln Ser Leu Pro Ser Val Thr Leu Val Arg Lys His Phe
1145 1150 1155

Ser Gly Lys Tyr Thr Ile Ser Ala Glu Glu Phe Thr Asn Glu Met
1160 1165 1170

Val Gly Ala Lys Ser Arg Asn Ile Ser Phe Leu Lys Gly Lys Val
1175 1180 1185

Pro Ser Trp Val Gly Ile Pro Thr Ser Val Ala Leu Pro Phe Gly
1190 1195 1200

Val Phe Glu Glu Val Leu Ser Asn Asp Ile Asn Lys Glu Ile Ala
1205 1210 1215

Ser Gln Leu Gln Leu Leu Lys Glu Lys Leu Ala Ile Gly Glu Phe
1220 1225 1230

Asn Ala Leu Leu Asp Ile Arg Lys Met Ile Leu Gln Leu Ala Ser
1235 1240 1245

Pro Ile Glu Leu Val Gln Glu Leu Lys Gly Lys Met Gln Ala Ser
1250 1255 1260

Gly Met Pro Trp Pro Gly Asp Glu Gly Glu Asp Arg Trp Glu Leu
1265 1270 1275

Ala Trp Met Ala Ile Lys Arg Val Trp Ala Ser Lys Trp Asn Glu
1280 1285 1290

Arg Ala Tyr Phe Ser Thr Arg Lys Val Lys Leu Asp His Asp Tyr
1295 1300 1305

Leu Cys Met Ala Val Leu Val Gln Glu Ile Ile Ser Ala Asp Tyr
1310 1315 1320

Ala Phe Val Ile His Thr Thr Asn Pro Ser Ser Gly Asp Ser Ser
1325 1330 1335

Glu Ile Tyr Ala Glu Val Val Lys Gly Leu Gly Glu Thr Leu Val
1340 1345 1350

Gly Ala Tyr Pro Gly Arg Ala Leu Ser Phe Val Cys Asn Lys Asn
1355 1360 1365

Asn Leu Asn Ser Pro Lys Val Leu Gly Phe Pro Ser Lys Pro Ile
1370 1375 1380

Gly Leu Phe Ile Lys Arg Ser Ile Ile Phe Arg Ser Asp Ser Asn
1385 1390 1395

Gly Glu Asp Leu Glu Gly Tyr Ala Gly Ala Gly Leu Tyr Asp Ser
1400 1405 1410

Val Pro Met Asp Glu Glu Glu Lys Val Val Leu Asp Tyr Val Ala
1415 1420 1425

Asp Pro Leu Ile Met Asp Lys Asn Phe Arg Asn Ser Leu Leu Ser
1430 1435 1440

Ser Ile Ala Arg Ala Gly Tyr Ala Ile Glu Glu Leu Tyr Gly Ser
1445 1450 1455

Pro Gln Asp Ile Glu Gly Val Val Lys Asp Gly Lys Ile Phe Val
1460 1465 1470

Val Gln Thr Arg Pro Gln Met
1475 1480

<210> 5
<211> 2400
<212> DNA
<213> Zea mays

<400> 5
atggcgttcc gggtttctgg ggcggtgctc ggtggggccg taagggctcc ccgactcacc 60

ggcggcgggg agggtagtct agtcttccgg cacaccggcc tcttcttaac tcggggtgct 120

cgagttggat gttcggggac gcacggggcc atgcgcgcgg cggccgcggc caggaaagcg 180

gtcatggttc ctgagggcga gaatgatggc ctcgcatcaa gggctgactc ggctcaattc 240

cagtcggatg aactggaggt accagacatt tctgaagaga caacgtgcgg tgctggtgtg 300

gctgatgctc aagccttgaa cagagttcga gtggtccccc caccaagcga tggacaaaaa 360

atattccaga ttgaccccat gttgcaaggc tataagtacc atcttgagta tcggtacagc 420

ctctatagaa gaatccgttc agacattgat gaacatgaag gaggcttgga agccttctcc 480

cgtagttatg agaagtttgg atttaatcgc agcgcggaag gtatcacata tcgagaatgg 540

gctcctggag cattttctgc agcattggtg ggtgacttca acaactggga tccaaatgca 600

gatcgtatga gcaaaaatga gtttggtgtt tgggaaattt ttctgcctaa caatgcagat 660

ggtacatcac ctattcctca tggatctcgt gtaaaggtga gaatggatac tccatcaggg 720

ataaaggatt caattccagc ctggatcaag tactcagtgc aggccccagg agaaatacca 780

tatgatggga tttattatga tcctcctgaa gaggtaaagt atgtgttcag gcatgcgcaa 840

cctaaacgac caaaatcatt gcggatatat gaaacacatg tcggaatgag tagcccggaa 900

ccgaagataa acacatatgt aaactttagg gatgaagtcc tcccaagaat aaaaaaactt 960

ggatacaatg cagtgcaaat aatggcaatc caagagcact catattatgg aagctttgga 1020

taccatgtaa ctaatttttt tgcgccaagt agtcgttttg gtaccccaga agaattgaag 1080

tctttgattg atagagcaca tgagcttggt ttgctagttc tcatggatgt ggttcatagt 1140

catgcgtcaa gtaatactct ggatgggttg aatggttttg atggtacaga tacacattac 1200

tttcacagtg gtccacgtgg ccatcactgg atgtgggatt ctcgcctatt taactatggg 1260

aactgggaag ttttaagatt tcttctctcc aatgctagat ggtggctcga ggaatataag 1320

tttgatggtt tccgttttga tggtgtgacc tccatgatgt acactcatca cggattacaa 1380

gtaacattta cggggaactt caatgagtat tttggctttg ccaccgatgt agatgcagtg 1440

gtttacttga tgctggtaaa tgatctaatt catggacttt atcctgaggc tgtaaccatt 1500

ggtgaagatg ttagtggaat gcctacattt gcccttcctg ttcacgatgg tggggtaggt 1560

tttgactatc ggatgcatat ggctgtggct gacaaatgga ttgaccttct caagcaaagt 1620

gatgaaactt ggaagatggg tgatattgtg cacacactga caaataggag gtggttagag 1680

aagtgtgtaa cttatgctga aagtcatgat caagcattag tcggcgacaa gactattgcg 1740

ttttggttga tggacaagga tatgtatgat ttcatggccc tcgatagacc ttcaactcct 1800

accattgatc gtgggatagc attacataag atgattagac ttatcacaat gggtttagga 1860

ggagagggct atcttaattt catgggaaat gagtttggac atcctgaatg gatagatttt 1920

ccaagaggtc cgcaaagact tccaagtggt aagtttattc cagggaataa caacagttat 1980

gacaaatgtc gtcgaagatt tgacctgggt gatgcagact atcttaggta tcatggtatg 2040

caagagtttg atcaggcaat gcaacatctt gagcaaaaat atgaattcat gacatctgat 2100

caccagtata tttcccggaa acatgaggag gataaggtga ttgtgttcga aaagggagat 2160

ttggtatttg tgttcaactt ccactgcaac aacagctatt ttgactaccg tattggttgt 2220

cgaaagcctg ggtgtataa ggtggtcttg gactccgacg ctggactatt tggtggattt 2280

agcaggatcc atcacgcagc cgagcacttc accgccgact gttcgcatga taataggcca 2340

tattcattct cggtttatac accaagcaga acatgtgtcg tctatgctcc agtggagtga 2400

<210> 6
<211> 799
<212> PRT
<213> Zea mays

<400> 6

Met Ala Phe Arg Val Ser Gly Ala Val Leu Gly Gly Ala Val Arg Ala
1               5                   10                  15

Pro Arg Leu Thr Gly Gly Gly Glu Gly Ser Leu Val Phe Arg His Thr
            20                  25                  30

Gly Leu Phe Leu Thr Arg Gly Ala Arg Val Gly Cys Ser Gly Thr His
        35                  40                  45

Gly Ala Met Arg Ala Ala Ala Ala Ala Arg Lys Ala Val Met Val Pro
    50                  55                  60

Glu Gly Glu Asn Asp Gly Leu Ala Ser Arg Ala Asp Ser Ala Gln Phe
65 70 75 80

Gln Ser Asp Glu Leu Glu Val Pro Asp Ile Ser Glu Glu Thr Thr Cys
85 90 95

Gly Ala Gly Val Ala Asp Ala Gln Ala Leu Asn Arg Val Arg Val Val
100 105 110

Pro Pro Pro Ser Asp Gly Gln Lys Ile Phe Gln Ile Asp Pro Met Leu
115 120 125

Gln Gly Tyr Lys Tyr His Leu Glu Tyr Arg Tyr Ser Leu Tyr Arg Arg
130 135 140

Ile Arg Ser Asp Ile Asp Glu His Glu Gly Gly Leu Glu Ala Phe Ser
145 150 155 160

Arg Ser Tyr Glu Lys Phe Gly Phe Asn Arg Ser Ala Glu Gly Ile Thr
165 170 175

Tyr Arg Glu Trp Ala Pro Gly Ala Phe Ser Ala Ala Leu Val Gly Asp
180 185 190

Phe Asn Asn Trp Asp Pro Asn Ala Asp Arg Met Ser Lys Asn Glu Phe
195 200 205

Gly Val Trp Glu Ile Phe Leu Pro Asn Asn Ala Asp Gly Thr Ser Pro
210 215 220

Ile Pro His Gly Ser Arg Val Lys Val Arg Met Asp Thr Pro Ser Gly
225 230 235 240

Ile Lys Asp Ser Ile Pro Ala Trp Ile Lys Tyr Ser Val Gln Ala Pro
245 250 255

Gly Glu Ile Pro Tyr Asp Gly Ile Tyr Tyr Asp Pro Pro Glu Glu Val
260 265 270

```
Lys Tyr Val Phe Arg His Ala Gln Pro Lys Arg Pro Lys Ser Leu Arg
        275                 280                 285

Ile Tyr Glu Thr His Val Gly Met Ser Ser Pro Glu Pro Lys Ile Asn
    290                 295                 300

Thr Tyr Val Asn Phe Arg Asp Glu Val Leu Pro Arg Ile Lys Lys Leu
305                 310                 315                 320

Gly Tyr Asn Ala Val Gln Ile Met Ala Ile Gln Glu His Ser Tyr Tyr
                325                 330                 335

Gly Ser Phe Gly Tyr His Val Thr Asn Phe Phe Ala Pro Ser Ser Arg
            340                 345                 350

Phe Gly Thr Pro Glu Glu Leu Lys Ser Leu Ile Asp Arg Ala His Glu
        355                 360                 365

Leu Gly Leu Leu Val Leu Met Asp Val Val His Ser His Ala Ser Ser
    370                 375                 380

Asn Thr Leu Asp Gly Leu Asn Gly Phe Asp Gly Thr Asp Thr His Tyr
385                 390                 395                 400

Phe His Ser Gly Pro Arg Gly His His Trp Met Trp Asp Ser Arg Leu
                405                 410                 415

Phe Asn Tyr Gly Asn Trp Glu Val Leu Arg Phe Leu Leu Ser Asn Ala
            420                 425                 430

Arg Trp Trp Leu Glu Glu Tyr Lys Phe Asp Gly Phe Arg Phe Asp Gly
        435                 440                 445

Val Thr Ser Met Met Tyr Thr His His Gly Leu Gln Val Thr Phe Thr
    450                 455                 460
```

```
Gly Asn Phe Asn Glu Tyr Phe Gly Phe Ala Thr Asp Val Asp Ala Val
465                 470                 475                 480

Val Tyr Leu Met Leu Val Asn Asp Leu Ile His Gly Leu Tyr Pro Glu
                485                 490                 495

Ala Val Thr Ile Gly Glu Asp Val Ser Gly Met Pro Thr Phe Ala Leu
            500                 505                 510

Pro Val His Asp Gly Gly Val Gly Phe Asp Tyr Arg Met His Met Ala
        515                 520                 525

Val Ala Asp Lys Trp Ile Asp Leu Leu Lys Gln Ser Asp Glu Thr Trp
    530                 535                 540

Lys Met Gly Asp Ile Val His Thr Leu Thr Asn Arg Arg Trp Leu Glu
545                 550                 555                 560

Lys Cys Val Thr Tyr Ala Glu Ser His Asp Gln Ala Leu Val Gly Asp
                565                 570                 575

Lys Thr Ile Ala Phe Trp Leu Met Asp Lys Asp Met Tyr Asp Phe Met
            580                 585                 590

Ala Leu Asp Arg Pro Ser Thr Pro Thr Ile Asp Arg Gly Ile Ala Leu
        595                 600                 605

His Lys Met Ile Arg Leu Ile Thr Met Gly Leu Gly Gly Glu Gly Tyr
    610                 615                 620

Leu Asn Phe Met Gly Asn Glu Phe Gly His Pro Glu Trp Ile Asp Phe
625                 630                 635                 640

Pro Arg Gly Pro Gln Arg Leu Pro Ser Gly Lys Phe Ile Pro Gly Asn
                645                 650                 655

Asn Asn Ser Tyr Asp Lys Cys Arg Arg Arg Phe Asp Leu Gly Asp Ala
            660                 665                 670
```

```
Asp Tyr Leu Arg Tyr His Gly Met Gln Glu Phe Asp Gln Ala Met Gln
        675                 680                 685

His Leu Glu Gln Lys Tyr Glu Phe Met Thr Ser Asp His Gln Tyr Ile
    690                 695                 700

Ser Arg Lys His Glu Glu Asp Lys Val Ile Val Phe Glu Lys Gly Asp
705                 710                 715                 720

Leu Val Phe Val Phe Asn Phe His Cys Asn Asn Ser Tyr Phe Asp Tyr
                725                 730                 735

Arg Ile Gly Cys Arg Lys Pro Gly Val Tyr Lys Val Val Leu Asp Ser
            740                 745                 750

Asp Ala Gly Leu Phe Gly Gly Phe Ser Arg Ile His His Ala Ala Glu
        755                 760                 765

His Phe Thr Ala Asp Cys Ser His Asp Asn Arg Pro Tyr Ser Phe Ser
    770                 775                 780

Val Tyr Thr Pro Ser Arg Thr Cys Val Val Tyr Ala Pro Val Glu
785                 790                 795

<210>  7
<211>  2310
<212>  DNA
<213>  Triticum aestivum

<400>  7
gggatggcga cgttcgcggt gtccggcgcg accctcggtg tggcgcggcc gccggcggcg      60

gcgcaacctg aagaattaca gatacctgaa gacatcgagg agcaaacggc tgaagtaaac     120

atgacagggg ggactgcaga aaaacttgaa tcttcagaac cgactcaagg cattgtggaa     180

acaatcactg atggtgtaac caaaggagtt aaggaactag tcgtgggggga gaaaccgcga     240

gttgtcccaa aaccaggaga tgggcagaaa atatacgaga ttgacccaac gctgaaagat     300
```

tttcggagcc atcttgacta ccgatacagc gaatacagga gaattcgtgc tgctattgac 360

caacatgaag gtggattgga agcattttct cgtggttatg aaaagcttgg atttacccgc 420

agtgctgaag gtatcactta ccgagaatgg gctcctggag cgcattctgc agcattagta 480

ggtgacttca acaattggaa tccgaatgca gatactatga ccagagatga ttatggtgtt 540

tgggagattt tcctccctaa caatgctgat ggatccccag ctattcctca tggctcacgt 600

gtaaagatac ggatggatac tccatctggt gtgaaggatt caatttctgc ttggatcaag 660

ttctctgtgc aggctccagg tgaaatacca ttcaatggca tatattatga tccacctgaa 720

gaggagaagt atgtcttcca acatcctcaa cctaaacgac cagagtcact gaggatttat 780

gaatcacaca ttggaatgag cagcccagaa ccgaagataa attcatatgc taattttagg 840

gatgaggtgc tgccaagaat taaaaggctt ggatacaatg cagtgcagat aatggcaatc 900

caggagcatt catactatgc gagctttggg taccatgtta ctaatttttt tgcaccaagt 960

agccgttttg gaactccaga ggacttaaaa tccctgatcg atagagcaca tgagcttggt 1020

ttgcttgttc ttatggatat tgttcatagt cattcatcaa ataataccct tgacggcttg 1080

aatggtttcg atggcactga tacacattac ttccacggtg gtccacgtgg ccatcattgg 1140

atgtgggatt ctcgtctatt caactatggg agttgggaag tattgagatt cttactgtca 1200

aacgcgagat ggtggcttga agaatataag tttgatggat ttcgatttga tggggtgacc 1260

tccatgatgt atactcacca tggattacaa atgacattta ctgggaacta tggcgagtat 1320

tttggatttg ctactgatgt tgatgcggta gtttacttga tgctggtcaa cgatctaatt 1380

catggacttc atcctgatgc tgtatccatt ggtgaagatg tcagtggaat gcccacattt 1440

tgcatccctg ttccagatgg tggtgttggt tttgactatc gcttgcatat ggctgtagca 1500

gataaatgga ttgaactcct caagcaaagt gacgaatctt ggaaaatggg tgatattgtg 1560

cacaccctaa caaatagaag gtggcttgag aagtgtgtaa cttatgcaga aagtcatgat 1620

caagcactag ttggtgacaa gactattgca ttctggttga tggataagga tatgtatgat 1680

ttcatggctc tggataggcc ttcaactcct cgcattgatc gtggcatagc attacataaa 1740

atgatcaggc ttgtcaccat gggtttaggt ggtgaaggct atcttaactt catgggaaat 1800

gagtttgggc atcctgaatg gatagatttt ccaagaggtc cgcaaactct tccaaccggc 1860

aaagttctcc ctggaaataa caatagttat gataaatgcc gccgtagatt tgatcttgga 1920

gatgcagatt ttcttagata tcatggtatg caagagttcg atcaggcaat gcagcatctt 1980

gaggaaaaat atgggtttat gacatctgag caccagtatg tttcacggaa acatgaggaa 2040

gataaggtga tcatcttcga aagaggagat ttggtatttg ttttcaactt ccactggagc 2100

aatagctttt ttgactaccg tgttgggtgt tccaggcctg ggaagtacaa ggtggcctta 2160

gactccgacg atgcactctt tggtggattc agcaggcttg atcatgatgt cgactacttc 2220

acaaccgaac atccgcatga caacaggccg cgctctttct cggtgtacac tccgagcaga 2280

actgcggtcg tgtatgccct tacagagtaa 2310

<210> 8
<211> 769
<212> PRT
<213> Triticum aestivum

<400> 8

```
Gly Met Ala Thr Phe Ala Val Ser Gly Ala Thr Leu Gly Val Ala Arg
1               5                   10                  15

Pro Pro Ala Ala Ala Gln Pro Glu Glu Leu Gln Ile Pro Glu Asp Ile
            20                  25                  30

Glu Glu Gln Thr Ala Glu Val Asn Met Thr Gly Gly Thr Ala Glu Lys
        35                  40                  45

Leu Glu Ser Ser Glu Pro Thr Gln Gly Ile Val Glu Thr Ile Thr Asp
    50                  55                  60

Gly Val Thr Lys Gly Val Lys Glu Leu Val Val Gly Glu Lys Pro Arg
65                  70                  75                  80

Val Val Pro Lys Pro Gly Asp Gly Gln Lys Ile Tyr Glu Ile Asp Pro
                85                  90                  95
```

Thr Leu Lys Asp Phe Arg Ser His Leu Asp Tyr Arg Tyr Ser Glu Tyr
100 105 110

Arg Arg Ile Arg Ala Ala Ile Asp Gln His Glu Gly Gly Leu Glu Ala
115 120 125

Phe Ser Arg Gly Tyr Glu Lys Leu Gly Phe Thr Arg Ser Ala Glu Gly
130 135 140

Ile Thr Tyr Arg Glu Trp Ala Pro Gly Ala His Ser Ala Ala Leu Val
145 150 155 160

Gly Asp Phe Asn Asn Trp Asn Pro Asn Ala Asp Thr Met Thr Arg Asp
165 170 175

Asp Tyr Gly Val Trp Glu Ile Phe Leu Pro Asn Asn Ala Asp Gly Ser
180 185 190

Pro Ala Ile Pro His Gly Ser Arg Val Lys Ile Arg Met Asp Thr Pro
195 200 205

Ser Gly Val Lys Asp Ser Ile Ser Ala Trp Ile Lys Phe Ser Val Gln
210 215 220

Ala Pro Gly Glu Ile Pro Phe Asn Gly Ile Tyr Tyr Asp Pro Pro Glu
225 230 235 240

Glu Glu Lys Tyr Val Phe Gln His Pro Gln Pro Lys Arg Pro Glu Ser
245 250 255

Leu Arg Ile Tyr Glu Ser His Ile Gly Met Ser Ser Pro Glu Pro Lys
260 265 270

Ile Asn Ser Tyr Ala Asn Phe Arg Asp Glu Val Leu Pro Arg Ile Lys
275 280 285

Arg Leu Gly Tyr Asn Ala Val Gln Ile Met Ala Ile Gln Glu His Ser
290 295 300

```
Tyr Tyr Ala Ser Phe Gly Tyr His Val Thr Asn Phe Phe Ala Pro Ser
305                 310                 315                 320

Ser Arg Phe Gly Thr Pro Glu Asp Leu Lys Ser Leu Ile Asp Arg Ala
                325                 330                 335

His Glu Leu Gly Leu Leu Val Leu Met Asp Ile Val His Ser His Ser
            340                 345                 350

Ser Asn Asn Thr Leu Asp Gly Leu Asn Gly Phe Asp Gly Thr Asp Thr
        355                 360                 365

His Tyr Phe His Gly Gly Pro Arg Gly His His Trp Met Trp Asp Ser
    370                 375                 380

Arg Leu Phe Asn Tyr Gly Ser Trp Glu Val Leu Arg Phe Leu Leu Ser
385                 390                 395                 400

Asn Ala Arg Trp Trp Leu Glu Glu Tyr Lys Phe Asp Gly Phe Arg Phe
                405                 410                 415

Asp Gly Val Thr Ser Met Met Tyr Thr His His Gly Leu Gln Met Thr
            420                 425                 430

Phe Thr Gly Asn Tyr Gly Glu Tyr Phe Gly Phe Ala Thr Asp Val Asp
        435                 440                 445

Ala Val Val Tyr Leu Met Leu Val Asn Asp Leu Ile His Gly Leu His
    450                 455                 460

Pro Asp Ala Val Ser Ile Gly Glu Asp Val Ser Gly Met Pro Thr Phe
465                 470                 475                 480

Cys Ile Pro Val Pro Asp Gly Gly Val Gly Phe Asp Tyr Arg Leu His
                485                 490                 495
```

Met Ala Val Ala Asp Lys Trp Ile Glu Leu Leu Lys Gln Ser Asp Glu
500 505 510

Ser Trp Lys Met Gly Asp Ile Val His Thr Leu Thr Asn Arg Arg Trp
515 520 525

Leu Glu Lys Cys Val Thr Tyr Ala Glu Ser His Asp Gln Ala Leu Val
530 535 540

Gly Asp Lys Thr Ile Ala Phe Trp Leu Met Asp Lys Asp Met Tyr Asp
545 550 555 560

Phe Met Ala Leu Asp Arg Pro Ser Thr Pro Arg Ile Asp Arg Gly Ile
565 570 575

Ala Leu His Lys Met Ile Arg Leu Val Thr Met Gly Leu Gly Gly Glu
580 585 590

Gly Tyr Leu Asn Phe Met Gly Asn Glu Phe Gly His Pro Glu Trp Ile
595 600 605

Asp Phe Pro Arg Gly Pro Gln Thr Leu Pro Thr Gly Lys Val Leu Pro
610 615 620

Gly Asn Asn Asn Ser Tyr Asp Lys Cys Arg Arg Arg Phe Asp Leu Gly
625 630 635 640

Asp Ala Asp Phe Leu Arg Tyr His Gly Met Gln Glu Phe Asp Gln Ala
645 650 655

Met Gln His Leu Glu Glu Lys Tyr Gly Phe Met Thr Ser Glu His Gln
660 665 670

Tyr Val Ser Arg Lys His Glu Glu Asp Lys Val Ile Ile Phe Glu Arg
675 680 685

Gly Asp Leu Val Phe Val Phe Asn Phe His Trp Ser Asn Ser Phe Phe
690 695 700

Asp Tyr Arg Val Gly Cys Ser Arg Pro Gly Lys Tyr Lys Val Ala Leu
705 710 715 720

Asp Ser Asp Asp Ala Leu Phe Gly Gly Phe Ser Arg Leu Asp His Asp
725 730 735

Val Asp Tyr Phe Thr Thr Glu His Pro His Asp Asn Arg Pro Arg Ser
740 745 750

Phe Ser Val Tyr Thr Pro Ser Arg Thr Ala Val Val Tyr Ala Leu Thr
755 760 765

Glu

<210> 9
<211> 2511
<212> DNA
<213> Triticum aestivum

<400> 9
atggctgcgc cggcattcgc agtttccgcg gcggggctgg cccggccgtc ggctcctcga 60

tccggcgggg cagagcggag gggcgcgggg gtggagctgc agtcgccatc gctgctcttc 120

ggccgcaaca agggcacccg ttcaccccgt gccgtcggcg tcggaggttc tggatggcgc 180

gtggtcatgc gcgcgggggg gccgtccggg gaggtgatga tccctgacgg cggtagtggc 240

ggaacaccgc cttccatcga cggtcccgtt cagttcgatt ctgatgatct gaaggttcca 300

ttcattgatg atgaaacaag cctacaggat ggaggtgaag atagtatttg gtcttcagag 360

acaaatcagg ttagtgaaga aattgatgct gaagacacga gcagaatgga caaagaatca 420

tctacgaggg agaaattacg cattctgcca ccaccgggaa atggacagca aatatacgag 480

attgacccaa cgctccgaga ctttaagtac catcttgagt atcgatatag cctatacagg 540

agaatacgtt cagacattga tgaacacgaa ggaggcatgg atgtattttc ccgcggttac 600

gagaagtttg gatttatgcg cagcgctgaa ggtatcactt accgagaatg ggctcctgga 660

gcagattctg cagcattagt tggcgacttc aacaattggg atccaaatgc agaccatatg 720

agcaaaaatg accttggtgt ttgggagatt tttctgccaa acaatgcaga tggttcgcca 780

ccaattcctc acggctcacg ggtgaaggtg cgaatgggta ctccatctgg gacaaaggat 840

tcaattcctg cttggatcaa gtactccgtg cagactccag gagatatacc atacaatgga 900

atatattatg atcctcccga agaggagaag tatgtattca agcatcctca acctaaacga 960

ccaaaatcat tgcggatata tgaaacacat gttggcatga gtagcccgga accaaagatc 1020

aacacatatg caaacttcag ggatgaggtg cttccaagaa ttaaaagact tggatacaat 1080

gcagtgcaaa taatggcaat ccaagagcac tcatactatg gaagctttgg gtaccatgtt 1140

accaatttct ttgcaccaag tagccgtttt gggtccccag aagatttaaa atctttgatt 1200

gatagagctc acgagcttgg cttggttgtc ctcatggatg ttgttcacag tcacgcgtca 1260

aataatacct tggacgggtt gaatggtttt gatggcacgg atacacatta cttccatggc 1320

ggttcacggg gccatcactg gatgtgggat tcccgtgtgt ttaactatgg gaataaggaa 1380

gttataaggt ttctactttc caatgcaaga tggtggctag aggagtataa gtttgatggt 1440

ttccgattcg atggcgcgac ctccatgatg tatacccatc atggattaca agtaaccttt 1500

acaggaagct accatgaata ttttggcttt gccactgatg tagatgcggt cgtttacttg 1560

atgctgatga atgatctaat tcatgggttt tatcctgaag ccgtaactat cggtgaagat 1620

gttagtggaa tgcctacatt tgcccttcct gttcaagttg gtggggttgg ttttgactat 1680

cgcttacata tggctgttgc ccgcaaatgg attgaacttc tcaaaggaaa cgatgaagct 1740

tgggagatgg gtaatattgt gcacacacta acaaacagaa ggtggctgga aaagtgtgtt 1800

acttatgctg aaagtcacga tcaagcactt gttggagaca agactattgc attctggttg 1860

atggacaagg atatgtatga tttcatggcg ctgaacggac cttcgacgcc taatattgat 1920

cgtggaatag cactgcataa aatgattaga cttatcacaa tgggtctagg aggagagggt 1980

tatcttaact ttatgggaaa tgagttcggg catcctgaat ggatagactt tccaagaggc 2040

ccacaagtac ttccaagtgg taagttcatc ccaggaaaca acaacagtta cgacaaatgc 2100

cgtcgaagat ttgacctggg tgatgcagaa tttcttaggt atcatggtat gcagcagttt 2160

gatcaggcaa tgcagcatct tgaggaaaaa tatggtttta tgacatcaga ccaccagtac 2220

gtatctcgga aacatgagga agataaggtg atcgtgtttg aaaaagggga cttggtattt 2280

gtgttcaact tccactggag tagtagctat ttcgactacc gggtcggctg tttaaagcct 2340

gggaagtaca aggtggtctt agactcggac gctggactct ttggtggatt tggtaggatc 2400

catcacactg cagagcactt cacttctgac tgccaacatg acaacaggcc ccattcattc 2460

tcagtgtaca ctcctagcag aacctgtgtt gtctatgctc caatgaacta a 2511

<210> 10
<211> 836
<212> PRT
<213> Triticum aestivum

<400> 10

Met Ala Ala Pro Ala Phe Ala Val Ser Ala Ala Gly Leu Ala Arg Pro
1 5 10 15

Ser Ala Pro Arg Ser Gly Gly Ala Glu Arg Arg Gly Arg Gly Val Glu
20 25 30

Leu Gln Ser Pro Ser Leu Leu Phe Gly Arg Asn Lys Gly Thr Arg Ser
35 40 45

Pro Arg Ala Val Gly Val Gly Gly Ser Gly Trp Arg Val Val Met Arg
50 55 60

Ala Gly Gly Pro Ser Gly Glu Val Met Ile Pro Asp Gly Gly Ser Gly
65 70 75 80

Gly Thr Pro Pro Ser Ile Asp Gly Pro Val Gln Phe Asp Ser Asp Asp
85 90 95

Leu Lys Val Pro Phe Ile Asp Asp Glu Thr Ser Leu Gln Asp Gly Gly
100 105 110

Glu Asp Ser Ile Trp Ser Ser Glu Thr Asn Gln Val Ser Glu Glu Ile
115 120 125

```
Asp Ala Glu Asp Thr Ser Arg Met Asp Lys Glu Ser Ser Thr Arg Glu
    130                 135                 140

Lys Leu Arg Ile Leu Pro Pro Pro Gly Asn Gly Gln Gln Ile Tyr Glu
145                 150                 155                 160

Ile Asp Pro Thr Leu Arg Asp Phe Lys Tyr His Leu Glu Tyr Arg Tyr
                165                 170                 175

Ser Leu Tyr Arg Arg Ile Arg Ser Asp Ile Asp Glu His Glu Gly Gly
            180                 185                 190

Met Asp Val Phe Ser Arg Gly Tyr Glu Lys Phe Gly Phe Met Arg Ser
        195                 200                 205

Ala Glu Gly Ile Thr Tyr Arg Glu Trp Ala Pro Gly Ala Asp Ser Ala
    210                 215                 220

Ala Leu Val Gly Asp Phe Asn Asn Trp Asp Pro Asn Ala Asp His Met
225                 230                 235                 240

Ser Lys Asn Asp Leu Gly Val Trp Glu Ile Phe Leu Pro Asn Asn Ala
                245                 250                 255

Asp Gly Ser Pro Pro Ile Pro His Gly Ser Arg Val Lys Val Arg Met
            260                 265                 270

Gly Thr Pro Ser Gly Thr Lys Asp Ser Ile Pro Ala Trp Ile Lys Tyr
        275                 280                 285

Ser Val Gln Thr Pro Gly Asp Ile Pro Tyr Asn Gly Ile Tyr Tyr Asp
    290                 295                 300

Pro Pro Glu Glu Glu Lys Tyr Val Phe Lys His Pro Gln Pro Lys Arg
305                 310                 315                 320
```

Pro Lys Ser Leu Arg Ile Tyr Glu Thr His Val Gly Met Ser Ser Pro
325 330 335

Glu Pro Lys Ile Asn Thr Tyr Ala Asn Phe Arg Asp Glu Val Leu Pro
340 345 350

Arg Ile Lys Arg Leu Gly Tyr Asn Ala Val Gln Ile Met Ala Ile Gln
355 360 365

Glu His Ser Tyr Tyr Gly Ser Phe Gly Tyr His Val Thr Asn Phe Phe
370 375 380

Ala Pro Ser Ser Arg Phe Gly Ser Pro Glu Asp Leu Lys Ser Leu Ile
385 390 395 400

Asp Arg Ala His Glu Leu Gly Leu Val Val Leu Met Asp Val Val His
405 410 415

Ser His Ala Ser Asn Asn Thr Leu Asp Gly Leu Asn Gly Phe Asp Gly
420 425 430

Thr Asp Thr His Tyr Phe His Gly Gly Ser Arg Gly His His Trp Met
435 440 445

Trp Asp Ser Arg Val Phe Asn Tyr Gly Asn Lys Glu Val Ile Arg Phe
450 455 460

Leu Leu Ser Asn Ala Arg Trp Trp Leu Glu Glu Tyr Lys Phe Asp Gly
465 470 475 480

Phe Arg Phe Asp Gly Ala Thr Ser Met Met Tyr Thr His His Gly Leu
485 490 495

Gln Val Thr Phe Thr Gly Ser Tyr His Glu Tyr Phe Gly Phe Ala Thr
500 505 510

Asp Val Asp Ala Val Val Tyr Leu Met Leu Met Asn Asp Leu Ile His
515 520 525

```
Gly Phe Tyr Pro Glu Ala Val Thr Ile Gly Glu Asp Val Ser Gly Met
    530                 535                 540

Pro Thr Phe Ala Leu Pro Val Gln Val Gly Gly Val Gly Phe Asp Tyr
545                 550                 555                 560

Arg Leu His Met Ala Val Ala Arg Lys Trp Ile Glu Leu Leu Lys Gly
                565                 570                 575

Asn Asp Glu Ala Trp Glu Met Gly Asn Ile Val His Thr Leu Thr Asn
            580                 585                 590

Arg Arg Trp Leu Glu Lys Cys Val Thr Tyr Ala Glu Ser His Asp Gln
        595                 600                 605

Ala Leu Val Gly Asp Lys Thr Ile Ala Phe Trp Leu Met Asp Lys Asp
    610                 615                 620

Met Tyr Asp Phe Met Ala Leu Asn Gly Pro Ser Thr Pro Asn Ile Asp
625                 630                 635                 640

Arg Gly Ile Ala Leu His Lys Met Ile Arg Leu Ile Thr Met Gly Leu
                645                 650                 655

Gly Gly Glu Gly Tyr Leu Asn Phe Met Gly Asn Glu Phe Gly His Pro
            660                 665                 670

Glu Trp Ile Asp Phe Pro Arg Gly Pro Gln Val Leu Pro Ser Gly Lys
        675                 680                 685

Phe Ile Pro Gly Asn Asn Asn Ser Tyr Asp Lys Cys Arg Arg Arg Phe
    690                 695                 700

Asp Leu Gly Asp Ala Glu Phe Leu Arg Tyr His Gly Met Gln Gln Phe
705                 710                 715                 720
```

```
Asp Gln Ala Met Gln His Leu Glu Glu Lys Tyr Gly Phe Met Thr Ser
                725                 730                 735

Asp His Gln Tyr Val Ser Arg Lys His Glu Glu Asp Lys Val Ile Val
            740                 745                 750

Phe Glu Lys Gly Asp Leu Val Phe Val Phe Asn Phe His Trp Ser Ser
        755                 760                 765

Ser Tyr Phe Asp Tyr Arg Val Gly Cys Leu Lys Pro Gly Lys Tyr Lys
    770                 775                 780

Val Val Leu Asp Ser Asp Ala Gly Leu Phe Gly Gly Phe Gly Arg Ile
785                 790                 795                 800

His His Thr Ala Glu His Phe Thr Ser Asp Cys Gln His Asp Asn Arg
                805                 810                 815

Pro His Ser Phe Ser Val Tyr Thr Pro Ser Arg Thr Cys Val Val Tyr
            820                 825                 830

Ala Pro Met Asn
        835


<210>  11
<211>  25
<212>  DNA
<213>  Artificial

<220>
<223>  PCR primer

<400>  11
tggataaata caaagagtga agcag                                         25


<210>  12
<211>  23
<212>  DNA
<213>  Artificial

<220>
```

<223> PCR primer

<400> 12
ggacattgaa ggtgttgtaa agg 23

<210> 13
<211> 25
<212> DNA
<213> Artificial

<220>
<223> Taqman Sonde

<400> 13
cttcgtcgtc caaacaagac cacag 25

<210> 14
<211> 19
<212> DNA
<213> Artificial

<220>
<223> PCR Primer

<400> 14
gtgggagcaa ctccagctt 19

<210> 15
<211> 19
<212> DNA
<213> Artificial

<220>
<223> PCR Primer

<400> 15
cggtgagggc agagttgtt 19

<210> 16
<211> 16
<212> DNA
<213> Artificial

<220>
<223> Taqman-Sonde

<400> 16
actccggcgc ccccgt 16